# EUROPEAN PATENT SPECIFICATION

(11) **EP 1 572 729 B1**
(45) Date of publication and mention of the grant of the patent: **22.09.2010**
(21) Application number: 03779785.9
(22) Date of filing: 28.08.2003
(51) Int. Cl.: A61K 39/095, C07K 14/22, C07K 16/12, G01N 33/48

(54) **MUTANT FRPB PROTEIN AND REFOLDING METHOD**
FRPB PROTEINMUTANTE UND VERFAHREN ZUR ERNEUTEN FALTUNG
FRPB PROTEINE MUTANTE ET PROCEDE DE REPLIEMENT

(30) Priority: 30.08.2002 GB 0220199
(43) Date of publication of application: 14.09.2005
(73) Proprietor: GlaxoSmithKline Biologicals s.a., 1330 Rixensart (BE); Utrecht University, 3584 CH Utrecht (NL); Technology Foundation (Stichting voor de Technische Wetenschappen), 3527 JP Utrecht (NL)
(72) Inventor: BIEMANS, Ralph, GlaxoSmithKline Biologicals S.A., B-1330 Rixensart (BE); DENOEL, Philippe, GlaxoSmithKline Biologicals S.A., B-1330 Rixensart (BE); FERON, Christiane, GlaxoSmithKline Biologicals SA, B-1330 Rixensart (BE); GORAJ, Carine, GlaxoSmithKline Biologicals S.A., B-1330 Rixensart (BE); KORTEKAAS, Jeroen, Utrecht University, NL-3584 CH Utrecht (NL); POOLMAN, Jan, GlaxoSmithKline Biologicals S.A., B-1330 Rixensart (BE); TOMMASSEN, Jan, Utrecht University, NL-3584 CH Utrecht (NL); WEYNANTS, Vincent, GlaxoSmithKline Biologicals SA, B-1330 Rixensart (BE)
(74) Representative: Lubienski, Michael John
(86) International application number: PCT/EP2003/009634
(87) International publication number: WO 2004/020463

(56) References cited:
- WO-A-01/55182
- US-B1- 6 265 567
- JANSEN C ET AL: "Biochemical and biophysical characterization of in vitro folded outer membrane porin PorA of Neisseria meningitidis" BIOCHIMICA ET BIOPHYSICA ACTA. BIOMEMBRANES, AMSTERDAM, NL, vol. 1464, no. 2, 5 April 2000 (2000-04-05), pages 284-298, XP004273195 ISSN: 0005-2736
- VAN DER LEY PETER ET AL: "Sequence variability of FrpB, a major iron-regulated outer-membrane protein in the pathogenic neisseriae" MICROBIOLOGY (READING), vol. 142, no. 11, 1996, pages 3269-3274, XP001182994 ISSN: 1350-0872 cited in the application
- PETTERSSON A ET AL: "MOLECULAR CHARACTERIZATION OF FRPB, THE 70-KILODALTON REGULATED OUTER MEMBRANE PROTEIN OF NEISSERIA MENINGITTIDIS" INFECTION AND IMMUNITY, AMERICAN SOCIETY FOR MICROBIOLOGY. WASHINGTON, US, vol. 63, no. 10, October 1995 (1995-10), pages 4181-4184, XP002941952 ISSN: 0019-9567

## Description

### Field of the Invention

The present invention relates to mutants of FrpB - an outer membrane protein of *Neisseria meningitidis* organisms, to a method of refolding FrpB proteins, to such refolded proteins, pharmaceutical compositions comprising FrpB proteins, and their use in the treatment, prevention and diagnosis of bacterial infections, such as Neisserial infections, and particularly, but not exclusively, *Neisseria meningitidis* and/or *Neisseria gonorrhoeae.*

### Background of the Invention

Neisserial strains of bacteria are the causative agents for a number of human pathologies, against which there is a need for effective vaccines to be developed. In particular *Neisseria gonorrhoeae* and *Neisseria meningitidis* cause pathologies which could be treated by vaccination.

*Neisseria gonorrhoeae* is the etiologic agent of gonorrhea, one of the most frequently reported sexually transmitted diseases in the world with an estimated annual incidence of 62 million cases (Gerbase et al 1998 Lancet 351; (Suppl 3) 2-4). The clinical manifestations of gonorrhea include inflammation of the mucus membranes of the urogenital tract, throat or rectum and neonatal eye infections. Ascending gonococcal infections in women can lead to infertility, ectopic pregnancy, chronic pelvic inflammatory disease and tubo-ovarian abscess formation. Septicemia, arthritis, endocarditis and meningitis are associated with complicated gonorrhea.

The high number of gonococcal strains with resistance to antibiotics contributes to increased morbidity and complications associated with gonorrhea. An attractive alternative to treatment of gonorrhea with antibiotics would be its prevention using vaccination. No vaccine currently exists for *N. gonorrhoeae* infections.

*Neisseria meningitidis* (meningococcus) is a Gram-negative bacterium frequently isolated from the human upper respiratory tract. It occasionally causes invasive bacterial diseases such as bacteremia and meningitis. Most caes of disease are in infants or young children. The incidence of meningococcal disease shows geographical seasonal and annual differences (Schwartz, B., Moore, P.S., Broome, C.V.; Clin. Microbiol. Rev. 2 (Supplement), S18-S24, 1989). Most disease in temperate countries is due to strains of serogroup B and varies in incidence from 1-10/100,000/year total population sometimes reaching higher values (Kaczmarski, E.B. (1997), Commun. Dis. Rep. Rev. 7: R55-9, 1995; Scholten, R.J.P.M., Bijlmer, H.A., Poolman, J.T. et al. Clin. Infect. Dis. 16: 237-246, 1993; Cruz, C., Pavez, G., Aguilar, E., et al. Epidemiol. Infect. 105: 119-126, 1990).

Epidemics dominated by serogroup A meningococci, mostly in central Africa, are encountered, sometimes reaching levels up to 1000/100.000/year (Schwartz, B., Moore, P.S., Broome, C.V. Clin. Microbiol. Rev. 2 (Supplement), S18-S24, 1989). Nearly all cases as a whole of meningococcal disease are caused by serogroup A, B, C, W-135 and Y meningococci and a tetravalent A, C, W-135, Y polysaccharide vaccine is available (Armand, J., Arminjon, F., Mynard, M.C., Lafaix, C., J. Biol. Stand. 10: 335-339, 1982).

The frequency of *Neisseria meningitidis* infections has risen dramatically in the past few decades. This has been attributed to the emergence of multiply antibiotic resistant strains and an increasing population of people with weakened immune systems. It is no longer uncommon to isolate *Neisseria meningitidis* strains that are resistant to some or all of the standard antibiotics. This phenomenon has created an unmet medical need and demand for new anti-microbial agents, vaccines, drug screening methods, and diagnostic tests for this organism.

Van der Ley et al (1996) Microbiology 142:3269-3274 reports that there is at present no vaccine available against group B organisms, which are the predominant cause of meningococcal disease. The use of the group B capsular polysaccharide as a vaccine has been hindered by the presence of identical structures on neural cell adhesion molecules in the human host, raising the possibility of inducing autoimmune pathology with such a vaccine. It has been proposed to use vaccines based on meningococcal outer membrane proteins. However, it has been found that a large proportion of the protective antibodies induced are strain specific. In addition, not all outer membrane components contribute to the induction of bactericidal antibodies. The FrpB protein from Neisseria is an iron-regulated outer membrane protein that shows the characteristics of a TonB-dependent outer membrane receptor. It has a molecule weight of around 77kDa; although this may vary between strains. It has been thought that the FrpB protein may be a good candidate for the production of bactericidal antibodies. However Ala'Aldeen et al (1994) Vaccine 12:535 found that monoclonal antibodies (mAbs) isolated against wild-type FrpB were found to be strain specific.

It has also been envisaged that recombinant FrpB expressed in cells could be produced for use in such new anti-microbial agents, vaccines, drug screening methods, and diagnostic tests. However, one of the major limitations on the expression of proteins is the inability of many recombinant proteins to fold into their biologically active conformations. Often only low yields of the recombinant protein are obtained due to aggregation and mis-folding of the unfolded species. Indeed, protein refolding, in which the protein acquires it native and active structure, is one of the biggest challenges in molecular biology. Refolding of PorA of *N*. *meningitidis* is discussed in Jansen et al. 2000 Biochimica et Biophysica Acta. Biomembranes, Amsterdam, NL, vol. 1464, No. 2, page 284-298.

Clearly, the need therefore remains to provide a vaccine against Neisseria disease comprising purified protective outer membrane proteins including mutant proteins in a refolded conformation suitable to elicit an effective immune response, preferably a cross-protective response.

### Summary of the Invention

The present invention provides loop-deletion mutant FrpB proteins, which may induce broad-spectrum bactericidal antibodies.

The present invention also provides a method for refolding the FrpB protein.

The present invention also relates to such refolded FrpB proteins.

The present invention also provides methods for using the proteins of the present invention, including their use in the prevention and treatment of microbial diseases, amongst others, in e.g. subunit and outer membrane vesicle vaccines. In a further aspect, the invention relates to diagnostic assays for detecting diseases associated with microbial infections and conditions associated with such infections.

### Statements of the Invention

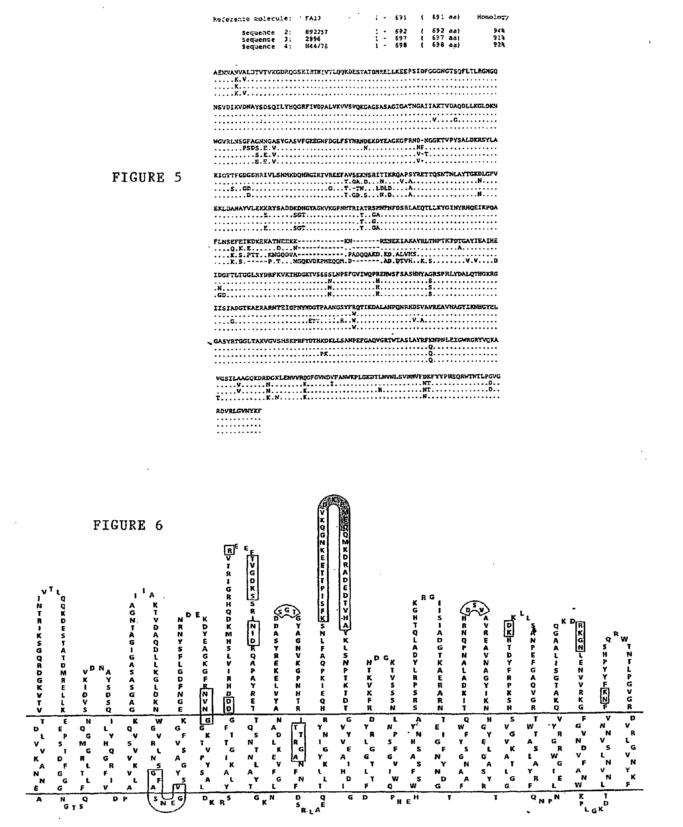

According to one aspect of the present invention there is provided a neisserial FrpB protein having one or more deletions of non-conserved amino acids of loop 7 and loop 5, according to the loop numbering of Figure 6, compared to a corresponding wild-type neisserial FrpB protein. These proteins of the present invention are referred to generally as "mutant" proteins. The term "deletion" as used herein includes the substitution, replacement or mutation of one or more of the non-conserved amino acids. The substituted sequence may or may not be the same size as the sequence it replaces. By "non-conserved" amino acids we include amino acids present in a wild-type FrpB protein from a first strain, but which may not be present in a wild-type FrpB protein from a second strain. The proteins of the present invention are isolated proteins. By "isolated" we include proteins that have been removed from their natural state or otherwise subjected to human intervention. Preferably the protein has also been purified to a level of more than 40, 50, 60, 70, 80, 90, 95 or 99%.

The present disclosure is therefore broadly directed to proteins having conserved amino acids of FrpB proteins.

Preferably the mutant protein according to this invention is non-strain-specific, cross-reactive, and/or cross-protective. By "strain-specific" we include the generation of an immune response which is directed to, or at least predominantly directed to, an autologous (homologous) *N. meningitidis* strain. By "cross-reactive" we include the generation of an immune response directed to one or more heterologous Neisserial, preferably *N. meningitidis,* strains. By "cross-protective" we include the generation of an immune response providing protection against infection by one of more heterologous Neisserial, preferably *N. meningitidis,* strains. Preferably the protein of the present invention is conformationally stable.

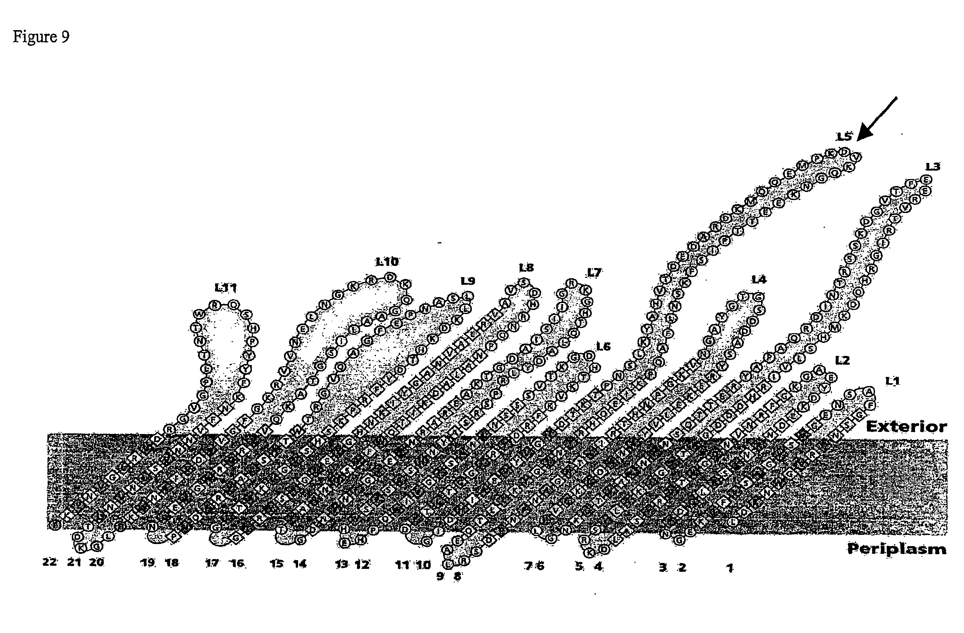

The protein is one in which one or more of the amino acids of at least 2 loops have been deleted. The loops may be determined by sequence comparison. The protein is one in which one or more of the amino acids of loop 7 and 5 have been deleted. The deletions are from loop 7 and loop 5. Loops 5 and 7 have been identified as being regions of FrpB proteins having relatively high frequencies of non-conserved amino acids. The numbering of the FrpB surface loops follows the numbering system used by Pettersson et al (1995) Infect. Immunology 63:4181-4184 (Figure 6); however corresponding deletions can be made according to other models which are or become available (for instance that of Figure 9, the "new model", where loops 3 and 5 correspond roughly with loops 5 and 7, respectively, of the old model of figure 6). It will be appreciated that corresponding deletions may be determined using sequence comparison methods.

The protein may also have one or more amino acid deletions from any one or more of loops 1, 2, 4, 6, 8, 9, 10, 11, 12 and 13 (or the corresponding loops according to any other topological model [e.g. the "new model"]); although the present invention preferably comprises one or more of these more constant regions of an FrpB protein.

In one embodiment the protein is one in which 11 to 33 amino acids have been deleted from loop 7, more preferably 23-33 amino acids, even more preferably 28 amino acids have been deleted from loop 7.

In another embodiment the protein is one in which 18-29 amino acids have been deleted from loop 5, more preferably 19-29 amino acids, even more preferably 24 amino acids have been deleted from loop 5.

In one embodiment there is provided a protein in which, with reference to FrpB strain H44/76, the amino acid deletion is made in the range of amino acids 376-413, more preferably 381-408, or a corresponding deletion made, from loop 7.

In a preferred embodiment there is provided a protein in which, with reference to FrpB strain H44/76, an amino acid sequence comprising TTEEKNGQKVDKPMEQQMKDRADEDTVH has been deleted, or a corresponding deletion made, from loop 7.

In a preferred embodiment there is provided a protein in which, with reference to FrpB strain H44/76, the amino acid deletion is made in the range of amino acids 247-280, more preferably 252-275, or a corresponding deletion made, from loop 5.

In a further preferred embodiment there is provided a protein in which, with reference to FrpB strain H44/76, an amino acid sequence comprising

QHRGIRTVREEFTVGDKSSRINID has been deleted, or a corresponding deletion made, from loop 5.

The present invention also provides a polynucleotide encoding the mutant protein of the present invention.

According to another aspect of the present invention there is provided an expression vector comprising the polynucleotide of the present invention.

According to another aspect of the present invention there is provided a host cell comprising the polynucleotide of the present invention.

According to another aspect of the present invention there is provided a method for producing the mutant protein of the present invention comprising: culturing the host cell of the invention, and recovering the expressed protein.

According to another aspect of the present invention there is provided a method for refolding an FrpB protein of the invention comprising contacting the FrpB protein with an alkaline refolding buffer comprising 3-dimethyldodecylammoniopropanesulfonate (Zwittergent 3-12 or SB-12).

In one embodiment the refolding buffer comprises ethanolamine and SB-12.

Preferably the ethanolamine is about 15-30, preferably about 20mM ethanolamine.

Preferably the refolding buffer has pH11.

Preferably the SB-12 is 0.2-1% SB-12 more preferably 0.3-0.8% SB-12, even more preferably 0.5% SB-12.

In a preferred embodiment the refolding buffer further comprises urea, NaCl (such as 0.4M NaCl) and/or guanidium chloride, more preferably guandinium chloride, most preferably 0.4M guanidium chloride.

According to another aspect of the present invention there is provided a method comprising (preferably all of) the following steps:
optionally expressing an FrpB protein in a host cell;
optionally breaking the host cell to obtain an inclusion body comprising the FrpB protein;
optionally washing the inclusion body;
optionally solubilisation of at least part of the inclusion body and the FrpB protein,
preferably with Guanidinium hydrochloride;
contacting the solubilised FrpB protein with the refolding buffer; and
optionally removing the refolding buffer from the FrpB protein.

According to another aspect of the present invention there is provided refolding buffer comprising ethanolamine, SB-12 and guanidium chloride (in the concentrations mentioned above) for use in the method of the present invention.

According to another aspect of the present invention there is provided an isolated, refolded FrpB protein of the invention obtained or obtainable by the method of the present invention.

According to another aspect of the present invention there is provided a pharmaceutical composition comprising at least one FrpB protein of the present invention, and a pharmaceutically acceptable carrier.

Preferably at least 30%, 50%, 70%, or 90% of the FrpB protein present in the composition is refolded.

Preferably the composition is in the form of a vaccine.

In one embodiment the pharmaceutical composition comprises a refolded FrpB protein of the invention derived from *Neisseria meningitidis.*

In another embodiment the pharmaceutical composition comprises a refolded FrpB protein of the invention derived from *Neisseria gonorrhoeae.*

Preferably the pharmaceutical composition comprises at least one other Neisserial antigen.

In one embodiment the pharmaceutical composition comprises at least one other Neisserial antigen derived from *Neisseria gonorrhoeae.*

In another embodiment the pharmaceutical composition comprises at least one other Neisserial antigen derived from *Neisseria meningitidis.*

In one embodiment the pharmaceutical composition is in the form of a subunit composition; although it may be admixed with an outer membrane vesicle preparation.

In another embodiment the pharmaceutical composition is in the form of an outer membrane vesicle preparation; although it may be mixed with a subunit composition.

Preferably said composition further comprises at least one Neisserial adhesin, or one other Neisserial outer membrane protein, or at least one Neisserial autotransporter, or at least one Neisserial toxin, or at least one Neisserial Fe acquisition protein. Most preferably, in addition to isolated FrpB of the invention, at least one Neisserial autotransporter and at least one Neisserial toxin is present, or at least one Neisserial autotransporter and at least one Neisserial Fe acquisition protein is present, or at least one Neisserial toxin and at least one Neisserial Fe acquisition protein is present, or at least one Neisserial autotransporter and at least one Neisserial Fe acquisition protein and at least one Neisserial toxin is present, or immunogenic fragments thereof.

More preferably the pharmaceutical composition comprises at least one further antigen (or fragment thereof) selected from at least one of the following classes:
at least one Neisserial adhesin selected from the group consisting of FhaB, NspA Hsf, NadA, PilC, Hap, MafA, MafB, Omp26, NMB0315, NMB0995 and NMB1119;
at least one Neisserial autotransporter selected from the group consisting of Hsf, Hap, IgA protease, AspA and NadA;
at least one Neisserial toxin selected from the group consisting of FrpA, FrpC, FrpA/C, VapD, NM-ADPRT, and either or both of LPS immunotype L2 and LPS immunotype L3;
at least one Neisserial Fe acquisition protein selected from the group consisting of TbpA high, TbpA low, TbpB high, TbpB low, LbpA, LbpB, P2086, HpuA, HpuB, Lipo28, Sibp, FbpA, BfrA, BfrB, Bcp, NMB0964 and NMB0293; and
at least one Neisserial membrane associated protein, preferably outer membrane protein, selected from the group consisting of PldA, NspA, TspA, FhaC, NspA, TbpA(high), TbpA(low), LbpA, HpuB, TdfH, PorB, HimD, HisD, GNA1870, OstA, HlpA, MltA, NMB 1124, NMB 1162, NMB 1220, NMB 1313, NMB 1953, HtrA, TspB, PilQ and OMP85.

Most preferably the pharmaceutical composition further comprises at least one other Neisserial antigen (or a fragment thereof) selected from one or more of the following classes:
a) at least one Neisserial adhesin selected from the group consisting of FhaB, Hsf and NadA;
b) at least one Neisserial autotransporter selected from the group consisting of Hsf, and Hap;
c) at least one Neisserial toxin selected from the group consisting of FrpA, FrpC, and either or both of LPS immunotype L2 and LPS immunotype L3;
d) at least one Neisserial Fe acquisition protein selected from the group consisting of TbpA, TbpB, LbpA and LbpB; and
e) at least one Neisserial outer membrane protein selected from the group consisting of PldA, NspA, TspA, TspB, PilQ and OMP85.

Where the additional antigens are present on a bleb (OMV) they are preferably upregulated in expression (compared to wild-type expression levels), for instance as described in WO 01/09350.

Preferably the pharmaceutical composition further comprises bacterial capsular polysaccharides or oligosaccharides. The capsular polysaccharides or oligosaccharides may be derived from bacteria selected from the group consisting of *Neisseria meningitidis* serogroup A, C, Y, and/or W-135, *Haemophilus influenzae b, Streptococcus pneumoniae,* Group A Streptococci, Group B Streptococci, *Staphylococcus aureus* and *Staphylococcus epidermidis,* and are most preferably conjugated to a source of T-hefper epitopes.

According to another aspect of the present invention there is provided use of an FrpB protein of the present invention (or a pharmaceutical composition of the present invention) in the preparation of a medicament for use in generating an immune response in an animal. Preferably the use is in the preparation of a medicament for treatment of prevention of Neisserial infection. In one embodiment *Neisseria meningitidis* infection is prevented or treated. In another embodiment *Neisseria gonorrhoeae* infection is prevented or treated.

According to another aspect of the present invention there is provided an antibody immunospecific for the FrpB protein of the present invention.

According to another aspect of the present invention there is provided a pharmaceutical composition useful in treating humans with a Neisserial disease comprising at least one antibody of the present invention and a suitable pharmaceutical carrier.

According to yet another aspect of the present invention there is provided use of the antibody of the present invention (or composition comprising it) in the manufacture of a medicament for the treatment or prevention of Neisserial disease. In one embodiment *Neisseria meningitidis* infection is prevented or treated. In another embodiment *Neisseria gonorrhoeae* infection is prevented or treated.

According to a further aspect of the present invention there is provided a method of diagnosing a Neisserial infection, comprising identifying an FrpB protein of the present invention, or an antibody as of the present invention, present within a biological sample from an animal suspected of having such an infection, or by using an FrpB protein or antibody of the present invention to detect whether FrpB or antibodies against FrpB are present within a biological sample from an animal. In one embodiment *Neisseria meningitidis* infection is diagnosed. In another embodiment *Neisseria gonorrhoeae* infection is diagnosed.

### Detailed Description

Various preferred features and embodiments of the present invention will now be described by way of non-limiting example with reference to the accompanying drawing in which:

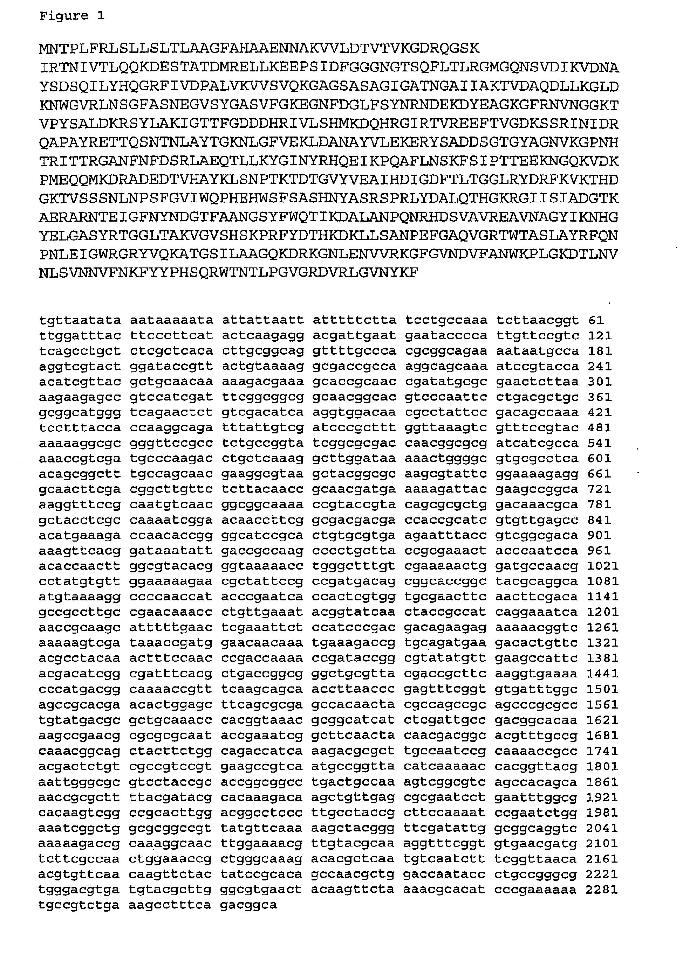

Figure 1 shows the amino acid and nucleotide sequences of FrpB strain H44/76;

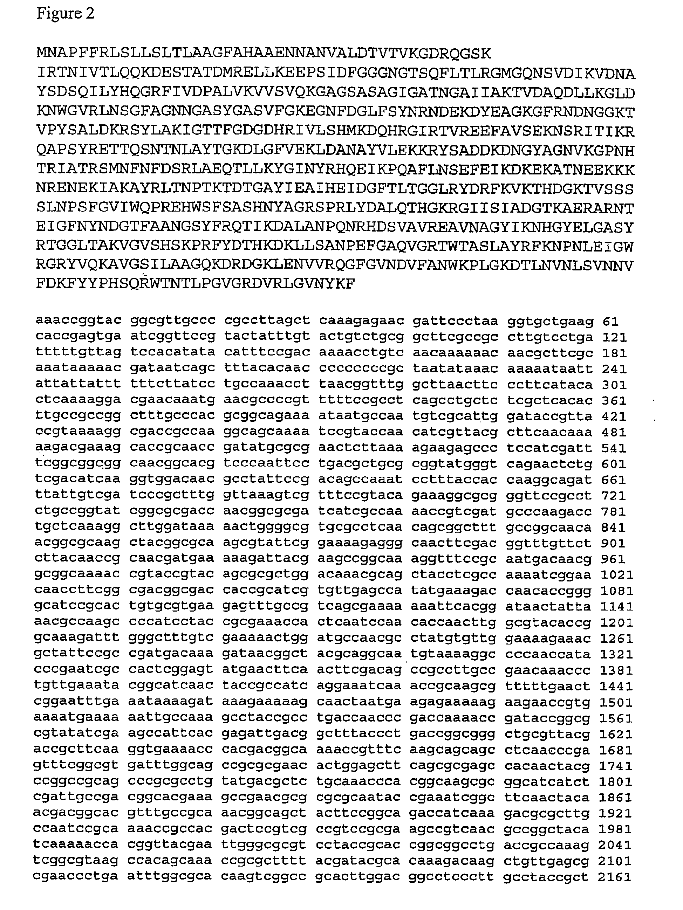

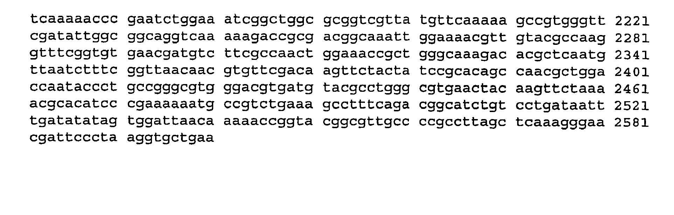

Figure 2 shows the amino acid and nucleotide sequences of FrpB strain FA 19;

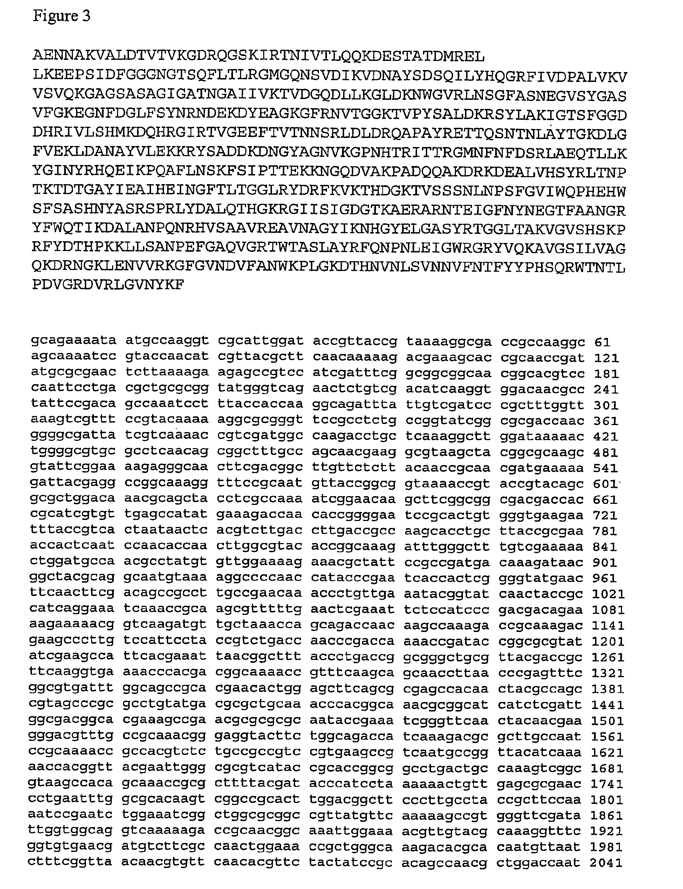

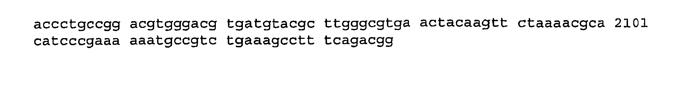

Figure 3 shows the amino acid and nucleotide sequences of FrpB strain 2996;
Figure 4 shows the amino acid and nucleotide sequences of FrpB strain 892257;
Figure 5 shows sequence comparison of the mature FrpB protein from *Neisseria gonorrhoeae* strain FA 19 and *Neisseria meningitidis* strains 892257, 2996, and H44/76;
Figure 6 shows the topology of FrpB strain H44/76 in the outer membrane according to the model of Pettersson et al (1995) Infect. Immunology 63:4181-4184;
Figure 7 shows sequence comparison of loop 7 region FrpB from eight different meningococcal strains. The H44/76 strain is used as the reference; and

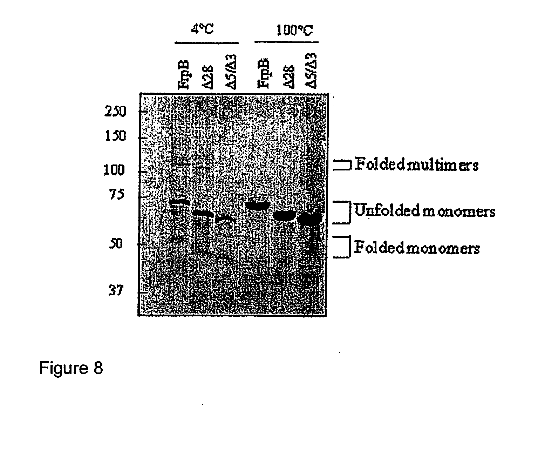

Figure 8 shows a gel demonstrating *in vitro* folding of urea-denatured FrpB, FrpBΔ28 and FrpBΔ7Δ5.
Figure 9 shows the new topology model of FrpB in its outer membrane, Loops 3 and 5 (equivalent of loops 5 and 7, respectively, in the old method) are shown.
Figure 10 shows A) acrylamide gel showing how FrpB is properly refolded, and B) CD spectrum recorded on a Jasco-810 spectropolarimeter [average of 30 scans] showing that a highly folded product is obtained.
Figure 11 Evaluation of the immuneresponse against *in vitro* folded FrpB (mutants) Western blot performed with the parent strain and 16 heterologous meningococcal strains (first lane shows the parent strain).
Figure 12: Dot blot analysis of *in vitro* folded FrpB-C1 and FrpB-C2. In vitro folded proteins were kept cold or heated to unfold the protein. Immunodetection was performed with mouse monoclonal antibody MNSC11G 1:100,000 (α-PorA 1.7, 16).

Although in general the techniques mentioned herein are well known in the art, reference may be made in particular to Sambrook et al., Molecular Cloning, A Laboratory Manual (1989) and Ausubel et al., Short Protocols in Molecular Biology (1999) 4th Ed, John Wiley & Sons, Inc (as well as the complete version Current Protocols in Molecular Biology).

The terms "comprising", "comprise" and "comprises" herein is intended by the inventors to be optionally substitutable with the terms "consisting of", "consist of", and "consists of", respectively, in every instance.

### FrpB protein

FrpB is the most abundant iron-limitation-inducible outer-membrane protein of *Neisseria meningitidis.* Thus, as used herein, the term "FrpB" includes Fe-regulated protein B and generally encompasses any protein having an amino acid sequence identical, or substantially identical, to the amino acid sequence of wild-type (also known as naturally occurring) FrpB. Further details on FrpB can be found in US 6,265,567. "FrpB" and "FrpB protein" as used interchangeably herein. FrpB is also be known as FetA. As used herein the term "protein" includes the term "polypeptide". The present invention relates to mutant forms of the FrpB protein, as well as refolded mutant FrpB protein. By "isolated" we mean that the FrpB protein is free from other proteins with which it is normal associated. The refolded FrpB protein is preferably a recombinant protein. By "recombinant" we mean that the protein has been obtained using the application of molecular biology. However the refolding method is also applicable to natural or synthetic proteins which require refolding, or purification by means of unfolding and refolding. Preferably the isolated FrpB of the invention is purified in that it is more than 40, 50, 60, 70, 80, 90, 95, or 99% pure. Most preferably the FrpB of the invention is biologically pure in that it is more than 40, 50, 60, 70, 80, 90, 95, or 99% free of other Neisserial proteins and/or other proteins of the host cell from which it was made.

The present invention provides mutant FrpB proteins. The term "mutant" in relation to the amino acid sequences of the present invention includes any substitution of, variation of, modification of, replacement of, deletion of or addition of one (or more) amino acids from or to the FrpB sequence providing the resultant amino acid sequence preferably is capable of raising antibodies which recognise an FrpB protein (if necessary when coupled to a carrier). For ease of reference this is referred to as deletion of one (or more) amino acids. The protein is mutated in the sense that it is varied from the corresponding wild-type FrpB sequence, preferably such that the immune response is directed to non-varied surface loops. Thus, the sequence may also be deleted in the sense that it is replaced by non-natural (a peptide from a difference Neisserial protein, or from a completely different species, preferably a Chlamydial peptide) sequence, preferably such that the immune response is directed to non-varied surface loops. In one embodiment the sequence is replaced with conserved sequence from another organism (e.g. Chlamydia trachomatis).

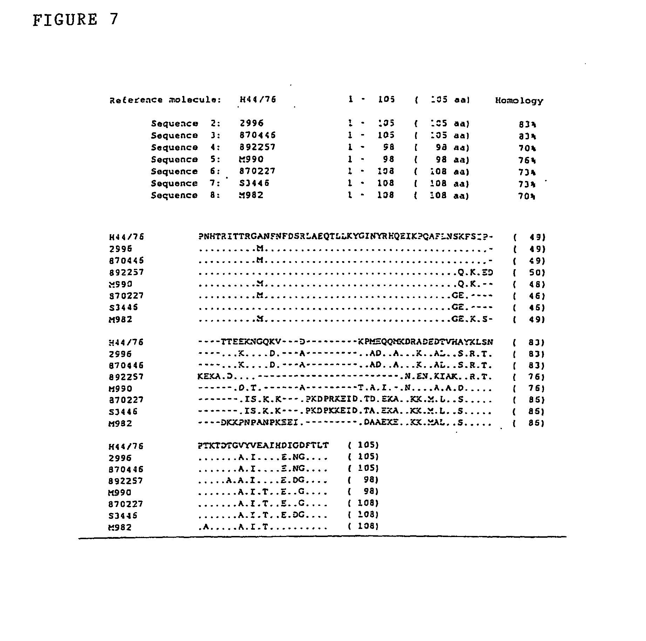

According to the present invention at least part of the surface exposed loops 7 and 5 of FrpB protein is deleted. These deletions will now be described for ease of reference to *Neisseria meningitidis* strain H44/76. However corresponding deletions can be made in any *Neisseria meningitidis* strain, and indeed any Neisserial organism or strain, such as for example *Neisseria gonorrhoeae.* Such corresponding deletions can be determined using sequence comparison as exemplified in Figures 5 and 7.

For ease we refer herein to the topological model of Pettersson; however, it will be appreciated that we could equally well refer to any other model (e.g. the new model of figure 9, which will be specifically referred to if it is intended to be used in the context of this invention).

As mentioned above the mutant comprises a deletion in loop 7. According to this feature (with reference to FrpB strain H44/76) in the range of 10 to 35 amino acids are deleted from loop 7, preferably 15-33, or 20-33, and most preferably 24 to 33 amino acids are deleted from loop 7. Preferably this deletion is made in the range of amino acids 376-413.

In a particularly preferred embodiment, about 28 amino acids are deleted from loop 7. This the amino acid deletion is preferably made in the range of amino acids 381-408.
Thus preferably an amino acid sequence comprising the sequence TTEEKNGQKVDKPMEQQMKDRADEDTVH is deleted from loop 7.

With regard to deletions in respect of loop 5, ( with reference to FrpB strain H44/76) in the range of 7-30 amino acids are deleted from loop 5, preferably 10-29, or 15-29, and most preferably 19 to 29 amino acids are deleted from loop 5. Preferably these deletions are made in the range of amino acids 247-280.

In a particularly preferred embodiment about 24 amino acids are deleted from loop 5. Preferably this amino acid deletion is made in the range of amino acids 252-275.
Thus, preferably an amino acid sequence comprising the sequence QHRGIRTVREEFTVGDKSSRINID is deleted from loop 5.

As mentioned above corresponding deletions can easily be determined. By way of example, such corresponding deletions will now be discussed in relation to *Neisseria gonorrhoeae* strain FA19 and *Neisseria meningitidis* strains 892257 and 2996. However the invention may be applicable to any FrpB protein which is or becomes available.

With reference to FrpB strain FA19 preferably in the range of 11 to 21 amino acids are deleted from loop 7. This amino acid deletion may be made in the range of amino acids 381-406.

Preferably, with reference to FrpB strain FA19, about 16 amino acids are deleted from loop 7. This amino acid deletion is preferably made in the range of amino acids 386-401. Thus, an amino acid sequence comprising the sequence TNEEKKKNRENEKIAK may be deleted from loop 7.

With reference to FrpB strain FA 19 preferably in the range of 19 to 29 amino acids are deleted from loop 5. This amino acid deletion is preferably made in the range of amino acids 247-280.

Preferably with reference to FrpB strain FA 19 about 24 amino acids are deleted from loop 5. This amino acid deletion is preferably made in the range of amino acids 252-275. Thus, an amino acid sequence comprising the sequence QHRGIRTVREEFAVSEKNSRITIK may be deleted from loop 5.

Preferably, with reference to FrpB strain 2996, in the range of 18 to 28 amino acids are deleted from loop 7. This amino acid deletion may be made in the range of amino acids 358-390.

Preferably 23 amino acids are deleted from loop 7. This amino acid deletion may be made in the range of amino acids 363-385. Thus, an amino acid sequence comprising the sequence NGQDVAKPADQQAKDRKDEALVH may be deleted from loop 7.

With reference to FrpB strain 2996 preferably in the range of 18 to 28 amino acids are deleted from loop 5. This amino acid deletion may be made in the range of amino acids 225-257.

Preferably about 23 amino acids are deleted from loop 5. This amino acid deletion may be made in the range of amino acids 230-252. Thus, an amino acid sequence comprising the sequence QHRGIRTVGEEFTVTNNSRLDLD may be deleted from loop 5.

With reference to FrpB strain 892257 preferably in the range of 11 to 21 amino acids are deleted from loop 7. This amino acid deletion is made in the range of amino acids 360-385.

Preferably about 16 amino acids are deleted from loop 7. This amino acid deletion may be made in the range of amino acids 365-380. Thus, an amino acid sequence comprising the sequence TDEEKNKNRENEKIAK may be deleted from loop 7.

With reference to FrpB strain 892257 preferably in the range of 19 to 29 amino acids are deleted from loop 5. This amino acid deletion may be made in the range of amino acids 226-259.

Preferably 24 amino acids are deleted from loop 5. This amino acid deletion may be made in the range of amino acids 231-259. Thus, an amino acid sequence comprising the sequence QHRGIRTVREEFTVGAKDSRINIK may be deleted from loop 5.

As indicated above at least some amino acids from at least one of the surface loops of the FrpB protein (preferably loop 7 and/or loop 5) may be replaced by non-natural, i.e. heterologous sequence. It will also be appreciated that shuffling of regions between FrpB proteins of different Neisserial strains is possible. Preferably such replacement sequence is from another bacterial outer membrane protein. It is preferred that such replacement sequences are conserved, i.e. able to generate an immune response against more than one strain of a bacterial organism. In one embodiment the replacement sequence is also derived from Neisserial outer membrane proteins, such as *Neisseria gonorrhoeae* or *Neisseria meningitidis.* An example of such a suitable outer membrane protein is given in US 5,912,336 which describes another Nessierial iron regulated protein, designated TbpA. Replacement sequence could conveniently be derived from any one or more of loops 2, 3, 4, 5 and 8 of TbpA. These loops correspond generally to amino acids 226-309; 348-395; 438-471; 512-576 and 707-723 ofTbpA respectively. Preferably one or more of loops 4, 5 and 8 are incorporated.

Another example of such a suitable outer membrane protein is given in WO01/55182, which describes the NhhA (or Hsf) surface antigen from *Neisseria meningitidis.* Replacement sequence could conveniently be derived from one or more constant regions of an NhhA protein generally designated as C1, C2, C3, C4 and C5. An example of another replacement sequence which could be used in the present invention is described in EP 0 586 266.

Further Neisserial OMP loops that may be substituted for FrpB loops (particularly loops 5 and/or 7) are PorA loop 4 [or variable region 2] (see http//neisseria.org/nm/typing/porA/); PorA loop 5 (described in "Topology of outer membrane porins in pathogenic Neisseria spp", van der Ley, Poolman, etc.., Infect Immun 1991, 59, 2963-71; its sequence in PorA P1.7,16 (H44/76) loop 5 being: RHANVGRNAFELFLIGSGSDQAKGTDPLKNH); LbpA surface exposed loops 4, 5, 7, 10 and 12, corresponding to amino acids 210-342, 366-441, 542-600, 726-766 and 844-871, respectively, with 12 being preferred (sequence KGKNPDELAYLAGDQKRYSTKRASSSWST) [see Prinz et al. 1999 J Bacter. 181:4417 for further details on LbpA surface loops incorporated by reference herein]; NspA surface exposed loops 1, 2, 3 or 4, corresponding to amino acid sequence 25-54, 61-87, 103-129 and 149-164, respectively, preferably where loop 2 (e.g. FAVDYTRYKNYKAPSTDFKLYSIGASA) and/or 3 (e.g. ARLSLNRASVDLGGSDSFSQTSIGLGVL) is inserted (as these loops are quite small not all the FrpB loop 5 and/or 7 would be ideally removed to introduce these loops, and if both are to be introduced, it is preferred that they are introduced on loop 5 or 7 (or vice versa) in order to try to preserve the conformational epitope that exists between loops 2 and 3 of NspA) [see Vandeputte-Rutten et al 2003 JBC 278:24825 for more details on NspA loops, incorporated by reference herein]; any of the surface exposed loops of Omp85 (see Science 2003 299:262-5, and supporting online material Fig S4, incorporated by reference herein).

Alternatively peptide mimotopes of bacterial carbohydrate antigens may be incorporated into FrpB in the above way. Preferably mimotopes of Neisserial LOS are incorporated into loops 5 and/or 7 to advantageously stimulate an immune response against this important antigen without having its toxic effects in a vaccine. LOS mimotopes are well known in the art (see WO 02/28888 and references cited therein, incorporated by reference herein).

It will be appreciated that the mutant proteins of the present invention may be prepared using conventional protein engineering techniques. For example, polynucleotides of the invention or coding for a wild-type FrpB may be mutated using either random mutagenesis, for example using transposon mutagenesis, or site-directed mutagenesis.

A successful FrpB protein for the prevention of infection by Neisseria may require more than one of the following elements: generation of serum and/or mucosal antibodies to facilitate complement mediated killing of the Neisseria, and/or to enhance phagocytosis and microbial killing by leukocytes such as polymorphonuclear leukocytes, and/or to prevent attachment of the Neisseria to the host tissues; induction of a cell mediated immune response may also participate to protection.

The improvement of efficacy of a FrpB mutant of the invention can be evaluated by analyzing the induced immune response for serum and/or mucosal antibodies that have antiadherence, and/or opsonizing properties, and/or bactericidal activity, as described by others (McChesney D et al, Infect. Immun. 36: 1006, 1982; Boslego J et al: Efficacy trial of a purified gonococcl pilus vaccine, in Program and Abstracts of the 24th Interscience Conference on Antimicrobial Agents and Chemotherapy, Washington, American Society for Microbiology, 1984; Siegel M et al, J. Infect. Dis 145: 300, 1982; de la Pas, Microbiology, 141 (Pt4): 913-20, 1995).

It will be understood that protein sequences of the invention or for use in the invention are provided as guidelines and the invention is not limited to the particular sequences or fragments thereof given here but also include homologous sequences obtained from any source, for example related bacterial proteins, and synthetic peptides, as well as variants (particularly natural variants) or derivatives thereof. Loop sequences given are meant as guidelines, and it is envisaged that any loop sequence comprising an epitope present in the loops described above may be utilised.

Thus, the present invention encompasses variants, homologues or derivatives of the amino acid sequences of the present invention or for use in the invention, as well as variants, homologues or derivatives of the amino acid sequences.

In the context of the present invention, a homologous sequence is taken to include an amino acid sequence which is at least 60, 70, 80 or 90% identical, preferably at least 95 or 98% identical at the amino acid level. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al.,* 1999 *ibid -* Chapter 18), FASTA (Atschul et al., 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

Where a protein is specifically mentioned herein, it is preferably a reference to a full-length protein but it may also encompass antigenic fragments thereof (particularly in the context of subunit vaccines). Preferred fragments include those which include an epitope. Particularly preferred fragments include those with at least one surface loop. With respect to the mutants of the present invention this loop is other than loop 7 and loop 5. These fragments may contain or comprise at least 10 amino acids, preferably 20 amino acids, more preferably 30 amino acids, more preferably 40 amino acids or most preferably 50 amino acids, taken contiguously from the amino acid sequence of the protein. In addition, antigenic fragments denotes fragments that are immunologically reactive with antibodies generated against the Neisserial proteins or with antibodies generated by infection of a mammalian host with Neisseria. Antigenic fragments also includes fragments that when administered at an effective dose, elicit a protective immune response against Neisserial infection, more preferably it is protective against *N*. *meningitidis* and/or *N. gonorrhoeae* infection, most preferably it is protective against *N*. *meningitidis* serogroup B infection.

The present invention also includes variants of the proteins mentioned herein, that is proteins that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, 1-3, 1-2 or I amino acids are substituted, deleted, or added in any combination.

The FrpB protein has also been identified in *Neisseria gonorrhoeae* (US 6,265,567). The present invention may thus also be applied to the FrpB protein from other Neisseria.

The FrpB produced by the present invention , fragment or variant thereof, preferably is a product which displays at least some of the immunological activity of the wild type FrpB protein. Preferably it will show at least one of the following:
An ability to induce the production of antibodies which recognise the wild type FrpB (if necessary when the FrpB protein of the present invention is coupled to a carrier);
An ability to induce the production of antibodies that can protect against experimental infection; and
An ability to induce, when administered to an animal, the development of an immunological response that can protect against Neisserial infection such as *Neisseria meningitidis* or *Neisseria gonorrhoeae* infection.

Preferably the mutant protein of the present invention is cross-reactive and more preferably cross-protective.

The FrpB protein of the present invention is useful in prophylactic, therapeutic and diagnostic composition for preventing treating and diagnosing diseases caused by Neisseria, particularly *Neisseria meningitidis;* although it may also have similar applications in relation to, e.g. *Neisseria gonorrhoeae* or *Neisseria lactamica.*

Standard immunological techniques may be employed with the FrpB protein of the present invention in order to use it as an immunogen and as a vaccine. In particular, any suitable host may be injected with a pharmaceutically effective amount of the FrpB protein to generate monoclonal or polyclonal anti-FrpB antibodies or to induce the development of a protective immunological response against a *Neisseria* disease. Prior to administration, the FrpB protein may be formulated in a suitable vehicle, and thus we provide a pharmaceutical composition comprising a pharmaceutically effective amount of one or more proteins of the present invention. As used herein "pharmaceutically effective amount" refers to an amount of FrpB (or other proteins of the invention) protein that elicits a sufficient titre of antibodies to treat or prevent infection. The pharmaceutical composition of the present invention may also comprise other antigens useful in treating or preventing disease.

The FrpB protein of this invention may also form the basis of a diagnostic test for infection. For example, the present invention provides a method for detection of a Neisserial antigen in a biological sample containing or suspected of containing the Neisserial antigen comprising:
generating an anti-FrpB antibody using the protein of the present invention;
isolating the biological sample from a patient;
incubating the anti-FrpB antibody or a fragment thereof with the biological sample; and
detecting bound antibody or bound fragment.

This invention also provides a method for the detection of antibody specific to FrpB protein in a biological sample containing or suspected of containing said antibody comprising:
isolating the biological sample from a patient;
incubating the FrpB protein of the invention with the biological sample; and
detecting bound antigen.

This diagnostic test may take several forms including ELISA and a radioimmunoassay.

Further details on such applications are given below.

### Bactericidal assays

### SBA bactericidal assays of the invention

Cross-protection and an immunological response may be measured using a serum bactericidal assay (SBA) which is the most commonly agreed immunological marker to estimate the efficacy of a meningococcal vaccine (Perkins et al. J Infect Dis. 1998, 177:683-691). For example an improved response of the mutant FrpB protein over the wild type protein may be characterised by the SBA elicited by the mutant FrpB being at least 50%, two time, three times, preferably four times, five times, six times, seven times, eight times, nine times and most preferably ten times higher than the SBA elicited by the corresponding wild-type protein. Preferably SBA is measured against a homologous strain from which the antigens are derived and preferably also against a panel of heterologous strains. Thus, cross-protection may be measured if a satisfactory heterologous SBA is given against a panel of e.g. three unrelated strains. Satisfactory SBA can be ascertained by any known method. SBA can be carried out using sera obtained from animal models, or from human subjects.

A preferred method of conducting SBA with human sera is the following. A blood sample is taken prior to the first vaccination, two months after the second vaccination and one month after the third vaccination (three vaccinations in one year being a typical human primary vaccination schedule administered at, for instance, 0, 2 and 4 months, or 0, 1 and 6 months). Such human primary vaccination schedules can be carried out on infants under 1 year old (for instance at the same time as Hib vaccinations are carried out) or 2-4 year olds or adolescents may also be vaccinated to test SBA with such a primary vaccination schedule. A further blood sample may be taken 6 to 12 months after primary vaccination and one month after a booster dose, if applicable.

SBA will be satisfactory for an antigen or bleb preparation with homologous bactericidal activity if one month after the third vaccine dose (of the primary vaccination schedule) (in 2-4 year olds or adolescents, but preferably in infants in the first year of life) the percentage of subjects with a four-fold increase in terms of SBA (antibody dilution) titre (compared with pre-vaccination titre) against the strain of meningococcus from which the antigens of the invention were derived is greater than 30%, preferably greater than 40%, more preferably greater than 50%, and most preferably greater than 60% of the subjects.

Of course an antigen or bleb preparation with heterologous bactericidal activity can also constitute bleb preparation with homologous bactericidal activity if it can also elicit satisfactory SBA against the meningococcal strain from which it is derived.

SBA will be satisfactory for an antigen or bleb preparation with heterologous bactericidal activity if one month after the third vaccine dose (of the primary vaccination schedule) (in 2-4 year olds or adolescents, but preferably in infants in the first year of life) the percentage of subjects with a four-fold increase in terms of SBA (antibody dilution) titre (compared with pre-vaccination titre) against three heterologous strains of meningococcus is greater than 20%, preferably greater than 30%, more preferably greater than 35%, and most preferably greater than 40% of the subjects. Such a test is a good indication of whether the antigen or bleb preparation with heterologous bactericidal activity can induce cross-bactericidal antibodies against various meningococcal strains. The three heterologous strains should preferably have different electrophoretic type (ET)-complex or multilocus sequence typing (MLST) pattern (see Maiden et al. PNAS USA 1998, 95:3140-5) to each other and preferably to the strain from which the antigen or bleb preparation with heterologous bactericidal activity is made or derived. A skilled person will readily be able to determine three strains with different ET-complex which reflect the genetic diversity observed amongst meningococci, particularly amongst meningococcus type B strains that are recognised as being the cause of significant disease burden and/or that represent recognised MenB hyper-virulent lineages (see Maiden et al. *supra*). For instance three strains that could be used are the following: BZ10 (B:2b:P1.2) belonging to the A-4 cluster; B16B6 (B:2a:P1.2) belonging to the ET-37 complex; and H44/76 (B:15:P1.7,16) belonging to the ET-5 complex, or any other strains belonging to the same ET/Cluster. Such strains may be used for testing an antigen or bleb preparation with heterologous bactericidal activity made or derived from, for instance, meningococcal strain CU385 (B:4:P1.15) which belongs to the ET-5 complex. Another sample strain that could be used is from the Lineage 3 epidemic clone (e.g. NZ124 [B:4:P1.7,4]). Another ET-37 strain is NGP165 (B:2a:P1.2).

Processes for measuring SBA activity are known in the art. For instance a method that might be used is described in WO 99/09176. In general terms, a culture of the strain to be tested is grown (preferably in conditions of iron depletion - by addition of an iron chelator such as EDDA to the growth medium) in the log phase of growth. This can be suspended in a medium with BSA (such as Hanks medium with 0.3% BSA) in order to obtain a working cell suspension adjusted to approximately 20000 CFU/ml. A series of reaction mixes can be made mixing a series of two-fold dilutions of sera to be tested (preferably heat-inactivated at 56°C for 30 min) [for example in a 50µl/well volume] and the 20000 CFU/ml meningococcal strain suspension to be tested [for example in a 25µl/well volume]. The reaction vials should be incubated (e.g. 37°C for 15 minutes) and shaken (e.g. at 210 rpm). The final reaction mixture [for example in a 100µl volume] additionally contains a complement source [such as 25 % final volume of pretested baby rabbit serum], and is incubated as above [e.g. 37°C for 60 min]. A sterile polystyrene U-bottom 96-well microtiter plate can be used for this assay. A aliquot [e.g. 10 µl] can be taken from each well using a multichannel pipette, and dropped onto Mueller-Hinton agar plates (preferably containing 1 % Isovitalex and 1 % heat-inactivated Horse Serum) and incubated (for example for 18 hours at 37°C in 5 % CO₂). Preferably, individual colonies can be counted up to 80 CFU per aliquot. The following three test samples can be used as controls: buffer + bacteria + complement; buffer + bacteria + inactivated complement; serum + bacteria + inactivated complement. SBA titers can be straightforwardly calculated using a program which processes the data to give a measurement of the dilution which corresponds to 50 % of cell killing by a regression calculation.

### Animal protection assays

Alternatively, the synergistic response may be characterised by the efficacy of the combination of antigens in an animal protection assay. For instance, the assays described in example 12 or 13 may be used. Preferably the number of animals protected by the.combination of antigens is significantly improved compared with using the antigens by themselves, particularly at suboptimal doses of antigen.

### Adhesion blocking assay

Alternatively, the immunological response may be characterised by the efficacy of the combination of antigens in an adhesion blocking assay. Preferably the extent of blocking induced by antisera raised against the mutant FrpB is significantly improved compared with using antisera raised against the wild-type FrpB, particularly at suboptimal doses of antibody.

### Polynucleotide

The present invention also provides polynucleotides which code for the mutant proteins of the present invention, including variants, derivatives and homologs thereof. Polynucleotides of the invention may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or life span of polynucleotides of the invention.

In one embodiment the mutant proteins of the present invention are produced using any one of the following techniques: site-directed mutagenesis including cassette mutatgenesis, single primer extension, a PCR method of site-directed mutagensis for example the four-primer method of Higuchi et al (1988) Nucleic Acids Res. 16:7351-67, unidirectional deletion; random mutagenesis; and selection of mutant proteins by phage display.

The terms "variant", "homologue" or "derivative" in relation to the nucleotide sequence of the present invention include any substitution of, variation of, modification of, replacement of, deletion of or addition of one (or more) nucleic acid from or to the sequence providing the resultant nucleotide sequence codes for a mutant FrpB polypeptide.

As indicated above, with respect to sequence homology, preferably there is at least 75%, more preferably at least 85%, more preferably at least 90% homology (preferably identity) to the sequences shown in the sequence listing herein. More preferably there is at least 95%, more preferably at least 98%, homology (preferably identity). Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

Also useful in the present invention are nucleotide sequences that are capable of hybridising selectively to the sequences presented herein, or any variant, fragment or derivative thereof, or to the complement of any of the above. Nucleotide sequences are preferably at least 15 nucleotides in length, more preferably at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides useful in the invention capable of selectively hybridising to the nucleotide sequences presented herein, or to their complement, will be generally at least 70%, preferably at least 80 or 90% and more preferably at least 95% or 98% homologous to the corresponding nucleotide sequences presented herein over a region of at least 20, preferably at least 25 or 30, for instance at least 40, 60 or 100 or more contiguous nucleotides. Preferred polynucleotides useful in the invention will comprise regions homologous to nucleotides which code for conserved regions, preferably at least 80 or 90% and more preferably at least 95% homologous (preferably identical) to these regions.

The term "selectively hybridizable" means that the polynucleotide used as a probe is used under conditions where a target polynucleotide of the invention is found to hybridize to the probe at a level significantly above background. The background hybridization may occur because of other polynucleotides present, for example, in the cDNA or genomic DNA library being screening. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e.g. with ³²P.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

In a preferred aspect, the present disclosure covers nucleotide sequences that can hybridise to the nucleotide sequence of the present invention under stringent conditions (e.g. 65°C and 0.1xSSC {1xSSC = 0.15 M NaCl, 0.015 M Na₃ Citrate pH 7.0}).

Where the polynucleotide of the invention is double-stranded, both strands of the duplex, either individually or in combination, are encompassed by the present invention. Where the polynucleotide is single-stranded, it is to be understood that the complementary sequence of that polynucleotide is also included within the scope of the present invention.

Polynucleotides which are not 100% homologous to the sequences of the present invention but fall within the scope of the invention can be obtained in a number of ways. Other variants of the sequences described herein may be obtained for example by probing DNA libraries made from a range of individuals, for example individuals from different populations. In addition, other bacterial homologues may be obtained and such homologues and fragments thereof in general will be capable of selectively hybridising to the sequences shown in the sequence listing herein.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Polynucleotides of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein. Preferred fragments are less than 5000, 2000, 1000, 500 or 200 nucleotides in length.

Polynucleotides such as a DNA polynucleotides and probes useful in the invention may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using PCR (polymerase chain reaction) cloning techniques. This will involve making a pair of primers (e.g. of about 15 to 30 nucleotides) flanking a region of the sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

### Refolding Method

The present invention provides a method for promoting the correct folding/refolding of a mutant FrpB protein according to the present invention, which method involves the use of the detergent SB-12 in an alkaline refolding buffer.

Typically the method of the present invention is used to assist in refolding recombinantly produced FrpB, which is obtained in an unfolded or misfolded form. Thus, recombinantly produced proteins may be contacted with the refolding buffer to unfold, refold and/or reactivate recombinant proteins which are inactive due to misfolding and/or are unfolded as a result of their extraction from the host cells in which they were expressed (such as from bacterial inclusion bodies). Such a process may also be termed "reconditioning".

The method of the invention may be employed to maintain the folded conformation of FrpB, for example during storage, in order to increase shelf life. Under storage conditions, many proteins lose their activity, as a result of disruption of correct folding. The presence of the refolding buffer of the present invention, reduces or reverses the tendency of proteins to become unfolded and thus greatly increases the shelf life thereof.

The method of the invention may be used to promote the correct folding of FrpB which, through storage, exposure to denaturing conditions or otherwise, have become misfolded. Thus, the invention may be used to recondition FrpB. For example, FrpB in need of reconditioning may be contacted with the refolding buffer in accordance with the invention.

The present invention also provides a method for altering the structure of an FrpB protein. Structural alterations include folding, unfolding and refolding. The effect of the alterations is preferably to improve the yield, specific activity and/or quality of the molecule. This may typically be achieved by resolubilising, reconditioning and/or reactivating incorrectly folded molecules post-synthesis.

The terms "reconditioning" and "reactivating" thus encompass *in vitro* procedures. Particular examples of *in vitro* procedures may include processing proteins that have been solubilised from cell extracts (such as inclusion bodies) using strong denaturants such as urea or guanidium chloride.

The terms "refold", "reactivate" and "recondition" are not intended as being mutually exclusive. For example, an inactive protein, perhaps denatured using urea, may have an unfolded structure. This inactive protein may then be refolded with a refolding buffer of the invention thereby reactivating it. In some circumstances there may be an increase in the specific activity of the refolded/reactivated protein compared to the protein prior to inactivation/denaturation: this is termed "reconditioning".

The molecule is typically an unfolded or misfolded protein which is in need of folding. Alternatively, however, it may be a folded protein which is to be maintained in a folded state.

The invention envisages at least two situations. A first situation is one in which the protein to be folded is in an unfolded or misfolded state, or both. In this case, its correct folding is promoted by the method of the invention. A second situation is one in which the protein is substantially already in its correctly folded state, that is all or most of it is folded correctly or nearly correctly. In this case, the method of the invention serves to maintain the folded state of the protein by affecting the folded/unfolded equilibrium so as to favour the folded state. This prevents loss of activity of an already substantially correctly folded protein. These, and other, eventualities are covered by the reference to "promoting" the folding of the protein.

As used herein, a protein may be unfolded when at least part of it has not yet acquired its correct or desired secondary or tertiary structure. A protein is misfolded when it has acquired at least partially incorrect or undesired secondary or tertiary structure. Techniques are known in the art for assessing protein structure - such as circular dichroism.

The refolding buffer of the present invention comprises 3-dimethyldodecylammoniopropanesulfonate (also referred to as N-Dodecyl-N,N-dimethyl-3-ammonio-1-propanesulfonate) and referred to herein as "SB-12". SB-12 is also referred to as Zwittergent 3-12. SB-12 is available from commercial sources, such as Fluka AG. Although SB-12 is highly preferred, the use of other salts of SB12 may also be used, as well as derivatives of SB-12 or molecules related to SB-12. Whilst not wishing to be bound by any theory, we believe the dilution step in SB-12 (or the presence of SB-12 in general) creates a hydrophobic environment for the protein, which is similar to the protein's natural *in vivo* environment.

SB-12 is a detergent and the concentration of SB-12 should be at least about 0.2% (w/v), since this is the concentration generally required for micelle formation. Thus, the concentration of SB-12 may be about 0.2% to about 5.0%, about 0.3% to about 4.0%, about 0.4% to about 3.0%, or about 0.5% to about 2.0%. Preferably the concentration is about 0.2%, 0.3%, 0.4%, 0.5%, 0.6%, 0.7%, 0.8%, 0.9% or 1.0%. In an especially preferred embodiment 0.5% SB-12 is used. By "about" it is preferably meant +/- 10% of the value of the figure quoted, but is most preferably the exact figure quoted.

In one embodiment the SB-12 is purified. Conveniently the SB-12 may be purified before use by passing a concentrated solution of SB-12 over an Al₂O₃ chromatography column, e.g. using in methanol/chloroform (1:1), but any suitable method may be used for purifying the SB-12.

We have found that the dilution of the denatured protein should be carried out in an alkaline environment to maximise the efficiency of refolding. Preferably the pH of the refolding buffer is about 11.0. Preferably the alkaline environment is obtained by the use of ethanolamine. In this preferred embodiment the refolding buffer comprises SB-12 and ethanolamine. Conveniently 20mM ethanolamine is used, but other concentrations, such as 10, 30, 40 or 50mM, may be useful.

We have also found that preferably the dilution of the protein may also be carried out in the presence of guanidium hydrochloride, NaCl (such as 0.4M NaCl) or urea (such as 8M urea), with guanidium hydrochloride being preferred. Conveniently 0.4M guanidium hydrochloride is used, but other concentrations may be useful. When one of the aforementioned components is used during solubilisation, it will be appreciated that there may be no need to add the component again during the refolding stage. Most preferably, however, if the protein of the invention is made via inclusion bodies, it is preferably to solubilise the inclusion body with Guanidinium hydrochloride; in such case the refolding buffer need not contain any denaturant, and the refolded solution may then exchange its buffer for 0.3% SB-12 at neutral pH (e.g. Hepes pH 7.5) to stabilise the folded protein.

We have found that a 1:10 dilution of the FrpB in the refolding buffer is preferred, but other ratios such as 1:20 may be used. Any suitable dilution to effect refolding may be employed readily by a skilled person.

The FrpB to be processed by the method of the invention is typically obtained from cell extracts of host cells expressing recombinant FrpB. Host cells include prokaryotes such as *E*. *coli,* yeast and insect cells (the baculovirus system is capable of very high level protein expression). Expression of the FrpB in the host cell is preferably at high levels to maximise yield. Further details on the expression of recombinant FrpB are given below.

We have found that the present invention is most efficient when used to refold a mutant mature FrpB protein. By "mature" we include a protein without a leader or secretory sequence, a pre-, or pro- or prepro-protein sequence, and also a protein, but the method can be used to refold proteins with such sequences. It is common during conventional purification techniques to make use of a marker sequence that facilitates purification, such as a hexa-histidine peptide, as provided in the pQE vector (Qiagen, Inc.) and described in Gentz et al., Proc. Natl. Acad. Sci., USA 86: 821-824 (1989), or an HA peptide tag (Wilson et al., Cell 37: 767 (1984). The process of the present invention can be used when such marker sequences are not present.

It is likely that a substantial proportion of the FrpB will be insoluble and consequently techniques to solubilise normally insoluble components of the cell extracts (such as inclusion bodies) to maximise extraction of the FrpB will typically be employed. Any conventional technique for preparation and extraction of the FrpB proteins from inclusion bodies and their subsequent solubilisation may be employed. Such techniques are described for example in "Current Protocols in Protein Science" published by JA Wiley & Sons. Such techniques generally include:
Cell Lysis - using, for example, a French press or sonication, to release inclusion bodies.
Typically the cells are placed in a cold buffer such as a TE buffer prior to lysis.
Sonication may be carried out using a Branson sonifier. Sonification may take place in the presence of a detergent, e.g. Brij or Triton. The inclusion bodies in the cell lysate may then be pelleted using low-speed centrifugation. The cells may be pretreated with lysozyme prior to lysis. The purpose of the pretreatment is to aid removal of the peptidoglycan and outer membrane protein contaminants during the washing steps. The lysed cells may be clarified by centrifugation and the supernatant discarded.

Inclusion Body Washing - to remove cell wall and other outer membrane components contaminants from the inclusion bodies recovered from cell lysates. Typically the pellet is resuspended in a wash buffer containing, e.g. buffer such as TE buffer and/or a detergent such as Triton. The suspension may then be resuspended and the supernatant discarded. This process may be repeated until the supernatant is clear. If required, the washed pellets can be frozen for storage.

The amount of recombinant protein in the washed pellet may be estimated using the following guidelines: (1) an expression level of 1% corresponds to ~1mg recombinant protein per 1g wet cells. (2) The recovery of highly aggregated recombinant protein in the washed pellets is ~75% that originally present in the cells. (3) About 60% of the total washed pellet protein is recombinant-derived. The total amount of recombinant protein can be directly determined be measuring the total protein concentration or by analysing the washed pellets via SDS-PAGE to determine the proportions of the protein constituents.

Protein solubilisation - the extracted protein is then extracted from the washed pellet and defolded using a denaturant which disassociates protein-protein interactions and unfolds the protein so that it consists of unfolded monomers. Denaturants include guanidine:HCl (such as 6M guanidine:HCl) and/or urea (such as 8M urea). In one embodiment of the present invention we conveniently use 8M urea for solubilisation. In a preferred embodiment 6M guanidine:HCl is used. Residual insoluble material and materials can be typically removed by ultracentrifugation (e.g. 100,000 g x for I hr.). The extract may be stored by freezing following pelleting.

Solubilised cell extracts may optionally be partially purified by, for example, a variety of affinity chromatography techniques prior to contacting with the refolding buffer according to the method of the invention.

The solubilised cell extract is then diluted into the refolding buffer in accordance with the present invention. It is typically, but not necessarily, left stirring at room temperature overnight.

Thus, the starting material for the refolding/reconditioning method of the invention is typically denatured proteins in solutions of agents such as urea/guanidium chloride. Alternatively, or in addition, soluble protein samples may be specifically denatured by the addition of appropriate denaturing agents prior to refolding.

The method of the invention may also employ the use of molecular chaperones.

Chaperones, including chaperonins, are proteins which promote protein folding by non-enzymatic means, in that they do not catalyse the chemical modification of any structures in folding proteins, but promote the correct folding of proteins by facilitating correct structural alignment thereof. Molecular chaperones are well known in the art, several families thereof being characterised. The invention may employ any molecular chaperone molecule, which term includes, for example, the molecular chaperones selected from the following non-exhaustive group:
p90 Calnexin, HSP family, HSP70 family, DNA K, DNAJ, HSP60 family (GroEL),
ER-associated chaperones, HSP90, Hsc70, sHsps; SecA; SecB, Trigger factor, zebrafish hsp 47, 70 and 90, HSP 47, GRP 94, Cpn 10, BiP, GRP 78, Clp, FtsH, Ig invariant chain,
mitochondrial hsp70, EBP, mitochondrial m-AAA, Yeast Ydj1, Hsp104, ApoE, Syc, Hip,
TriC family, CCT, PapD and calmodulin (see WO99/05163 for references).

The method of the present invention may also make use of a foldase. In general terms, a foldase is an enzyme which participates in the promotion of protein folding through its enzymatic activity to catalyse the rearrangement or isomerisation of bonds in the folding protein. They are thus distinct from a molecular chaperone, which bind to proteins in unstable or non-native structural states and promote correct folding without enzymatic catalysis of bond rearrangement. Many classes of foldase are known, and they are common to animals, plants and bacteria. They include peptidyl prolyl isomerases and thiol/disulphide oxidoreductases. The invention may employ any of the foldases which are capable of promoting protein folding through covalent bond rearrangement.

At the end of the refolding/reconditioning process, the refolded FrpB may be desalted by dialysis against a suitable storage buffer and/or the use of a desalting column into a suitable storage buffer. Suitable buffers include 25 mM sodium phosphate, 150 mM NaCl and 0.1% PEG 6000 (pH 7.4).

In one embodiment of the present invention, following removal of urea and guanidium hydrochloride, the buffer may be changed from ethanolamine pH11 to Tris-HCl pH7.2 containing the Zwittergent 3-12.

### Vectors, Host Cells, Expression Systems

The invention may employ vectors that comprise a polynucleotide which codes for at least an FrpB protein and may comprise polynucleotides of the present invention which code for a mutant FrpB protein of the present invention, host cells that are genetically engineered with vectors of the invention and the production of FrpB proteins by recombinant techniques. Cell-free translation systems can also be employed to produce such proteins using RNAs derived from DNA constructs.

Recombinant proteins of the present invention may be prepared by processes well known to those skilled in the art from genetically engineered host cells comprising expression systems.

For recombinant production of the proteins of the invention, host cells can be genetically engineered to incorporate expression systems or portions thereof or polynucleotides of the invention. Introduction of a polynucleotide into the host cell can be effected by methods described in many standard laboratory manuals, such as Davis, et al., BASIC METHODS IN MOLECULAR BIOLOGY, (1986) and Sambrook, et al., MOLECULAR CLONING: A LABORATORY MANUAL, 2nd Ed., Cold Spring Harbor Laboratory Press, Cold Spring Harbor, N.Y. (1989), such as, calcium phosphate transfection, DEAE-dextran mediated transfection, transvection, microinjection, cationic lipid-mediated transfection, electroporation, transduction, scrape loading, ballistic introduction and infection.

Representative examples of appropriate hosts include bacterial cells, such as cells of streptococci, staphylococci, enterococci, *E. coli,* streptomyces, cyanobacteria, *Bacillus subtilis, Moraxella catarrhalis, Haemophilus influenzae* and *Neisseria meningitidis;* fungal cells, such as cells of a yeast, *Kluveromyces, Saccharomyces,* a basidiomycete, *Candida albicans* and *Aspergillus;* insect cells such as cells of *Drosophila* S2 and *Spodoptera* Sf9; animal cells such as CHO, COS, HeLa, C127, 3T3, BHK, 293, CV-1 and Bowes melanoma cells; and plant cells, such as cells of a gymnosperm or angiosperm.

A great variety of expression systems can be used to produce the proteins of the invention. Such vectors include, among others, chromosomal-, episomal- and virus-derived vectors, for example, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses, picomaviruses, retroviruses, and alphaviruses and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a protein in a host may be used for expression in this regard. The appropriate DNA sequence may be inserted into the expression system by any of a variety of well-known and routine techniques, such as, for example, those set forth in Sambrook *et al., MOLECULAR CLONING, A LABORATORY MANUAL, (supra).*

In recombinant expression systems in eukaryotes, for secretion of a translated protein into the lumen of the endoplasmic reticulum, into the periplasmic space or into the extracellular environment, appropriate secretion signals may be incorporated into the expressed protein. These signals may be endogenous to the protein or they may be heterologous signals.
Proteins of the present invention can be recovered and purified from recombinant cell cultures by the method of the present invention.

### Antibodies

The proteins of the invention can be used as immunogens to produce antibodies immunospecific for such proteins.

In an embodiment of the invention there is provided antibodies against the FrpB protein of the invention.

Antibodies generated against the proteins of the invention can be obtained by administering the proteins of the invention, or epitope-bearing fragments of either or both, analogues of either or both, to an animal, preferably a nonhuman, using routine protocols. For preparation of monoclonal antibodies, any technique known in the art that provides antibodies produced by continuous cell line cultures can be used. Examples include various techniques, such as those in Kohler, G. and Milstein, C., Nature 256: 495-497 (1975); Kozbor et al., Immunology Today 4: 72 (1983); Cole et al., pg. 77-96 in MONOCLONAL ANTIBODIES AND CANCER THERAPY, Alan R. Liss, Inc. (1985).

Techniques for the production of single chain antibodies (U.S. Patent No. 4,946,778) can be adapted to produce single chain antibodies to proteins of this invention. Also, transgenic mice, or other organisms or animals, such as other mammals, may be used to express humanized antibodies immunospecific to the proteins of the invention.

Alternatively, phage display technology may be utilized to select antibody genes with binding activities towards a protein of the invention either from repertoires of PCR amplified v-genes of lymphocytes from humans screened for possessing anti-FrpB or from naive libraries (McCafferty, et al., (1990), Nature 348, 552-554; Marks, et al., (1992) Biotechnology 10, 779-783). The affinity of these antibodies can also be improved by, for example, chain shuffling (Clackson et al., (1991) Nature 352: 628).

The above-described antibodies may be employed to isolate or to identify clones expressing FrpB proteins of the invention to purify the proteins or polynucleotides by, for example, affinity chromatography.

Thus, among others, antibodies against the FrpB protein of the invention may be employed to treat infections, particularly bacterial infections.

Preferably, the antibody or variant thereof is modified to make it less immunogenic in the individual. For example, if the individual is human the antibody may most preferably be "humanized," where the complimentarity determining region or regions of the hybridoma-derived antibody has been transplanted into a human monoclonal antibody, for example as described in Jones et al. (1986), Nature 321, 522-525 or Tempest et al., (1991) Biotechnology 9, 266-273.

A protein of the present invention can be administered to a recipient who then acts as a source of immune globulin, produced in response to challenge from the specific vaccine. A subject thus treated would donate plasma from which hyperimmune globulin would be obtained via conventional plasma fractionation methodology. The hyperimmune globulin would be administered to another subject in order to impart resistance against or treat Neisserial infection. Hyperimmune globulins of the invention are particularly useful for treatment or prevention of Neisserial disease in infants, immune compromised individuals or where treatment is required and there is no time for the individual to produce antibodies in response to vaccination.

An additional aspect of the invention is a pharmaceutical composition comprising a monoclonal antibody (or fragments thereof; preferably human or humanised) reactive against the pharmaceutical composition of the invention, which could be used to treat or prevent infection by Gram negative bacteria, preferably Neisseria, more preferably *Neisseria meningitidis* or *Neisseria gonorrhoeae* and most preferably *Neisseria meningitidis* serogroup B.

Such pharmaceutical compositions comprise monoclonal antibodies that can be whole immunoglobulins of any class e.g. IgG, IgM, IgA, IgD or IgE, chimeric antibodies or hybrid antibodies with specificity to two or more antigens of the invention. They may also be fragments e.g. F(ab')2, Fab', Fab, Fv and the like including hybrid fragments.

Methods of making monoclonal antibodies are well known in the art and can include the fusion of splenocytes with myeloma cells (Kohler and Milstein 1975 Nature 256; 495; Antibodies - a laboratory manual Harlow and Lane 1988). Alternatively, monoclonal Fv fragments can be obtained by screening a suitable phage display library (Vaughan TJ et al 1998 Nature Biotechnology 16; 535). Monoclonal antibodies may be humanised or part humanised by known methods.

### Antagonists and Agonists - Assays and Molecules

The refolded proteins of the invention may also be used to assess the binding of small molecule substrates and ligands in, for example, cell-free preparations, chemical libraries, and natural product mixtures. These substrates and ligands may be natural substrates and ligands or may be structural or functional mimetics. See, e.g., Coligan et al., Current Protocols in Immunology 1 (2): Chapter 5 (1991).

The screening methods may simply measure the binding of a candidate compound to the protein. Alternatively, the screening method may involve competition with a labeled competitor. The screening methods may simply comprise the steps of mixing a candidate compound with a solution containing a protein of the present invention, to form a mixture, measuring FrpB protein activity in the mixture, and comparing the FrpB protein activity of the mixture to a standard.

The polynucleotides, proteins and antibodies that bind to and/or interact with a protein of the present invention may also be used to configure screening methods for detecting the effect of added compounds on the production of mRNA and/or protein in cells. For example, an ELISA assay may be constructed for measuring secreted or cell associated levels of protein using monoclonal and polyclonal antibodies by standard methods known in the art. This can be used to discover agents which may inhibit or enhance the production of protein (also called antagonist or agonist, respectively) from suitably manipulated cells or tissues.

The disclosure also provides a method of screening compounds to identify those which enhance (agonist) or block (antagonist) the action of FrpB proteins and polynucleotides, particularly those compounds that are bacteristatic and/or bactericidal. The method of screening may involve high-throughput techniques. For example, to screen for agonists or antagonists, a synthetic reaction mix comprising FrpB protein and a labeled substrate or ligand of such protein is incubated in the absence or the presence of a candidate molecule that may be a FrpB agonist or antagonist. The ability of the candidate molecule to agonize or antagonize the FrpB protein is reflected in decreased binding of the labeled ligand or decreased production of product from such substrate. Molecules that bind gratuitously, i.e., without inducing the effects of FrpB protein are most likely to be good antagonists. Reporter systems that may be useful in this regard include but are not limited to colorimetric, labeled substrate converted into product, a reporter gene that is responsive to changes in FrpB protein activity, and binding assays known in the art.

Another example of an assay for FrpB agonists is a competitive assay that combines FrpB and a potential agonist with FrpB-binding molecules, recombinant FrpB binding molecules, natural substrates or ligands, or substrate or ligand mimetics, under appropriate conditions for a competitive inhibition assay. FrpB can be labeled, such as by radioactivity or a colorimetric compound, such that the number of FrpB molecules bound to a binding molecule or converted to product can be determined accurately to assess the effectiveness of the potential antagonist.

Potential antagonists include, among others, small organic molecules, peptides, proteins and antibodies that bind to a polynucleotide and/or protein of the invention and thereby inhibit or extinguish its activity or expression. Potential antagonists also may be small organic molecules, a peptide, a protein such as a closely related protein or antibody that binds the same sites on a binding molecule, such as a binding molecule.

Potential antagonists include a small molecule that binds to and occupies the binding site of the protein thereby preventing binding to cellular binding molecules, such that normal biological activity is prevented. Examples of small molecules include but are not limited to small organic molecules, peptides or peptide-like molecules. Other potential antagonists include antisense molecules (see Okano, J. Neurochem. 56: 560 (1991); OLIGODEOXYNUCLEOTIDES AS ANTISENSE INHIBITORS OF GENE EXPRESSION, CRC Press, Boca Raton, FL (1988), for a description of these molecules). Potential antagonists include compounds related to and variants of FrpB.

The invention also provides the use of the protein, polynucleotide, agonist or antagonist of the invention to interfere with the initial physical interaction between a pathogen or pathogens and a eukaryotic, preferably mammalian, host responsible for sequelae of infection. In particular, the molecules of the invention may be used: in the prevention of adhesion of bacteria, in particular gram positive and/or gram negative bacteria, to eukaryotic, preferably mammalian, extracellular matrix proteins on in-dwelling devices or to extracellular matrix proteins in wounds; to block bacterial adhesion between eukaryotic, preferably mammalian, extracellular matrix proteins and bacterial FrpB proteins that mediate tissue damage and/or; to block the normal progression of pathogenesis in infections initiated other than by the implantation of in-dwelling devices or by other surgical techniques.

There is also described FrpB agonists and antagonists, preferably bacteristatic or bactericidal agonists and antagonists.

The antagonists and agonists of the invention may be employed, for instance, to prevent, inhibit and/or treat diseases.

### Vaccines

Another aspect of the invention relates to a method for inducing an immunological response in an individual, particularly a mammal, preferably humans, which comprises inoculating the individual with FrpB protein of the present invention, or a fragment or variant thereof, adequate to produce antibody and/ or T cell immune response to protect (or treat) said individual from infection, particularly bacterial infection and most particularly *Neisseria meningitidis* infection. Also provided are methods whereby such immunological response slows bacterial replication.

A further aspect of the invention relates to an immunological composition that when introduced into an individual, preferably a human, capable of having induced within it an immunological response, induces an immunological response in such individual to a FrpB protein of the present invention, wherein the composition comprises a recombinant FrpB of the invention. The immunological response may be used therapeutically or prophylactically and may take the form of antibody immunity and/or cellular immunity, such as cellular immunity arising from CTL or CD4+ T cells.

A FrpB protein, variant or a fragment thereof may be fused with co-protein or chemical moiety which may or may not by itself produce antibodies, but which is capable of producing a fused or modified protein which will have antigenic and/or immunogenic properties, and preferably protective properties, and optionally may stabilise the FrpB, or render it easier to purify. The co-protein may act as an adjuvant in the sense of providing a generalized stimulation of the immune system of the organism receiving the protein.

Also provided by this invention are compositions, particularly vaccine compositions, and methods comprising the proteins of the invention and immunostimulatory DNA sequences, such as those described in Sato, Y. et al. Science 273: 352 (1996).

The invention thus also includes a vaccine formulation which comprises an immunogenic protein of the invention or a fragment or a variant thereof, together with a suitable carrier, such as a pharmaceutically acceptable carrier. Since the proteins may be broken down in the stomach, each is preferably administered parenterally, including, for example, administration that is subcutaneous, intramuscular, intravenous, or intradermal. Formulations suitable for parenteral administration include aqueous and non-aqueous sterile injection solutions which may contain anti-oxidants, buffers, bacteristatic compounds and solutes which render the formulation isotonic with the bodily fluid, preferably the blood, of the individual; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The formulation may also be administered mucosally, e.g. intranasally.

The vaccine formulation of the invention may also include adjuvant systems for enhancing the immunogenicity of the formulation. Typically aluminium phosphate or aluminium hydroxide may be used. Preferably the adjuvant system raises preferentially a TH 1 type of response.

An immune response may be broadly distinguished into two extreme categories, being a humoral or cell mediated immune responses (traditionally characterised by antibody and cellular effector mechanisms of protection respectively). These categories of response have been termed TH1-type responses (cell-mediated response), and TH2-type immune responses (humoral response).

Extreme TH1-type immune responses may be characterised by the generation of antigen specific, haplotype restricted cytotoxic T lymphocytes, and natural killer cell responses. In mice TH1-type responses are often characterised by the generation of antibodies of the IgG2a subtype, whilst in the human these correspond to IgG1 type antibodies. TH2-type immune responses are characterised by the generation of a broad range of immunoglobulin isotypes including in mice IgG1, IgA, and IgM.

It can be considered that the driving force behind the development of these two types of immune responses are cytokines. High levels of TH1-type cytokines tend to favour the induction of cell mediated immune responses to the given antigen, whilst high levels of TH2-type cytokines tend to favour the induction of humoral immune responses to the antigen.

The distinction of TH 1 and TH2-type immune responses is not absolute. In reality an individual will support an immune response which is described as being predominantly TH1 or predominantly TH2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, TH1-type responses are associated with the production of the INF-γ and IL-2 cytokines by T-lymphocytes. Other cytokines often directly associated with the induction of TH1-type immune responses are not produced by T-cells, such as IL-12. In contrast, TH2- type responses are associated with the secretion of IL-4, IL-5, IL-6 and IL-13.

It is known that certain vaccine adjuvants are particularly suited to the stimulation of either TH1 or TH2 - type cytokine responses. Traditionally the best indicators of the TH 1:TH2 balance of the immune response after a vaccination or infection includes direct measurement of the production of TH 1 or TH2 cytokines by T lymphocytes *in vitro* after restimulation with antigen, and/or the measurement of the IgG1:IgG2a ratio of antigen specific antibody responses.

Thus, a TH1-type adjuvant is one which preferentially stimulates isolated T-cell populations to produce high levels of TH1-type cytokines when re-stimulated with antigen *in vitro,* and promotes development of both CD8+ cytotoxic T lymphocytes and antigen specific immunoglobulin responses associated with TH1-type isotype.

Adjuvants which are capable of preferential stimulation of the TH I cell response are described in International Patent Application No. WO 94/00153 and WO 95/17209.

3 De-O-acylated monophosphoryl lipid A (3D-MPL) is one such adjuvant, and is preferred. This is known from GB 2220211 (Ribi). Chemically it is a mixture of 3 De-O-acylated monophosphoryl lipid A with 4, 5 or 6 acylated chains and is manufactured by Ribi Immunochem, Montana. A preferred form of 3 De-O-acylated monophosphoryl lipid A is disclosed in European Patent 0 689 454 B1 (SmithKline Beecham Biologicals SA). Altenatively, other non-toxic derivatives of LPS may be used.

Preferably, the particles of 3D-MPL are small enough to be sterile filtered through a 0.22micron membrane (European Patent number 0 689 454). 3D-MPL will be present in the range of 10µg - 100µg preferably 25-50µg per dose wherein the antigen will typically be present in a range 2-50µg per dose.

Another preferred adjuvant comprises QS21, an Hplc purified non-toxic fraction derived from the bark of Quillaja Saponaria Molina. Optionally this may be admixed with 3 De-O-acylated monophosphoryl lipid A (3D-MPL), or other non-toxic LPS derivative, optionally together with a carrier.

The method of production of QS21 is disclosed in US patent No. 5,057,540.

Non-reactogenic adjuvant formulations containing QS21 have been described previously (WO 96/33739). Such formulations comprising QS21 and cholesterol have been shown to be successful TH 1 stimulating adjuvants when formulated together with an antigen.

Further adjuvants which are preferential stimulators of TH1 cell response include immunomodulatory oligonucleotides, for example unmethylated CpG sequences as disclosed in WO 96/02555.

Combinations of different TH 1 stimulating adjuvants, such as those mentioned hereinabove, are also contemplated as providing an adjuvant which is a preferential stimulator of TH1 cell response. For example, QS21 can be formulated together with 3D-MPL. The ratio of QS21 : 3D-MPL will typically be in the order of I : 10 to 10 : 1; preferably 1:5 to 5 : 1 and often substantially I : 1. The preferred range for optimal synergy is 2.5 : 1 to 1 : 1 3D-MPL: QS21.

Preferably a carrier is also present in the vaccine composition according to the invention. The carrier may be an oil in water emulsion, or an aluminium salt, such as aluminium phosphate or aluminium hydroxide.

A preferred oil-in-water emulsion comprises a metabolisible oil, such as squalene, alpha tocopherol and Tween 80. In a particularly preferred aspect the antigens in the vaccine composition according to the invention are combined with QS21 and 3D-MPL in such an emulsion. Additionally the oil in water emulsion may contain span 85 and/or lecithin and/or tricaprylin.

Typically for human administration QS21 and 3D-MPL will be present in a vaccine in the range of 1 µg - 200µg, such as 10-100 µg, preferably 10µg - 50µg per dose. Typically the oil in water will comprise from 2 to 10% squalene, from 2 to 10% alpha tocopherol and from 0.3 to 3% tween 80. Preferably the ratio of squalene: alpha tocopherol is equal to or less than 1 as this provides a more stable emulsion. Span 85 may also be present at a level of 1%. In some cases it may be advantageous that the vaccines of the present invention will further contain a stabiliser.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline.

A particularly potent adjuvant formulation involving QS21, 3D-MPL and tocopherol in an oil in water emulsion is described in WO 95/17210.

The present invention also provides a polyvalent vaccine composition comprising a vaccine formulation of the invention in combination with other antigens, in particular antigens useful for treating cancers, autoimmune diseases and related conditions. Such a polyvalent vaccine composition may include a TH-1 inducing adjuvant as hereinbefore described.

### Subunit composition

The composition of the present invention may be in the form of a subunit composition. When the mutant FrpB protein of the invention is in a folded conformation it is preferably in the form of such a subunit vaccine. Subunit compositions are compositions in which the components have been isolated and purified to at least 50%, preferably at least 60%, 70%, 80%, 90% pure before mixing the components to form the antigenic composition.

Subunit compositions may comprise aqueous solutions of water soluble proteins. They may comprise detergent, preferably non-ionic, zwitterionic or ionic detergent in order to solubilise hydrophobic portions of the antigens. They may comprise lipids so that liposome structures could be formed, allowing presentation of antigens with a structure that spans a lipid membrane. Further details on compositions is given below.

### Outer membrane vesicle preparations

The composition of the present invention may also be in the form of an outer membrane vesicle preparation, when the composition comprises the mutant FrpB protein of the present invention.

*N. meningitidis* serogroup B (menB) excretes outer membrane blebs in sufficient quantities to allow their manufacture on an industrial scale. An outer membrane vesicles may also be prepared via the process of detergent extraction of the bacterial cells (see for example EP 11243).

The immunogenic composition of the invention may also comprise an outer membrane vesicle preparation having at least two antigens which have been recombinantly upregulated. Examples of antigens which would be upregulated in such a outer membrane vesicle preparation in addition to the FrpB protein of the present invention include; NspA, Hsf, Hap, OMP85, TbpA (high), TbpA (low), LbpA, TbpB, LbpB, PilQ and PldA. Such preparations would optionally also comprise either or both of LPS immunotype L2 and LPS immunotype L3.

The manufacture of bleb preparations from Neisserial strains may be achieved by any of the methods well known to a skilled person. Preferably the methods disclosed in EP 301992, US 5,597,572, EP 11243 or US 4,271,147, Frederikson et al. (NIPH Annals [1991], 14:67-80), Zollinger et al. (J. Clin. Invest. [1979], 63:836-848), Saunders et al. (Infect. Immun. [1999], 67:113-119), Drabick et al. (Vaccine [2000], 18:160-172) or WO 01/09350 (Example 8) are used. In general, OMVs are extracted with a detergent, preferably deoxycholate, and nucleic acids are optionally removed enzymatically. Purification is achieved by ultracentrifugation optionally followed by size exclusion chromatography. If 2 or more different blebs of the invention are included, they may be combined in a single container to form a multivalent preparation of the invention (although a preparation is also considered multivalent if the different blebs of the invention are separate compositions in separate containers which are administered at the same time [the same visit to a practitioner] to a host). OMV preparations are usually sterilised by filtration through a 0.2 µm filter, and are preferably stored in a sucrose solution (e.g. 3%) which is known to stabilise the bleb preparations.

Upregulation of proteins within outer membrane vesicle preparations may be achieved by insertion of an extra copy of a gene into the Neisserial strain from which the OMV preparation is derived. Alternatively, the promoter of a gene can be exchanged for a stronger promoter in the Neisserial strain from which the OMV preparation is derived. Such techniques are described in WO01/09350. If an extra copy of the gene is introduced, it too can have a non-native strong promoter attached for overexpression. Upregulation of a protein will lead to a higher level of protein being present in OMV compared to the level of protein present in OMV derived from unmodified *N. meningitidis* (for instance strain H44/76). Preferably the level will be 1.5, 2, 3, 4, 5, 7, 10 or 20 times higher.

Where LPS is intended to be an additional antigen in the OMV, a protocol using a low concentration of extracting detergent ( for example deoxycholate or DOC) may preferably be used in the OMV preparation method so as to preserve high levels of bound LPS whilst removing particularly toxic, poorly bound LPS. The concentration of DOC used is preferably 0-0.3% DOC, more preferably 0.05%-0.2% DOC, most preferably around 0.1% DOC.

"Stronger promoter sequence" refers to a regulatory control element that increases transcription for a gene encoding antigen of interest.

"Upregulating expression" refers to any means to enhance the expression of an antigen of interest, relative to that of the non-modified (i.e., naturally occurring) bleb. It is understood that the amount of 'upregulation' will vary depending on the particular antigen of interest but will not exceed an amount that will disrupt the membrane integrity of the bleb. Upregulation of an antigen refers to expression that is at least 10% higher than that of the non-modified bleb. Preferably it is at least 50% higher. More preferably it is at least 100% (2 fold) higher. Alternatively or additionally, upregulating expression may refer to rendering expression non-conditional on metabolic or nutritional changes, particularly in the case of FrpB, TbpA, TbpB, LbpA and LbpB. In general where FrpB is overexpressed in an bleb this may be done by removing regulatory sequences from the promoter, or by replacement of the promoter for a strong, non-regulated promoter such as PorA.

Again for the purpose of clarity, the terms 'engineering a bacterial strain to produce less of said antigen' or down regulation refers to any means to reduce the expression of an antigen (or the expression of a functional gene product) of interest, relative to that of the non-modified (i.e., naturally occurring bleb), preferably by deletion, such that expression is at least 10% lower than that of the non-modified bleb. Preferably it is at least 50% lower and most preferably completely absent. If the down regulated protein is an enzyme or a functional protein, the downregulation may be achieved by introducing one or more mutations resulting in a 10%, 20%, 50%, 80% or preferably a 100% reduction in enzymatic or functional activity.

The engineering steps required to modulate the expression of Neisserial proteins can be carried out in a variety of ways known to the skilled person. For instance, sequences (e.g. promoters or open reading frames) can be inserted, and promoters/genes can be disrupted by the technique of transposon insertion. For instance, for upregulating a gene's expression, a strong promoter could be inserted via a transposon up to 2 kb upstream of the gene's initiation codon (more preferably 200-600 bp upstream, most preferably approximately 400 bp upstream). Point mutation or deletion may also be used (particularly for down-regulating expression of a gene).

Such methods, however, may be quite unstable or uncertain, and therefore it is preferred that the engineering step is performed via a homologous recombination event. Preferably, the event takes place between a sequence (a recombinogenic region) of at least 30 nucleotides on the bacterial chromosome, and a sequence (a second recombinogenic region) of at least 30 nucleotides on a vector transformed within the strain. Preferably the regions are 40-1000 nucleotides, more preferably 100-800 nucleotides, most preferably 500 nucleotides). These recombinogenic regions should be sufficiently similar that they are capable of hybridising to one another under highly stringent conditions.

Methods used to carry out the genetic modification events herein described (such as the upregulation or downregulation of genes by recombination events and the introduction of further gene sequences into a Neisserial genome) are described in WO01/09350. Typical strong promoters that may be integrated in *Neisseria* are *porA, porB, lgtF,* Opa, *p110, lst,* and *hpuAB.* PorA and PorB are preferred as constitutive, strong promoters. It has been established that the PorB promoter activity is contained in a fragment corresponding to nucleotides -1 to -250 upstream of the initation codon of *porB.*

### Down regulation/Removal of Variable and non-protective immunodominant antigens

Many surface antigens are variable among bacterial strains and as a consequence are protective only against a limited set of closely related strains. An aspect of this invention covers outer membrane vesicles of the invention in which the expression of other proteins is reduced, or, preferably, gene(s) encoding variable surface protein(s) are deleted. Such deletion results in a bacterial strain producing blebs which, when administered in a vaccine, have a stronger potential for cross-reactivity against various strains due to a higher influence exerted by conserved proteins (retained on the outer membranes) on the vaccinee's immune system. Examples of such variable antigens in Neisseria that may be downregulated in the bleb immunogenic compositions of the invention include PorA, PorB, Opa.

Other types of gene that could be down-regulated or switched off are genes which, *in vivo,* can easily be switched on (expressed) or off by the bacterium. As outer membrane proteins encoded by such genes are not always present on the bacteria, the presence of such proteins in the bleb preparations can also be detrimental to the effectiveness of the vaccine for the reasons stated above. A preferred example to down-regulate or delete is *Neisseria* Opc protein. Anti-Opc immunity induced by an Opc containing bleb vaccine would only have limited protective capacity as the infecting organism could easily become Opc⁻.

For example, these variable or non-protective genes may be down-regulated in expression, or terminally switched off. This has the advantage of concentrating the immune system on better antigens that are present in low amounts on the outer surface of blebs. By down-regulation it is also meant that surface exposed, variable immunodominant loops of the above outer membrane proteins may be altered or deleted in order to make the resulting outer membrane protein less immunodominant.

Methods for downregulation of expression are disclosed in WO01/09350. Preferred combinations of proteins to be downregulated in the bleb immunogenic compositions of the invention include PorA and OpA; PorA and OpC; OpA and OpC; PorA and OpA and OpC.

### Detoxification of LPS

The blebs in the immunogenic compositions of the invention may be detoxified via methods for detoxification of LPS which are disclosed in WO01/09350. In particular methods for detoxification of LPS of the invention involve the downregulation of htrB and/or msbB enzymes are disclosed in WO01/09350. Such methods are preferably combined with methods of bleb extraction involving low levels of DOC, preferably 0-0.3% DOC, more preferably 0.05%-0.2% DOC, most preferably around 0.1% DOC.

### Cross-reactive polysaccharides

The isolation of bacterial outer-membrane blebs from encapsulated Gram-negative bacteria often results in the co-purification of capsular polysaccharide. In some cases, this "contaminant" material may prove useful since polysaccharide may enhance the immune response conferred by other bleb components. In other cases however, the presence of contaminating polysaccharide material in bacterial bleb preparations may prove detrimental to the use of the blebs in a vaccine. For instance, it has been shown at least in the case of *N. meningitidis* that the serogroup B capsular polysaccharide does not confer protective immunity and is susceptible to induce an adverse autoimmune response in humans. Consequently, outer membrane vesicles of the invention may be isolated from a bacterial strain for bleb production, which has been engineered such that it is free of capsular polysaccharide. The blebs will then be suitable for use in humans. A particularly preferred example of such a bleb preparation is one from *N*. *meningitidis* serogroup B devoid of capsular polysaccharide.

This may be achieved by using modified bleb production strains in which the genes necessary for capsular biosynthesis and/or export have been impaired. Inactivation of the gene coding for capsular polysaccharide biosynthesis or export can be achieved by mutating (point mutation, deletion or insertion) either the control region, the coding region or both (preferably using the homologous recombination techniques described above), or by any other way of decreasing the enzymatic function of such genes. Moreover, inactivation of capsular biosynthesis genes may also be achieved by antisense over-expression or transposon mutagenesis. A preferred method is the deletion of some or all of the *Neisseria meningitidis cps* genes required for polysaccharide biosynthesis and export. For this purpose, the replacement plasmid pMF121 (described in Frosh et al.1990, Mol. Microbiol. 4:1215-1218) can be used to deliver a mutation deleting the *cpsCAD* (+ *galE*) gene cluster.

Preferably the *siaD* gene is deleted, or down-regulated in expression or the gene product enzymatically inactivated by any other way (the meningococcal *siaD* gene encodes alpha-2,3-sialyltransferase, an enzyme required for capsular polysaccharide and LOS synthesis). This mutation is preferred in order to cause minimum disruption to LPS epitopes which are preferably conserved in the preparations of the invention.

In bleb preparations, particularly in preparations extracted with low DOC concentrations LPS may be used as an antigen in the immunogenic composition of the invention. It is however advantageous to downregulate/delete/inactivate enzymatic function of either the IgtE or preferably IgtB genes/gene products in order to remove human like lacto-N-neotetraose structures. The Neisserial locus (and sequence thereof) comprising the Igt genes for the biosynthesis of LPS oligosaccharide structure is known in the art (Jennings et al Micorbiology 1999 145; 3013-3021). Downregulation/deletion of IgtB (or functional gene product) is preferred since it leaves the LPS protective epitope intact. In *N. meningitidis* serogroup B bleb preparations of the invention, the downregulation/deletion of both siaD and IgtB is preferred, leading to a bleb preparation with optimal safety and LPS protective epitope retention.

Immunogenic composition of the invention may comprise at least, one, two, three, four or five different outer membrane vesicle preparations. Where two or more OMV preparations are included, at least one antigen of the invention is upregulated in each OMV. Such OMV preparations may be derived from Neisserial strains of the same species and serogroup or preferably from Neisserial strains of different class, serogroup, serotype, subserotype or immunotype. For example, an immunogenic composition may comprise one or more outer membrane vesicle preparation(s) which contains LPS of immunotype L2 and one or more outer membrane vesicle preparation which contains LPS of immunotype L3. L2 or L3 OMV preparations are preferably derived from a stable strain which has minimal phase variability in the LPS oligosaccharide synthesis gene locus.

### Combinations

The immunogenic compositions of the present invention, whether in subunit or outer membrane vesicle form, may also comprise at least one or more of the following:
a. one or more subunit vaccines;
b. one or more outer membrane vesicles with one or more antigens upregulated; and
c. a mixture of a. and b.

Any of a - c includes an FrpB protein of the present invention.

The immunogenic compositions of the invention may thus also comprise both a subunit composition and an outer membrane vesicle.

The outer membrane vesicle preparation may have at least one different antigen selected from the following list which has been recombinantly upregulated in an outer membrane vesicle: NspA, Hsf, Hap, OMP85, TbpA (high), TbpA (low), LbpA, TbpB, LbpB, NadA, TspA, TspB, PilQ and PldA; and optionally comprise either or both of LPS immunotype L2 and LPS immunotype L3.

There are several antigens that are particularly suitable for inclusion in a subunit composition due to their solubility. Examples of such proteins include; FhaB, NspA, passenger domain of Hsf, passenger domain of Hap, OMP85, FrpA, FrpC, TbpB, LbpB, PilQ.

Neisserial infections progress through several different stages. For example, the meningococcal life cycle involve nasopharyngeal colonisation, mucosal attachment, crossing into the bloodstream, multiplication in the blood, induction of toxic shock, crossing the blood/brain barrier and multiplication in the cerebrospinal fluid and/or the meninges. Different molecules on the surface of the bacterium will be involved in different steps of the infection cycle. By targeting the immune response against an effective amount of a combination of particular antigens, involved in different processes of Neisserial infection, a Neisserial vaccine with surprisingly high efficacy can be achieved.

In particular, combinations of certain Neisserial antigens from different classes with the FrpB protein of the invention can elicit an immune response which protects against multiple stages of infection. Such combinations of antigens can surprisingly lead to synergistically improved vaccine efficacy against Neisserial infection where more that one function of the bacterium is targeted by the immune response in an optimal fashion. Some of the further antigens which can be included are involved in adhesion to host cells, some are involved in iron acquisition, some are autotransporters and some are toxins.

The efficacy of vaccines can be assessed through a variety of assays. Protection assays in animal models are well known in the art. Furthermore, serum bactericidal assay (SBA) is the most commonly agreed immunological marker to estimate the efficacy of a meningococcal vaccine (Perkins et al. J Infect Dis. 1998, 177:683-691).

Some combinations of antigens can lead to improved protection in animal model assays and/or synergistically higher SBA titres. Without wishing to be bound by theory, such synergistic combinations of antigens are enabled by a number of characteristics of the immune response to the antigen combination. The antigens themselves are usually surface exposed on the Neisserial cells and tend to be conserved but also tend not to be present in sufficient quantity on the surface cell for an optimal bactericidal response to take place using antibodies elicited against the antigen alone. Combining the antigens of the invention can result in a formulation eliciting an advantageous combination of bactericidal antibodies which interact with the Neisserial cell beyond a critical threshold. At this critical level, sufficient antibodies of sufficient quality bind to the surface of the bacterium to allow efficient killing by complement and much higher bactericidal effects are seen as a consequence. As serum bactericidal assays (SBA) closely reflect the efficacy of vaccine candidates, the attainment of good SBA titres by a combination of antigens is a good indication of the protective efficacy of a vaccine containing that combination of antigens.

An additional advantage of the invention is that the combination of the antigens of the invention from different families of proteins in an immunogenic composition will enable protection against a wider range of strains.

The invention thus also relates to immunogenic compositions comprising a plurality of proteins selected from at least two different categories of protein, having different functions within Neisseria. Examples of such categories of proteins are adhesins, autotransporter proteins, toxins and Fe acquisition proteins. The vaccine combinations of the invention show surprising improvement in vaccine efficacy against homologous Neisserial strains (strains from which the antigens are derived) and preferably also against heterologous Neisserial strains.

In particular, the invention provides immunogenic compositions that comprise at least one, two, three, four five, six, seven, eight, nine or ten different additional Neisseria antigens (to FrpB) selected from at least one, two, three, four or five groups of proteins selected from the following:
at least one Neisserial adhesin selected from the group consisting of FhaB, Hsf, NspA, NadA, PilC, Hap, MafA, MafB, Omp26, NMB0315, NMB0995 and NMB1119;
at least one Neisserial autotransporter selected from the group consisting of Hsf, Hap, IgA protease, AspA and NadA;
at least one Neisserial toxin selected from the group consisting of FrpA, FrpC, FrpA/C, VapD, NM-ADPRT, and either or both of LPS immunotype L2 and LPS immunotype L3;
at least one Neisserial Fe acquisition protein selected from the group consisting of TbpA high, TbpA low, TbpB, high, TbpB low, LbpA, LbpB, P2086, HpuA, HpuB, Lipo28, Sibp, FbpA, BfrA, BfrB, Bcp, NMB0964 and NMB0293; and
at least one Neisserial membrane associated protein, preferably outer membrane protein, selected from the group consisting of PldA, TspA, FhaC, NspA, TbpA(high), TbpA(low), LbpA, HpuB, TdfH, PorB, HimD, HisD, GNA1870, OstA, HlpA, MltA, NMB 1124, NMB 1162, NMB 1220, NMB 1313, NMB 1953, HtrA, TspB, PilQ and OMP85.
and preferably:
a. at least one Neisserial adhesin selected from the group consisting of FhaB, Hsf and NadA;
b. at least one Neisserial autotransporter selected from the group consisting of Hsf, Hap and NadA;
c. at least one Neisserial toxin selected from the group consisting of FrpA, FrpC, and either or both of LPS immunotype L2 and LPS immunotype L3;
d. at least one Neisserial Fe acquisition protein selected from the group consisting of TbpA, TbpB, LbpA and LbpB; and
e. at least one Neisserial outer membrane protein selected from the group consisting of TspA, TspB, NspA, PilQ, OMP85, and PldA.

Preferably the first four (and most preferably all five) groups of antigen are represented in the immunogenic composition of the invention.

As previously mentioned where a protein is specifically mentioned herein, it is preferably a reference to a native, full-length protein but it may also encompass antigenic fragments thereof (particularly in the context of subunit vaccines). These are fragments containing or comprising at least 10 amino acids, preferably 20 amino acids, more preferably 30 amino acids, more preferably 40 amino acids or most preferably 50 amino acids, taken contiguously from the amino acid sequence of the protein. In addition, antigenic fragments denotes fragments that are immunologically reactive with antibodies generated against the Neisserial proteins or with antibodies generated by infection of a mammalian host with Neisseria. Antigenic fragments also includes fragments that when administered at an effective dose, elicit a protective immune response against Neisserial infection, more preferably it is protective against *N. meningitidis* and/or *N. gonorrhoeae* infection, most preferably it is protective against *N. meningitidis* serogroup B infection.

Also included in the invention are recombinant fusion proteins of Neisserial proteins useful in the invention, or fragments thereof. These may combine different Neisserial proteins or fragments thereof in the same protein. Alternatively, the invention also includes individual fusion proteins of Neisserial proteins or fragments thereof, as a fusion protein with heterologous sequences such as a provider of T-cell epitopes, or viral surface proteins such as influenza virus haemagglutinin, tetanus toxoid, diphtheria toxoid, CRM 197.

### Addition antigens of the invention

NMB references refer to reference numbers to sequences which can be accessed from www.neisseria.org.

### 1. Adhesins

Adhesins include FhaB (WO98/02547), NadA (J. Exp.Med (2002) 195:1445; NMB 1994), Hsf also known as NhhA (NMB 0992) (WO99/31132), Hap (NMB 1985)(WO99/55873), NspA (WO96/29412), MafA (NMB 0652) and MafB (NMB 0643) (Annu Rev Cell Dev Biol. 16; 423-457 (2000); Nature Biotech 20; 914-921 (2002)), Omp26 (NMB 0181), NMB 0315, NMB 0995, NMB 1119 and PilC (Mol. Microbiol.1997, 23; 879-892). These are proteins that are involved in the binding of Neisseria to the surface of host cells. Hsf is an example of an adhesin, as well as being an autotranporter protein. Immunogenic compositions of the invention may therefore include combinations of Hsf and other autotransporter proteins where Hsf contributes in its capacity as an adhesin. These adhesins may be derived from *Neisseria meningitidis* or *Neisseria gonorrhoeae* or other Neisserial strains. The invention also includes other adhesins from Neisseria.

### FhaB

This antigen has been described in WO98/02547 SEQ ID NO 38 (nucleotides 3083-9025) - see also NMB0497. The present inventors have found FhaB to be particularly effectively at inducing anti-adhesive antibodies alone and in particular with other antigens of the invention. Although full length FhaB could be used, the inventors have found that particular C-terminal truncates are surprisingly at least as effective and preferably even more effective in terms of cross-strain effect. Such truncates have also been advantageously shown to be far easier to clone. FhaB truncates of the invention typically correspond to the N-terminal two-thirds of the FhaB molecule, preferably the new C-terminus being situated at position 1200-1600, more preferably at position 1300-1500, and most preferably at position 1430-1440. Specific embodiments have the C-terminus at 1433 or 1436. Accordingly such FhaB truncates of the invention and vaccines comprising such truncates are preferred components of the combination immunogenic compositions of the invention. The N-terminus may also be truncated by up to 10, 20, 30, 40 or 50 amino acids.

### 2. Autotransporter proteins

Autotransporter proteins typically are made up of a signal sequence, a passenger domain and an anchoring domain for attachment to the outer membrane. Examples of autotransporter proteins include Hsf (WO99/31132) (NMB 0992), HMW, Hia (van Ulsen et al Immunol. Med. Microbiol. 2001 32; 53-64), Hap (NMB 1985) (WO99/55873; van Ulsen et al Immunol. Med. Microbiol. 2001 32; 53-64), UspA, UspA2, NadA (NMB 1994) (Comanducci et al J. Exp. Med. 2002 195; 1445-1454), AspA (Infection and Immunity 2002, 70(8); 4447-4461; NMB 1029), Aida-1 like protein, SSh-2 and Tsh. NadA (J. Exp.Med (2002) 195:1445) is another example of an autotransporter proteins, as well as being an adhesin. Immunogenic compositions of the invention may therefore include combinations of NadA and adhesins where NadA contributes in its capacity as an autotransporter protein.These proteins may be derived from *Neisseria meningitidis* or *Neisseria gonorrhoeae* or other Neiserial strians. The invention also includes other autotransporter proteins from Neisseria.

### Hsf

Hsf has a structure that is common to autotransporter proteins. For example, Hsf from *N. meningitidis* strain H44/76 consists of a signal sequence made up of amino acids 1-51, a head region at the amino terminus of the mature protein (amino acids 52-479) that is surface exposed and contains variable regions (amino acids 52-106, 121-124, 191-210 and 230-234), a neck region (amino acids 480-509), a hydrophobic alpha-helix region (amino acids 518-529) and an anchoring domain in which four transmembrane strands span the outer membrane (amino acids 539-591).

Although full length Hsf may be used in immunogenic compositions of the invention, various Hsf truncates and deletions may also be advantageously used depending on the type of vaccine.

Where Hsf is used in a subunit vaccine, it is preferred that a portion of the soluble passenger domain is used; for instance the complete domain of amino acids 52 to 479, most preferably a conserved portion thereof, for instance the particularly advantageous sequence of amino acids 134 to 479. Preferred forms of Hsf may be truncated so as to delete variable regions of the protein disclosed in WO01/55182. Preferred variants would include the deletion of one, two, three, four, or five variable regions as defined in WO01/55182. The above sequences and those described below, can be extended or truncated by up to 1, 3, 5, 7, 10 or 15 amino acids at either or both N or C termini.

Preferred fragments of Hsf therefore include the entire head region of Hsf, preferably containing amino acids 52-473. Additional preferred fragments of Hsf include surface exposed regions of the head including one or more of the following amino acid sequences; 52-62, 76-93, 116-134, 147-157, 157-175, 199-211, 230-252, 252-270, 284-306, 328-338, 362-391, 408-418, 430-440 and 469-479.

Where Hsf is present in an outer membrane vesicle preparation, it may be expressed as the full-length protein or preferably as an advantageous variant made up of a fusion of amino acids 1-51 and 134-591 (yielding a mature outer membrane protein of amino acid sequence 134 to the C-terminus). Preferred forms of Hsf may be truncated so as to delete variable regions of the protein disclosed in WO01/55182. Preferred variants would include the deletion of one, two, three, four, or five variable regions as defined in WO01/55182. Preferably the first and second variable regions are deleted. Preferred variants would delete residues from between amino acid sequence 52 through to 237 or 54 through to 237, more preferably deleting residues between amino acid 52 through to 133 or 55 through to 133. The mature protein would lack the signal peptide.

### Hap

Computer analysis of the Hap-like protein from *Neisseria meningitidis* reveals at least three structural domains. Considering the Hap-like sequence from strain H44/76 as a reference, Domain 1, comprising amino-acid 1 to 42, encodes a sec-dependant signal peptide characteristic of the auto-transporter family, Domain 2, comprising aminoacids 43 to 950, encode the passenger domain likely to be surface exposed and accessible to the immune system, Domain 3, comprising residues 951 to the C-terminus (1457), is predicted to encode a beta-strands likely to assemble into a barrel-like structure and to be anchored into the outer-membrane. Since domains 2 is likely to be surface-exposed, well conserved (more than 80% in all strain tested) and could be produced as subunit antigens in *E*. *coli,* it represents an interesting vaccine candidates. Since domains 2 and 3 are likely to be surface-exposed, are well conserved (Pizza et al. (2000), Science 287: 1816-1820), they represent interesting vaccine candidates. Domain 2 is known as the passenger domain.

Immunogenic compositions of the invention may comprise the full-length Hap protein, preferably incorporated into an OMV preparation. Immunogenic compositions of the invention may also comprise the passenger domain of Hap which in strain H44/76 is composed of amino acid residues 43-950. This fragment of Hap would be particularly advantageously used in a subunit composition of the invention.The above sequence for the passenger domain of Hap can be extended or truncated by up to 1, 3, 5, 7, 10,15, 20, 25, or 30 amino acids at either or both N or C termini.

### 3. Iron acquisition proteins

Iron aquisition proteins include TbpA (NMB 0461) (WO92/03467, US5912336, WO93/06861 and EP586266), TbpB (NMB 0460) (WO93/06861 and EP586266), LbpA (NMB 1540) (Med Microbiol (1999) 32:1117), LbpB (NMB 1541)(WO/99/09176), HpuA (U73112.2) (Mol Microbiol. 1997, 23; 737-749), HpuB (NC_003116.1) (Mol Microbiol. 1997, 23; 737-749), P2086 also known as XthA (NMB 0399) (13^{th} International Pathogenic Neisseria Conference 2002), FbpA (NMB 0634), FbpB, BfrA (NMB 1207), BfrB (NMB 1206), Lipo28 also known as GNA2132 (NMB 2132), Sibp (NMB 1882), HmbR, HemH, Bcp (NMB 0750), Iron (III) ABC transporter-permease protein (Tettelin et al Science 287; 1809-1815 2000), Iron (III) ABC transporter - periplasmic (Tettelin et al Science 287; 1809-1815 2000), TonB-dependent receptor (NMB 0964 and NMB 0293)(Tettelin et al Science 287; 1809-1815 2000) and transferrin binding protein related protein (Tettelin et al Science 287; 1809-1815 2000). These proteins may be derived from *Neisseria meningitidis, Neisseria gonorrhoeae* or other Neisserial strains. The invention also includes other iron aquisition proteins from Neisseria.

### TbpA

TbpA interacts with TbpB to form a protein complex on the outer membrane of Neisseria, which binds transferrin. Structurally, TbpA contains an intracellular N-terminal domain with a TonB box and plug domain, multiple transmembrane beta strands linked by short intracellular and longer extracellular loops.

Two families of TbpB have been distinguished, having a high molecular weight and a low molecular weight respectively. High and low molecular weight forms of TbpB associate with different families of TbpA which are distinguishable on the basis of homology. Despite being of similar molecular weight, they are known as the high molecular weight and low molecular weight families because of their association with the high or low molecular weight form of TbpB (Rokbi et al FEMS Microbiol. Lett. 100; 51, 1993). The terms TbpA(high) and TbpA(low) are used to refer to these two forms of TbpA, and similarly for TbpB. Immunogenic compositions of the invention may comprise TbpA and TbpB from serogroups A, B, C, Y and W-135 of N. *meningitidis* as well as iron acquisition proteins from other bacteria including N *gonorrhoeae.* Transferrin binding proteins TbpA and TbpB have also been referred to as Tbp1 and Tbp2 respectively (Cornelissen et al Infection and Immunity 65; 822, 1997).

TbpA contains several distinct regions. For example, in the case of TbpA from *N. meningitidis* strain H44/76, the amino terminal 186 amino acids form an internal globular domain, 22 beta strands span the membrane, forming a beta barrel structure. These are linked by short intracellular loops and larger extracellular loops. Extracellular loops 2, 3 and 5 have the highest degree of sequence variability and loop 5 is surface exposed. Loops 5 and 4 are involved in ligand binding.

Preferred fragments of TbpA include the extracellular loops of TbpA. Using the sequence of TbpA from *N. meningitidis* strain H44/76, these loops correspond to amino acids 200-202 for loop1, amino acids 226-303 for loop 2, amino acids 348-395 for loop 3, amino acids 438-471 for loop 4, amino acids 512-576 for loop 5, amino acids 609-625 for loop 6, amino acids 661-671 for loop 7, amino acids 707-723 for loop 8, amino acids 769-790 for loop 9, amino acids, 814-844 for loop 10 and amino acids 872-903 for loop 11. The corresponding sequences, after sequence alignment, in other Tbp proteins would also constitute preferred fragments. Most preferred fragments would include amino acid sequences constituting loop 2, loop 3, loop 4 or loop 5 of Tbp.

Where the immunogenic compositions of the invention comprise TbpA, it is preferable to include both TbpA(high) and TbpA (low).

Although TbpA is preferably presented in an OMV vaccine, it may also be part of a subunit vaccine. For instance, isolated iron acquisition proteins which could be introduced into an immmunogenic composition of the invention are well known in the art (WO00/25811). They may be expressed in a bacterial host, extracted using detergent (for instance 2% Elugent) and purified by affinity chromatography or using standard column chromatography techniques well known to the art (Oakhill et al Biochem J. 2002 364; 613-6).

Where TbpA is presented in an OMV vaccine, its expression can be upregulated by genetic techiques discussed herein or in WO 01/09350, or may preferably be upregulated by growth of the parent strain under iron limitation conditions. This process will also result in the upregulation of variable iron-regulated proteins, particularly wild-type FrpB which may become immunodominant and it is therefore advantageous to downregulate the expression of (and preferably delete the genes encoding) such proteins (particularly wild-type FrpB) as described in WO 01/09350, or remove its immunodominant loops as described above, to ensure that the immunogenic composition of the invention elicits an immune response against antigens present in a wide range of Neisserial strains. If wild-type FrpB is deleted, an additional copy of a non immunodominant mutant FrpB gene may be introduced into the cell. It is preferred to have both TbpA(high) and TbpA(low) present in the immunogenic composition and this is preferably achieved by combining OMVs derived from two strains, expressing the alternative forms of TbpA.

### 4. Toxins

Toxins include FrpA (NMB 0585; NMB 1405), FrpA/C (see below for definition), FrpC ( NMB 1415; NMB 1405) (WO92/01460), NM-ADPRT (NMB 1343) (13^{th} International Pathogenic Neisseria Conference 2002 Masignani et al p135), VapD (NMB 1753), lipopolysaccharide (LPS; also called lipooligosaccharide or LOS) immunotype L2 and LPS immunotype L3. FrpA and FrpC contain a region which is conserved between these two proteins and a preferred fragment of the proteins would be a polypeptide containing this conserved fragment, preferably comprising amino acids 227-1004 of the sequence of FrpA/C. These antigens may be derived from *Neisseria meningitidis* or *Neisseria gonorrhoeae* or other Neisserial strains. The invention also includes other toxins from Neisseria.

In an alternative embodiment, toxins may include antigens involved in the regulation of toxicity, for example OstA which functions in the synthesis of lipopolysaccharides.

### FrpA and FrpC

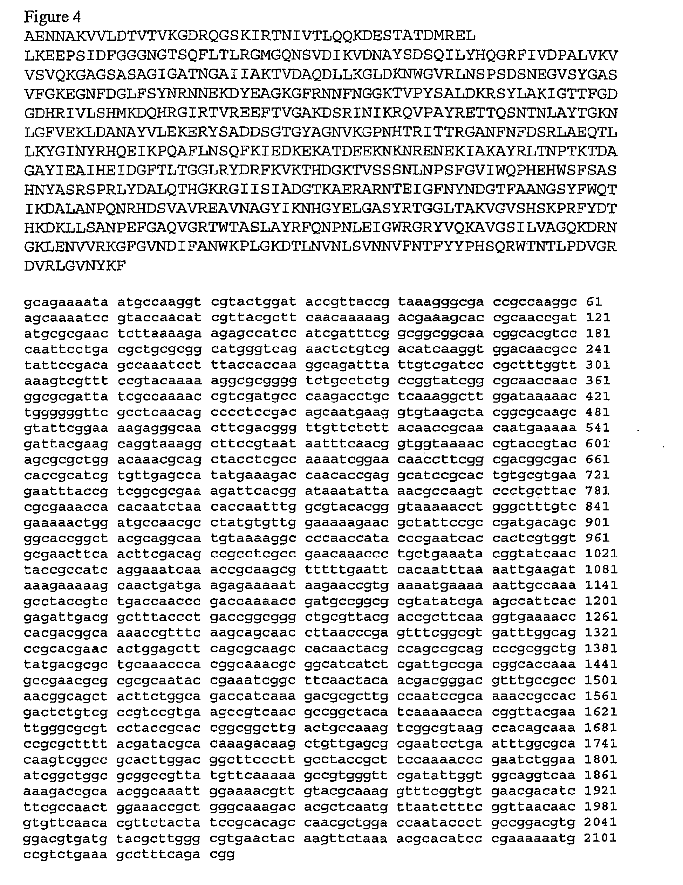

*Neisseria meningitidis* encodes two RTX proteins, referred to as FrpA & FrpC secreted upon iron limitation (Thompson et al., (1993) J. Bacteriol. 175:811-818; Thompson et al., (1993) Infect. Immun.. 61:2906-2911). The RTX (Repeat ToXin) protein family have in common a series of 9 amino acid repeat near their C-termini with the consensus: Leu Xaa Gly Gly Xaa Gly (Asn/Asp) Asp Xaa. (LXGGXGN_{/D}DX). The repeats in *E. coli* HlyA are thought to be the site of Ca2+ binding. As represented in Figure 4, meningococcal FrpA and FrpC proteins, as characterized in strain FAM20, share extensive amino-acid similarity in their central and C-terminal regions but very limited similarity (if any) at the N-terminus. Moreover, the region conserved between FrpA and FrpC exhibit some polymorphism due to repetition (13 times in FrpA and 43 times in FrpC) of a 9 amino acid motif.

Immunogenic compositions of the invention may comprise the full length FrpA and/or FrpC or preferably, a fragment comprising the sequence conserved between

FrpA and FrpC. The conserved sequence is made up of repeat units of 9 amino acids. Immunogenic compositions of the invention would preferably comprise more that three repeats, more than 10 repeats, more than 13 repeats, more than 20 repeats or more than 23 repeats.

Such truncates have advantageous properties over the full length molecules, and vaccines comprising such antigens are preferred for being incorporated in the immunogenic compositions of the invention.

Sequences conserved between FrpA and FrpC are designated FrpA/C and whereever FrpA or FrpC forms a constituent of immunogenic compositions of the invention, FrpA/C could be advantageously used. Amino acids 277-1004 of the FrpA sequence is the preferred conserved region. The above sequence can be extended or truncated by up to 1, 3, 5, 7, 10; 15, 20, 25, or 30 amino acids at either or both N or C termini.

### LPS

LPS (lipopolysaccharide, also known as LOS - lipooligosaccharide) is the endotoxin on the outer membrane of Neisseria. The polysaccharide moiety of the LPS is known to induce bactericidal antibodies.

Heterogeneity within the oligosaccharide moiety of the LPS generates structural and antigenic diversity among different neisserial strains (Griffiss et al. Inf. Immun. 1987; 55: 1792-1800). This has been used to subdivide meningococcal strains into 12 immunotypes (Scholtan et al. J Med Microbiol 1994, 41:236-243). Immunotypes L3, L7, & L9 are immunologically identical and are structurally similar (or even the same) and have therefore been designated L3,7,9 (or, for the purposes of this specification, generically as "L3"). Meningococcal LPS L3,7,9 (L3), L2 and L5 can be modified by sialylation, or by the addition of cytidine 5'-monophosphate-N-acetylneuraminic acid. Although L2, L4 and L6 LPS are distinguishable immunologically, they are structurally similar and where L2 is mentioned herein, either L4 or L6 may be optionally substituted within the scope of the invention. See M. P. Jennings et al, Microbiology 1999, 145, 3013-3021 and Mol Microbiol 2002, 43:931-43 for further illustration of LPS structure and heterogeneity.

Where LPS, preferably meningococcal LPS, is included in a vaccine of the invention, preferably and advantageously either or both of immunotypes L2 and L3 are present. LPS is preferably presented in an outer membrane vesicle (preferably where the vesicle is extracted with a low percentage detergent, more preferably 0-0.5%, 0.02-0.4%, 0.04-0.3%, 0.06-0.2%, 0.08-0.15% or 0.1% , most preferably deoxycholate [DOC]) but may also be part of a subunit vaccine. LPS may be isolated using well known precedure including the hot water-phenol procedure (Wesphal and Jann Meth. Carbo. Chem. 5; 83-91 1965). See also Galanos et al. 1969, Eur J Biochem 9:245-249, and Wu et al. 1987, Anal Bio Chem 160:281-289. LPS may be used plain or conjugated to a source of T-cell epitopes such as tetanus toxoid, Diphtheria toxoid, CRM-197 or OMV outer membrane proteins. Techniques for conjugating isolated LOS are also known (see for instance EP 941738 incorporated by reference herein).

Where LOS (in particular the LOS of the invention) is present in a bleb formulation the LOS is preferably conjugated in situ by methods allowing the conjugation of LOS to one or more outer membrane proteins also present on the bleb preparation (e.g. PorA or PorB in meningococcus).

This process can advantageously enhance the stability and/or immunogenicity (providing T-cell help) and/or antigenicity of the LOS antigen within the bleb formulation - thus giving T-cell help for the T-independent oligosaccharide immunogen in its most protective conformation - as LOS in its natural environment on the surface of meningococcal outer membrane. In addition, conjugation of the LOS within the bleb can result in a detoxification of the LOS (the Lipid A portion being stably buried in the outer membrane thus being less available to cause toxcity). Thus the detoxification methods mentioned herein of isolating blebs from htrB⁻ or msbB⁻ mutants, or by adding non toxic peptide functional equivalent of polymyxin B [a molecule with high affinity to Lipid A] to the composition (see WO 93/14115, WO 95/03327, Velucchi et al (1997) J Endotoxin Res 4: 1-12, and EP 976402 for further details of non-toxic peptide functional equivalents of polymyxin B - particularly the use of the peptide SAEP 2 (of sequence KTKCKFLKKC where the 2 cysteines form a disulphide bridge)) may not be required (but which may be added in combination for additional security). Thus the inventors have found that a composition comprising blebs wherein LOS present in the blebs has been conjugated in an intra-bleb fashion to outer membrane proteins also present in the bleb can form the basis of a vaccine for the treatment or prevention of diseases caused by the organism from which the blebs have been derived, wherein such vaccine is substantially non-toxic and is capable of inducing a T-dependent bactericidal response against LOS in its native environment.

Such bleb preparations may be isolated from the bacterial in question (see WO 01/09350), and then subjected to known conjugation chemistries to link groups (e.g. NH₂ or COOH) on the oligosaccharide portion of LOS to groups (e.g. NH₂ or COOH) on bleb outer membrane proteins. Cross-linking techniques using glutaraldehyde, formaldehyde, or glutaraldehyde/formaldehyde mixes may be used, but it is preferred that more selective chemistries are used such as EDAC or EDAC/NHS (J.V. Staros, R.W. Wright and D. M. Swingle. Enhancement by N-hydroxysuccinimide of water-soluble carbodiimide-mediated coupling reactions. Analytical chemistry 156: 220-222 (1986); and Bioconjugates Techniques. Greg T. Hermanson (1996) pp173-176). Other conjugation chemistries or treatments capable of creating covalent links between LOS and protein molecules that could be used are described in EP 941738.

Preferably the bleb preparations are conjugated in the absence of capsular polysaccharide. The blebs may be isolated from a strain which does not produce capsular polysaccharide (naturally or via mutation as described below), or may be purified from most and preferably all contaminating capsular polysaccharide. In this way, the intra-bleb LOS conjugation reaction is much more efficient.

Preferably more than 10, 20, 30, 40, 50, 60, 70, 80, 90, or 95% of the LOS present in the blebs is cross-linked/conjugated.

Intrableb conjugation should preferably incorporate 1, 2 or all 3 of the following process steps: conjugation pH should be greater than pH 7.0, preferably greater than or equal to pH 7.5 (most preferably under pH 9); conditions of 1-5% preferably 2-4% most preferably around 3% sucrose should be maintained during the reaction; NaCl should be minimised in the conjugation reaction, preferably under 0.1M, 0.05M, 0.01M, 0.005M, 0.001M, and most preferably not present at all. All these process features make sure that the blebs remain stable and in solution throughout the conjugation process.

The EDAC/NHS conjugation process is a preferred process for intra-bleb conjugation. EDAC/NHS is preferred to formalydehyde which can cross-link to too high an extent thus adversely affecting filterability. EDAC reacts with carboxylic acids (such as KDO in LOS) to create an active-ester intermediate. In the presence of an amine nucleophile (such as lysines in outer membrane proteins such as PorB), an amide bond is formed with release of an isourea by-product. However, the efficiency of an EDAC-mediated reaction may be increased through the formation of a Sulfo-NHS ester intermediate. The Sulfo-NHS ester survives in aqueous solution longer than the active ester formed from the reaction of EDAC alone with a carboxylate. Thus, higher yields of amide bond formation may be realized using this two-stage process. EDAC/NHS conjugation is discussed in J.V. Staros, R.W. Wright and D. M. Swingle. Enhancement by N-hydroxysuccinimide of water-soluble carbodiimide-mediated coupling reactions. Analytical chemistry 156: 220-222 (1986); and Bioconjugates Techniques. Greg T. Hermanson (1996) pp173-176. Preferably 0.01-5 mg EDAC / mg bleb is used in the reaction, more preferably 0.05-1 mg EDAC/mg bleb. The amount of EDAC used depends on the amont of LOS present in the sample which in turn depends on the deoxycholate (DOC) % used to extract the blebs. At low % DOC (e.g. 0.1%), high amounts of EDAC are used (1mg/mg and beyond), however at higher % DOC (e.g. 0.5%), lower amounts of EDAC are used (0.025-0.1mg/mg) to avoid too much inter-bleb crosslinking.

A preferred process of the invention is therefore a process for producing intra-bleb conjugated LOS (preferably meningococcal) comprising the steps of conjugating blebs in the presence of EDAC/NHS at a pH between pH 7.0 and pH 9.0 (preferably around pH 7.5), in 1-5% (preferably around 3%) sucrose, and optionally in conditions substantially devoid of NaCl (as described above), and isolating the conjugated blebs from the reaction mix.

The reaction may be followed on Western separation gels of the reaction mixture using anti-LOS (e.g. anti-L2 or anti-L3) mAbs to show the increase of LOS molecular weight for a greater proportion of the LOS in the blebs as reaction time goes on.

Yields of 99% blebs can be recovered using such techniques.

EDAC was found to be an excellent intra-bleb cross-linking agent in that it cross-linked LOS to OMP sufficiently for improved LOS T-dependent immunogenicity, but did not cross link it to such a high degree that problems such as poor filterability, aggregation and inter-bleb cross-linking occurred. The morphology of the blebs generated is similar to that of unconjugated blebs (by electron microscope). In addition, the above protocol avoided an overly high cross-linking to take place (which can decrease the immunogenicity of protective OMPs naturally present on the surface of the bleb e.g. TbpA or Hsf.

It is preferred that the meningococcal strain from which the blebs are derived is a mutant strain that cannot produce capsular polysaccharide (in particular siaD⁻). It is also preferred that immunogenic compositions effective against meningococcal disease comprise both an L2 and and L3 bleb, wherein the L2 and L3 LOS are both conjugated to bleb outer membrane proteins. Furthermore, it is preferred that the LOS structure within the intra-bleb conjugated bleb is consistent with it having been derived from an IgtE⁻ or, preferably, lgtb⁻ meningococcal strain. Most preferably immunogenic compositions comprise intrableb-conjugated blebs: derived from a mutant meningococcal strain that cannot produce capsular polysaccharide and is IgtB⁻; comprising L2 and L3 blebs derived from mutant meningococcal strains that cannot produce capsular polysaccharide; comprising L2 and L3 blebs derived from mutant meningococcal strains that are IgtB⁻; or most preferably comprising L2 and L3 blebs derived from mutant meningococcal strains that cannot produce capsular polysaccharide and are IgtB⁻.

Typical L3 meningococcal strain that can be used for the present invention is H44/76 menB strain. A typical L2 strain is the B16B6 menB strain or the 39E meningococcus type C strain.

As stated above, the blebs of the invention have been detoxified to a degree by the act of conjugation, and need not be detoxified any further, however further detoxification methods may be used for additional security, for instance using blebs derived from a meningococcal strain that is htrB⁻ or msbB⁻ or adding a non-toxic peptide functional equivalent of polymyxin B [a molecule with high affinity to Lipid A] (preferably SEAP 2) to the bleb composition (as described above).

In the above way meningococcal blebs and immunogenic compositions comprising blebs are provided which have as an important antigen LOS which is substantially non-toxic, devoid of autoimmunity problems, has a T-dependent character, is present in its natural environment, and is capable of inducing a bactericidal antibody response against more than 90% of meningococcal strains (in the case of L2+L3 compositions).

### 5. Integral outer membrane proteins

Other categories of Neisserial proteins may also be candidates for inclusion in the Neisserial vaccines of the invention and may be able to combine with other antigens in a surprisingly effective manner. Membrane associated proteins, particularly integral membrane proteins and most advantageously outer membrane proteins, especially integral outer membrane proteins may be used in the compositions of the present invention. An example of such a protein is PldA also known as, Omp1A (NMB 0464) (WO00/15801) which is a Neisserial phospholipase outer membrane protein. Further examples are TspA (NMB 0341) (Infect. Immun. 1999, 67; 3533-3541) and TspB (T-cell stimulating protein) (WO 00/03003; NMB 1548, NMB 1628 or NMB 1747). Further examples include PilQ (NMB 1812) (WO99/61620), OMP85 - also known as D15- (NMB 0182) (WO00/23593), NspA (U52066) (WO96/29412), FhaC (NMB 0496 or NMB 1780), PorB (NMB 2039) (Mol. Biol. Evol. 12; 363-370, 1995), HpuB (NC_003116.1), TdfH, (NMB 1497) (Microbiology 2001, 147; 1277-1290), OstA (NMB 0280), MltA also known as GNA33 and Lipo30 (NMB0033), HtrA (NMB 0532; WO 99/55872), HimD (NMB 1302), HisD (NMB 1581), GNA 1870 (NMB 1870), HlpA (NMB 1946), NMB 1124, NMB 1162, NMB 1220, NMB 1313, NMB 1953, HtrA, TbpA (NMB 0461) (WO92/03467) (see also above under iron acquisition proteins) and LbpA (NMB 1541).

### OMP85

OMP85/D15 is an outer membrane protein having a signal sequence, a N-terminal surface-exposed domain and an integral membrane domain for attachment to the outer membrane. Immunogenic compositions of the invention may also comprise the full length OMP85, preferably as part of an OMV preparation. Fragments of OMP85 may also be used in immunogenic compositions of the invention, in particularly, the N terminal surface-exposed domain of OMP85 made up of amino acid residues 1-475 or 50-475 is preferably incorporated into a subunit component of the immunogenic compositions of the invention. The above sequence for the N terminal surface-exposed domain of OMP85 can be extended or truncated by up to 1, 3, 5, 7, 10, 15, 20, 25, or 30 amino acids at either or both N or C termini. It is preferred that the signal sequence is omitted from the OMP85 fragment.

### OstA

OstA functions in the synthesis of lipopolysaccharides and may be considered to be a regulator of toxicity. OstA may alternatively be included in the toxin category where the toxin category is broadened to contain regulators of toxicity as well as toxins.

Preferably the subunit composition comprises FrpB of the present invention together with:
i) at least one further antigen selected from the following list: FhaB, passenger domain of Hsf, passenger domain of Hap, NadA, N-terminal surface exposed domain of OMP85, FrpA, FrpC, FrpA/C, TpbA, TbpB, LpbA, LbpB, PldA, PilQ, NspA and either or both of LPS immunotype L2 and LPS immunotype L3; and/or
ii) at least a Neisserial (preferably meningococcal) outer membrane vesicle (OMV) preparation. Preferably the OMV preparation has at least one antigen (more preferably 2, 3, 4 or 5) selected from the following list which has been recombinantly upregulated in the outer membrane vesicle: NspA, Hsf, Hap, OMP85, TbpA (high), TbpA (low), LbpA, TbpB, LbpB, PilQ and PldA; and optionally comprising either or both of LPS immunotype L2 and LPS immunotype L3.

When i) is present the additional antigen is preferably selected from one or more of the groups of proteins given above.

In another embodiment the outer membrane vesicle of the present invention has at least one further antigen (more preferably 2, 3, 4 or 5) is recombinantly upregulated in the outer membrane vesicle and selected from the following list: NspA, Hsf, Hap, OMP85, TbpA (high), TbpA (low), LbpA, TbpB, LbpB, PilQ and PldA; and optionally comprising either or both of LPS immunotype L2 and LPS immunotype L3. This outer membrane vesicle may be used with one or more further outer membrane vesicles in which has at least one further antigen (more preferably 2, 3, 4 or 5) is recombinantly upregulated in the outer membrane vesicle and selected from the following list: FrpB, NspA, Hsf, Hap, OMP85, TbpA (high), TbpA (low), LbpA, TbpB, LbpB, PilQ and PldA; and optionally comprising either or both of LPS immunotype L2 and LPS immunotype L3.

The immunogenic compositions of the invention may comprise antigens (proteins, LPS and polysaccharides) derived from *Neisseria meningitidis* serogroups A, B, C, Y, W-135 or *Neisseria gonorrhoeae.*

### Further combinations

The immunogenic composition of the invention may further comprise bacterial capsular polysaccharides or oligosaccharides. The capsular polysaccharides or oligosaccharides may be derived from one or more of: *Neisseria meningitidis* serogroup A' C, Y, and/or W-135, *Haemophilus influenzae* b, *Streptococcus pneumoniae,* Group A Streptococci, Group B Streptococci, *Staphylococcus aureus* and *Staphylococcus epidermidis.*

A further aspect of the invention are vaccine combinations comprising the antigenic composition of the invention with other antigens which are advantageously used against certain disease states including those associated with viral or Gram positive bacteria.

In one preferred combination, the antigenic compositions of the invention are formulated with 1, 2, 3 or preferably all 4 of the following meningococcal capsular polysaccharides or oligosaccharides which may be plain or conjugated to a protein carrier: A, C, Y or W-135. Preferably the immunogenic compositions of the invention are formulated with A and C; or C; or C and Y. Such a vaccine containing proteins from *N. meningitidis* serogroup B may be advantageously used as a global meningococcus vaccine.

In a further preferred embodiment, the antigenic compositions of the invention, preferably formulated with 1, 2, 3 or all 4 of the plain or conjugated meningococcal capsular polysaccharides or oligosaccharides A, C, Y or W-135 (as described above), are formulated with a conjugated *H. influenzae* b capsular polysaccharide (or oligosaccharides), and/or one or more plain or conjugated pneumococcal capsular polysaccharides (or oligosaccharides) (for instance those described below). Optionally, the vaccine may also comprise one or more protein antigens that can protect a host against *Streptococcus pneumoniae* infection. Such a vaccine may be advantageously used as a global meningitis vaccine.

In a still further preferred embodiment, the immunogenic composition of the invention is formulated with capsular polysaccharides or oligosaccharides derived from one or more *ofNeisseria meningitidis, Haemophilus influenza b, Streptococcus pneumoniae,* Group A Streptococci, Group B Streptococci, *Staphylococcus aureus* or *Staphylococcus epidermidis.* The pneumococcal capsular polysaccharide or oligosaccharide antigens are preferably selected from serotypes 1, 2, 3, 4, 5, 6B, 7F, 8, 9N, 9V, 10A, 11A, 12F, 14, 15B, 17F, 18C, 19A, 19F, 20, 22F, 23F and 33F (most preferably from serotypes 1, 3, 4, 5, 6B, 7F, 9V, 14, 18C, 19F and 23F). A further preferred embodiment would contain the PRP capsular polysaccharides or oligosaccharides of *Haemophilus influenzae.* A further preferred embodiment would contain the Type 5, Type 8 or 336 capsular polysaccharides of *Staphylococcus aureus.* A further preferred embodiment would contain the Type **I,** Type II or Type III capsular polysaccharides of *Staphylococcus epidermidis.* A further preferred embodiment would contain the Type Ia, Type Ic, Type II or Type III capsular polysaccharides of Group B streptocoocus. A further preferred embodiment would contain the capsular polysaccharides of Group A streptococcus, preferably further comprising at least one M protein and more preferably multiple types of M protein.

Such capsular polysaccharides or oligosaccharides useful in the invention may be unconjugated or conjugated to a carrier protein such as tetatus toxoid, tetanus toxoid fragment C, diphtheria toxoid, CRM197, pneumolysin, Protein D (US6342224). The polysaccharide or oligosaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heteroligation chemistry involving the formation of the thioether linkage. Such conjugates are described in PCT published application WO93/15760 Uniformed Services University.

The conjugates can also be prepared by direct reductive amination methods as described in US 4365170 (Jennings) and US 4673574 (Anderson). Other methods are described in EP-0-161-188, EP-208375 and EP-0-477508. A further method involves the coupling ofa cyanogen bromide activated polysaccharide derivatised with adipic acid hydrazide (ADH) to the protein carrier by Carbodiimide condensation (Chu C. et al Infect. Immunity, 1983 245 256).

Preferred pneumococcal proteins antigens are those pneumococcal proteins which are exposed on the outer surface of the pneumococcus (capable of being recognised by a host's immune system during at least part of the life cycle of the pneumococcus), or are proteins which are secreted or released by the pneumococcus. Most preferably, the protein is a toxin, adhesin, 2-component signal tranducer, or lipoprotein of *Streptococcus pneumoniae,* or fragments thereof. Particularly preferred proteins include, but are not limited to: pneumolysin (preferably detoxified by chemical treatment or mutation) [Mitchell et al. Nucleic Acids Res. 1990 Jul 11; 18(13): 4010 "Comparison of pneumolysin genes and proteins from Streptococcus pneumoniae types I and 2.", Mitchell et al. Biochim Biophys Acta 1989 Jan 23; 1007(1): 67-72 "Expression of the pneumolysin gene in *Escherichia coli:* rapid purification and biological properties.", WO 96/05859 (A. Cyanamid), WO 90/06951 (Paton et al), WO 99/03884 (NAVA)]; PspA and transmembrane deletion variants thereof (US 5804193 - Briles et al.)*;* PspC and transmembrane deletion variants thereof (WO 97/09994 - Briles et al); PsaA and transmembrane deletion variants thereof (Berry & Paton, Infect Immun 1996 Dec;64(12):5255-62 "Sequence heterogeneity of PsaA, a 37-kilodalton putative adhesin essential for virulence of Streptococcus pneumoniae*"*)*;* pneumococcal choline binding proteins and transmembrane deletion variants thereof; CbpA and transmembrane deletion variants thereof (WO 97/41151; WO 99/51266); Glyceraldehyde-3-phosphate - dehydrogenase (Infect. Immun. 1996 64:3544); HSP70 (WO 96/40928); PcpA (Sanchez-Beato et al. FEMS Microbiol Lett 1998, 164:207-14); M like protein, (EP 0837130) and adhesin 18627, (EP 0834568). Further preferred pneumococcal protein antigens are those disclosed in WO 98/18931, particularly those selected in WO 98/18930 and PCT/US99/30390.

The immunogenic composition/vaccine of the invention may also optionally comprise outer membrane vesicle preparations made from other Gram negative bacteria, for example *Moraxella catarrhalis* or *Haemophilus influenzae.*

### Compositions, kits and administration

As previously mentioned the invention provides compositions comprising a FrpB protein for administration to a cell or to a multicellular organism.

An immunogenic composition is a composition comprising at least one antigen which is capable of generating an immune response when administered to a host. Preferably, such immunogenic preparations are capable of generating a protective immune response against Neisserial, preferably *Neisseria meningitidis* and/or *Neisseria gonorrhoeae* infection.

The disclosure also relates to compositions comprising a protein discussed herein or their agonists or antagonists. The proteins of the invention may be employed in combination with a non-sterile or sterile carrier or carriers for use with cells, tissues or organisms, such as a pharmaceutical carrier suitable for administration to an individual. Such compositions comprise, for instance, a media additive or a therapeutically effective amount of a protein of the invention and a pharmaceutically acceptable carrier or excipient. Such carriers may include, but are not limited to, saline, buffered saline, dextrose, water, glycerol, ethanol and combinations thereof. The formulation should suit the mode of administration. The invention further relates to diagnostic and pharmaceutical packs and kits comprising one or more containers filled with one or more of the ingredients of the aforementioned compositions of the invention.

Proteins and other compounds of the invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

In therapy or as a prophylactic, the active agent may be administered to an individual as an injectable composition, for example as a sterile aqueous dispersion, preferably isotonic.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a protein or other compounds of the present invention can be formulated in an enteric or an encapsulated formulation, oral administration may also be possible. Administration of these compounds may also be topical and/or localized, in the form of salves, pastes, gels, solutions, powders and the like.

For administration to mammals, and particularly humans, it is expected that the daily dosage level of the active agent will be from 0.01 mg/kg to 10 mg/kg, typically around 1 mg/kg. The physician in any event will determine the actual dosage which will be most suitable for an individual and will vary with the age, weight and response of the particular individual. The above dosages are exemplary of the average case. There can, of course, be individual instances where higher or lower dosage ranges are merited, and such are within the scope of this invention.

The dosage range required depends on the choice of peptide, the route of administration, the nature of the formulation, the nature of the subject's condition, and the judgement of the attending practitioner. Suitable dosages, however, are in the range of 0.1-100 µg/kg of subject.

A vaccine composition is conveniently in injectable form. Conventional adjuvants may be employed to enhance the immune response. A suitable unit dose for vaccination is 0.5-5 microgram/kg of antigen, and such dose is preferably administered 1-3 times and with an interval of 1-3 weeks. With the indicated dose range, no adverse toxicological effects will be observed with the compounds of the invention which would preclude their administration to suitable individuals.

Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Preferred features and embodiment of the present invention will now be described further with reference to the following Examples:

### Examples

### Example 1- Refolding of FrpB

FrpB is the most abundant iron-limitation-inducible outer-membrane protein of *Neisseria meningitidis.* The high expression levels together with considerable sequence conservation between strains and its ability to induce bactericidal antibodies make it an interesting vaccine candidate for the prevention of meningococcal disease. However, the protein does contain some variability, which is mostly confined to two large extracellular loops, L5 and L7, respectively (van der Ley et al. 1996).

In an attempt to direct the immune response to the more conserved parts of the protein, a deletion of 28 amino acid residues was created in loop L7. A second mutant was created which besides the 28 amino acids deletion in loop L7, contains an additional deletion of 24 amino acids in loop L5. By removing L5 in addition to L7, we aim to construct an FrpB protein that consists of only very-well conserved domains of FrpB, which could potentially induce broad-spectrum bactericidal antibodies. FrpB and FrpB loop-deletion mutants were produced in inclusion bodies in *Escherichia coli* and subsequently refolded *in vitro.* Refolding was successful and immune responses against these proteins may be analysed.

### I: Construction of genes encoding FrpB and genes encoding FrpB loop deletion mutants

The gene encoding *Neisseria meningitidis* H44/76 FrpB was cloned without signal sequence in pET11a resulting in plasmid pET11a-FrpB. An FrpB loop deletion mutant was prepared by using the four-primer PCR method to create a deletion of 28 amino acids in the extracellular loop L7. By this way, the encoded protein will lack amino acids *tteekngqkvdkpmeqqmkdradedtvh* from loop L7. This gene was transferred to pET11a without signal sequence, resulting in plasmid pET11a-FrpBΔ28. This construct was used to create plasmid pET11a-FrpBΔ7Δ5. This plasmid encodes a protein, which besides the 28 amino acids deletion in loop L7, contains an additional deletion of amino acids *qhrgirtvreeftvgdkssrinid* from loop L5. The above uses the old model of loop nomenclature of Figure 6.

### II: Overexpression and in vitro refolding of FrpB, FrpBΔ28 and FrpBΔ7Δ5

FrpB, FrpBΔ28 and FrpBΔ7Δ5 were produced in *Escherichia coli* BL21, after which inclusion bodies were isolated. The inclusion bodies were subsequently solubilized in 8 M urea. Refolding was performed with 1:10 dilution of the solubilized material in 20 mM ethanolamine pH 11, 0.5% Zwittergent 3-12 (SB-12), 0.4 M guanidium hydrochloride. Refolding was performed at room temperature for 16 hrs. Urea and guanidium hydrochloride were removed and buffer was changed from ethanolamine pH 11 to Tris-HCl pH 7.2. Refolded forms were detected by semi-native poly acrylamide gel electrophoresis. Electrophoresis was performed with gels lacking SDS.

The running buffer contained 0.02% SDS, and 0.075% (cold samples) or 2% SDS (boiled samples) was used in the sample buffer. Samples were kept cold or boiled for 5 minutes, after which electrophoresis was performed at constant current of 10 mA on ice.

### III: Results

The urea-denatured proteins were diluted 1:10 to ethanolamine pH 11, 0.5% Zwittergent 12, 0.4 M guanidium hydrochloride. After 16 hrs. samples were desalted and buffer was exchanged for Tris-HCl pH 7.2. The running buffer contained 0.02% SDS, and 0.075% (cold samples) or 2% SDS (boiled samples) was used in the sample buffer. Samples were kept cold or boiled for 5 minutes, after which electrophoresis was performed at constant current of 10 mA on ice.

The results obtained from semi-native gel electrophoresis show folded monomers of FrpB, FrpBΔ28 and FrpBΔ7Δ5. These folded forms can be observed as proteins with higher electrophoretic mobility as compared to the denatured protein. It is also clear that *in vitro* refolded FrpB as well as FrpBΔ28 can assemble into oligomeric complexes, which is a key characteristic of native FrpB. The ability of FrpBΔ7Δ5 to form oligomeric complexes is clearly impaired, however the presence of folded monomers indicates correct folding.

These correctly folded proteins may be be used to study the ability of these proteins to induce synthesis of protective antibodies against *Neisseria meningitidis.* Correct folding of these proteins is considered crucial for obtaining a protective immune response, as native conformational epitopes are more likely to be present.

### Example 2 - Improved protocol for refolding FrpB, and mutants thereof

Inclusion bodies were solubilized in 6 M Guanidium hydrochloride and subsequently quickly (vortex) diluted 1:20 in 20-27 mM Ethanolamine (no HCl or NaOH added) + 0.5 % SB-12 (Zwittergent 3-12). The next day, the buffer was exchanged for Hepes pH 7.5 + 0.3 % SB-12 to stabilize the proteins.

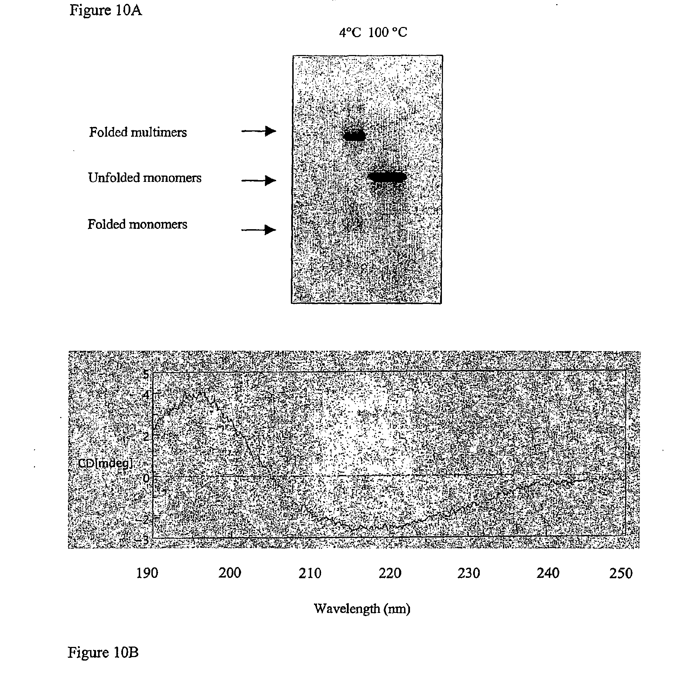

This improved protocol prevents the occurrence of aggregates during refolding. In Figure 10A a picture of *in vitro* folded FrpB according to the new protocol is shown, together with its corresponding CD spectrum recorded on a Jasco-810 spectropolarimeter [average of 30 scans] (Fig. 10B).

### Example 3: Evaluation of the immune response against in vitro folded FrpB (mutants)

Immunization experiments were performed with *in vitro* folded FrpB, FrpBΔ28 and FrpBΔ5/Δ3 (new loop nomenclature - see fig 9). Sera were analysed by ELISA with *in vitro* folded FrpB as antigen.

These sera were analysed with a Western-blot on the parent strain and 16 heterologous stains (fig 10A). FrpB was detected in all strains, indicating recognition of conserved epitopes. Improved detection of strains BNCV and 2996 (*) was observed with the antiserum raised against FrpBΔ5/Δ3.

Conclusion: Good responses were observed from all three immunizations, resulting in serum that was cross-reactive between stains. Furthermore, the enhanced recognition of strains 2996 and BNCV, suggests that the immune response can be re-directed towards different domains of the protein by immunization with a deletion mutant.

### Example 4: Immunization with OMVs carrying overexpressed FrpB (mutants) or with refolded wild-type FrpB

To analyse the immune response against OMVs carrying the FrpB (mutant) proteins, the proteins of interest (FrpB, FrpBΔ28, and FrpBΔ5/Δ3 [using the new Figure 9 loop nomenclature]) were overproduced in meningococci (pFP10 derived expression in CE2001 (H44/76 *PorA(-)).* A vaccine was produced according to Fredriksen et al. ( NIPH Ann. 1991 Dec;14(2):67-79; discussion 79-80). Immunization experiments were subsequently performed with the bleb vaccines as well as with refolded wild-type FrpB to obtain sera for SBA experiments against Nmen H44/76.

The preparations were tested in the OF1 mouse model. Animals, 10 per group, received three injections by the IM route on Day 0, 21 and 35. Per injection, mice received 5 µg of antigen formulated in adjuvant (Al³⁺ salts + 3DMPL). Blood samples were taken 7 days after the second and the third injection.

Pooled Post II and Post III sera as well as individual post III sera were tested in SBA on homologous (H44/76) and heterologous MenB strains as well as on a H44/76 FrpB KO strain.

| **Specific anti-FrpB antibodies by ELISA on pooled sera 7PII and 7PIII** | | | |
|---|---|---|---|
| | | | |
| | | | |
| **Group** | **Antigen** | **Mid-point Titers** | |
| | | 7PII | 7PIII |
| 1 | Frpb WT DOC extracts | 373 | 542 |
| 2 | FrpBD28 DOC extracts | 304 | 532 |
| 3 | FrpBD5/D3 DOC bleb | 212 | 423 |
| 4 | Frpb WT refolded | 3406 | 4837 |
| 5 | (-) | 25 | 25 |

The WT FrpB refolded and in blebs as well as mutants induced the production of anti-FrpB Abs (see ELISA table above).

Wild-type FrpB in a bleb, and wild-type refolded FrpB produced high bactericidal antibody titres against H44/76. The use of the H44/76 FrpB KO strain demonstrated that these bactericidal Abs are directed against FrpB. Blebs carrying FrpBΔ28 and FrpBΔ5/Δ3 produced low bactericidal antibody titres. Although blebs carrying FrpB, FrpBΔ28 and FrpBΔ5/Δ3 induced low bactericidal titres against a heterologous MenB strain M97250687, FrpBΔ28 blebs induced a higher SBA than the other two (with a titre over 1/100 dilution).

### Example 5: Epitope display in FrpB loop positions

As stated previously, sequence variation in FrpB is mostly confined to two domains of the protein, corresponding to the proposed loop L3 and loop L5 of the new model (Figure 9). Of these two loops, loop L5 was previously shown to be highly immunogenic and bactericidal antibodies are directed against this part of the protein. To make use of the tolerance of variation in this loop and its ability to induce bactericidal antibodies in the wild-type configuration, this part of the protein was chosen to serve as an insertion site of foreign epitopes.

As proof of principle, a very well studied epitope of PorA from H44/76 was selected to function as the foreign epitope. The high variable region corresponding to loop L5 of FrpB (new model) (TTEEKNGQKVDKPMEQQMKDRADEDTVH) was removed and replaced by a short sequence consisting of two restriction sites (MunI and NheI) with a short sequence encoding a PorA 1.7,16 fragment from loop 4 [FrpB-C1 containing QLKDTNNNAS, sequence corresponding to loop 4 or PorA are underlined, QL and AS flanking the PorA sequence are encoded by the restriction sites incorporated in the carrier protein].

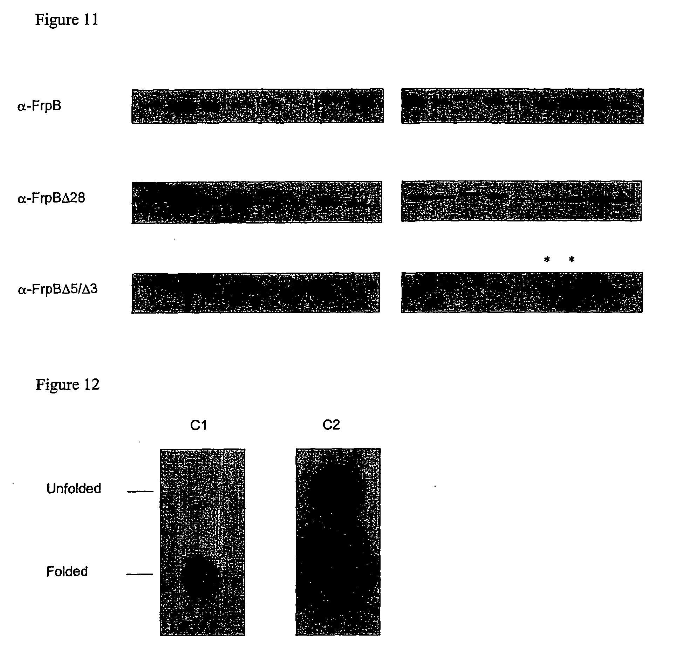

The flanking restriction sites were subsequently used to insert an oligonucleotide encoding a larger part of loop 4 [FrpB-C2 - QLKSAYTPAYYTKDTNNNLTLVPAVVAS]. Both genes were subsequently transferred without signal sequence to pET11a (Novagen) plasmids. Protein expression was performed in *Escherichia coli* BL21. Expression of these constructs resulted in inclusion bodies, which were isolated. The inclusion bodies were subsequently solubilized and *in vitro* folded according to the protocol described earlier. A Western blot as well as a dot-blot assay (Figure 12) was performed with the monoclonal antibody MN5C11G (1:100,000) (Toropainen et al 2001 Microb Pathog 30:139), which recognizes the minimal epitope KDTNNN of P1.7,16.

Both chimeric proteins were successfully refolded *in vitro* and detection of the proteins with the PorA antibody indicated correct insertion of the foreign fragments. Strikingly, folded forms of the chimeric proteins reacted more efficiently as compared with denatured protein, suggesting correct folding of the foreign epitopes in FrpB.

### Example 6: Mimotope display in FrpB loop positions

FrpB loops are also ideal for displaying peptide mimotopes (for instance of meningococcal LOS). Displaying mimotopes in a correctly folded context allows an immune response against LOS, for example, to be generated without having the toxic effects of LOS present in the vaccine.

Mab216 is a monoclonal antibody that recognizes an epitope of the inner core LOS. By biopanning on a library of cyclized heptamer peptides with this Mab, a mimetic peptide was identified in the art (Ian Feavers).

Six amino acids of the peptide were inserted into FrpB loop 5 (new loop nomenclature of Figure 9) and the chimera was over expressed (pFP10 plasmid) in H44/76 *Neisseria* in an LOS negative context (*lpxA* Knock Out mutant). See sequence of resulting protein below. OMVs were purified and injected into mice (OF1 female 6-8 weeks old, 3 injections DO-21-28 by IM route; 5 ug of blebs in ALOH/3DMPL were administered and sera collected on day 41). By Western Blot, Mab 216 was found to recognize the FrpB-mimotope.

### SEQUENCES:

The following pages show the sequence of FrpBΔ28 and FrpBΔ5/Δ3 [new loop nomenclature] (which contains a point mutation resulting in one amino-acid change). The sequences depicted here correspond to the genes which are present in the pFP10 plasmid. Therefore, the signal sequence is still present. For expression of the proteins in inclusion bodies in *E*. *coli,* the corresponding genes without their signal sequences were used. For the constructs without signal sequence (pET11a), the "G" at position 110 corresponds to the final nucleotide of the newly created ATG.
FrpBΔ28: Amino acids which are deleted in double mutant are shown in BOLD-UNDERLINED. 
MNTPLFRLSLLSLTLAAGFAHAAENNAKVVLDTVTVKGDRQGSKIRTNIVTLQQKDE
STATDMRELLKEEPSIDFGGGNGTSQFLTLRGMGQNSVDIKVDNAYSDSQILYHQGR
FIVDPALVKVVSVQKGAGSASAGIGATNGAIIAKTVDAQDLLKGLDKNWGVRLNSGF
ASNEGVSYGASVFGKEGNFDGLFSYNRNDEKDYEAGKGFRNVNGGKTVPYSALDKRS
 
YLAKIGTTFGDDDHRIVLSHMKDQHRGIRTVREEFTVGDKSSRINIDRQAPAYRETT
QSNTNLAYTGKNLGFVEKLDANAYVLEKERYSADDSGTGYAGNVKGPNHTRITTRGA
NFNFDSRLAEQTLLKYGINYRHQEIKPQAFLNSKFSIPAYKLSNPTKTDTGVYVEAI
HDIGDFTLTGGLRYDRFKVKTHDGKTVSSSNLNPSFGVIWQPHEHWSFSASHNYASR
SPRLYDALQTHGKRGIISIADGTKAERARNTEIGFNYNDGTFAANGSYFWQTIKDAL
ANPQNRHDSVAVREAVNAGYIKNHGYELGASYRTGGLTAKVGVSHSKPRFYDTHKDK
LLSANPEFGAQVGRTWTASLAYRFQNPNLEIGWRGRYVQKATGSILAAGQKDRKGNL
ENVVRKGFGVNDVFANWKPLGKDTLNVNLSVNNVFNKFYYPHSQRWTNTLPGVGRDV
RLGVNYKF
FrpBΔ5/A3: Point mutation results in amino-acid change K to R in loop 6 (BOLD-UNDERLINED) 
MNTPLFRLSLLSLTLAAGFAHAAENNAKVVLDTVTVKGDRQGSKIRTNIVTLQQKDE
STATDMRELLKEEPSIDFGGGNGTSQFLTLRGMGQNSVDIKVDNAYSDSQILYHQGR
FIVDPALVKVVSVQKGAGSASAGIGATNGAIIAKTVDAQDLLKGLDKNWGVRLNSGF
ASNEGVSYGASVFGKEGNFDGLFSYNRNDEKDYEAGKGFRNVNGGKTVPYSALDKRS
YLAKIGTTFGDDDHRIVLSHMKDRQAPAYRETTQSNTNLAYTGKNLGFVEKLDANAY
VLEKERYSADDSGTGYAGNVKGPNHTRITTRGANFNFDSRLAEQTLLKYGINYRHQE
IKPQAFLNSKFSIPAYKLSNPTKTDTGVYVEAIHDIGDFTLTGGLRYDRFKVKTHDG
RTVSSSNLNPSFGVIWQPHEHWSFSASHNYASRSPRLYDALQTHGKRGIISIADGTK
AERARNTEIGFNYNDGTFAANGSYFWQTIKDALANPQNRHDSVAVREAVNAGYIKNH
GYELGASYRTGGLTAKVGVSHSKPRFYDTHKDKLLSANPEFGAQVGRTWTASLAYRF
QNPNLEIGWRGRYVQKATGSILAAGQKDRKGNLENVVRKGFGVNDVFANWKPLGKDT
LNVNLSVNNVFNKFYYPHSQRWTNTLPGVGRDVRLGVNYKF
FrpBΔ28: START IS INDICATED IN BOLD AND UNDERLINED 
GGCCGCTTGGATTTACTTCCCTTCATACTCAAGAGGACGATTGAATGAATACCCCAT
TGTTCCGTCTCAGCCTGCTCTCGCTCACACTTGCGGCAGGTTTTGCCCACGCGGCAG
AAAATAATGCCAAGGTCGTACTGGATACCGTTACTGTAAAGGCGACCGCCAAGGCA
GCAAAATCCGTACCAACATCGTTACGCTGCAACAAAAAGACGAAAGCACCGCAACCG
ATATGCGCGAACTCTTAAAAGAAGAGCCGTCCATCGATTTCGGCGGCGGCAACGGCA
CGTCCCAATTCCTGA
CGCTGCGCGGCATGGGTCAGAACTCTGTCGACATCAAGGTGGACAACGCCTATTCCG
ACAGCCAAATCCTTTACCACCAAGGCAGATTTATTGTCGATCCCGCTTTGGTTAAAG
TCGTTTCCGTACAAAAAGGCGCGGGTTCCGCCTCTGCCGGTATCGGCGCGACCAACG
GCGCGATCATCGCCAAAACCGTCGATGCCCAAGACCTGCTCAAAGGCTTGGATAAAA
ACTGGGGCGTGCGCCTCAACAGCGGCTTTGCCAGCAACGAAGGCGTAAGCTACGGCG
CAAGCGTATTCGGAA
AAGAGGGCAACTTCGACGGCTTGTTCTCTTACAACCGCAACGATGAAAAAGATTACG
AAGCCGGCAAAGGTTTCCGCAATGTCAACGGCGGCAAAACCGTACCGTACAGCGCGC
TGGACAAACGCAGCTACCTCGCCAAAATCGGAACAACCTTCGGCGACGACGACCACC
GCATCGTGTTGAGCCACATGAAAGACCAACACCGGGGCATCCGCACTGTGCGTGAAG
AATTTACCGTCGGCGACAAAAGTTCACGGATAAATATTGACCGCCAAGCCCCTGCTT
ACCGCGAAACTACCCAATCCAACACCAACTTGGCGTACACGGGTAAAAACCTGGGCT
TTGTCGAAAAACTGGATGCCAACGCCTATGTGTTGGAAAAAGAACGCTATTCCGCCG
ATGACAGCGGCACCGGCTACGCAGGCAATGTAAAAGGCCCCAACCATACCCGAATCA
CCACTCGTGGTGCGAACTTCAACTTCGACAGCCGCCTTGCCGAACAAACCCTGTTGA
AATACGGTATCAACTACCGCCATCAGGAAATCAAACCGCAAGCATTTTTGAACTCGA
 
AATTCTCCATCCCCGCCTACAAACTTTCCAACCCGACCAAAACCGATACCGGCGTAT
ATGTTGAAGCCATTCACGACATCGGCGATTTCACGCTGACCGGCGGCTGCGTTACG
ACCGCTTCAAGGTGAAAACCCATGACGGCAAAACCGTTTCAAGCAGCAACCTTAACC
CGAGTTTCGGTGTGATTTGGCAGCCGCACGAACACTGGAGCTTCAGCGCGAGCCACA
ACTACGCCAGCCGCAGCCCGCGCCTGTATGACGCGCTGCAAACCCACGGTAAACGCG
GCATCATCTCGATTGCCGACGGCACAAAAGCCGAACGCGCGCAATACCGAAATCG
GCTTCAACTACAACGACGGCACGTTTGCCGCAAACGGCAGCTACTTCTGGCAGACCA
TCAAAGACGCGCTTGCCAATCCGCAAAACCGCCACGACTCTGTCGCCGTCCGTGAAG
CCGTCAATGCCGGTTACATCAAAAACCACGGTTACGAATTGGGCGCGTCCTACCGCA
CCGGCGGCCTGACTGCCAAAGTCGGCGTCAGCCACAGCAAACCGCGCTTTTACGATA
CGCACAAAGACAAGCTGTTGAGCGCGAATCCTGAATTTGGCGCACAAGTCGGCCGCA
CTTGGACGGCCTCCCTTGCCTACCGCTTCCAAAATCCGAATCTGGAAATCGGCTGGC
GCGGCCGTTATGTTCAAAAAGCTACGGGTTCGATATTGGCGGCAGGTCAAAAAGACC
GCAAAGGCAACTTGGAAAACGTTGTACGCAAAGGTTTCGGTGTGAACGATGTCTTCG
CCAACTGGAAACCGCTGGGCAAAGACACGCTCAATGTCAATCTTTCGGTTAACAACG
TGTTCAACAAGTTCTACTATCCGCACAGCCAACGCTGGACCAATACCCTGCCGGGCG
TGGGACGTGATGTACGCT
TGGGCGTGAACTACAAGTTCTAAAACGCACGACGT
FrpBΔ5/Δ3: START AND POINT MUTATION ARE INDICATED IN BOLD + UNDERLINED 
GGCCGCTTGGATTTACTTCCCTTCATACTCAAGAGGACGATTGAATGAATACCCCAT
TGTTCCGTCTCAGCCTGCTCTCGCTCACACTTGCGGCAGGTTTTGCCCACGCGGCAG
AAAATAATGCCAAGGTCGTACTGGATACCGTTACTGTAAAGGCGACCGCCAAGGCA
GCAAAATCCGTACCAACATCGTTACGCTGCAACAAAAAGACGAAAGCACCGCAACCG
ATATGCGCGAACTCTTAAAAGAAGAGCCGTCCATCGATTTCGGCGGCAACGGCA
CGTCCCAATTCCTGA
CGCTGCGCGGCATGGGTCAGAACTCTGTCGACATCAAGGTGGACAACGCCTATTCCG
ACAGCCAAATCCTTTACCACCAAGGCAGATTTATTGTCGATCCCGCTTTGGTTAAAG
TCGTTTCCGTACAAAAAGGCGCGGGTTCCGCCTCTGCCGGTATCGGCGCGACCAACG
GCGCGATCATCGCCAAAACCGTCGATGCCCAAGACCTGCTCAAAGGCTTGGATAAAA
ACTGGGGCGTGCGCCTCAACAGCGGCTTTGCCAGCAACGAAGGCGTAAGCTACGGCG
CAAGCGTATTCGGAA
AAGAGGGCAACTTCGACGGCTTGTTCTCTTACAACCGCAACGATGAAAAAGATTACG
AAGCCGGCAAAGGTTTCCGCAATGTCAACGGCGGCAAAACCGTACCGTACAGCGCGC
TGGACAAACGCAGCTACCTCGCCAAAATCGGAACAACCTTCGGCGACGACGACCACC
GCATCGTGTTGAGCCACATGAAAGACCGCCAAGCCCCTGCTTACCGCGAAACTACCC
AATCCAACACCAACTTGGCGTACACGGGTAAAAACCTGGGCTTTGTCGAAAAACTGG
ATGCCAACGCCTATGTGTTGGAAAAAGAACGCTATTCCGCCGATGACAGCGGCACCG
GCTACGCAGGCAATGTAAAAGGCCCCAACCATACCCGAATCACCACTCGTGGTGCGA
ACTTCAACTTCGACAGCCGCCTTGCCGAACAAACCCTGTTGAAATACGGTATCAACT
ACCGCCATCAGGAAATCAAACCGCAAGCATTTTTGAACTCGAAATTCTCCATCCCCG
CCTACAAACTTTCCAACCCGACCAAAACCGATACCGGCGTATATGTTGAAGCCATTC
ACGACATCGGCGATTTCACGCTGACCGGCGGGCTGCGTTACGACCGCTTCAAGGTGA
AAACCCATGACGGCAGAACCGTTTCAAGCAGCAACCTTAACCCGAGTTTCGGTGTGA
TTTGGCAGCCGCACGAACACTGGAGCTTCAGCGCGAGCCACAACTACGCCAGCCGCA
GCCCGCGCCTGTATGACGCGCTGCAAACCCACGGTAAACGCGGCATCATCTCGATTG
CCGACGGCACAAAAGCCGAACGCGCGCAATACCGAAATCGGCTTCAACTACAACG
ACGGCACGTTTGCCGCAAACGGCAGCTACTTCTGGCAGACCATCAAAGACGCGCTTG
CCAATCCGCAAAACCGCCACGACTCTGTCGCCGTCCGTGAAGCCGTCAATGCCGGTT
 
ACATCAAAAACCACGGTTACGAATTGGGCGCGTCCTACCGCACCGGCGGCCTGACTG
CCAAAGTCGGCGTCAGCCACAGCAAACCGCGCTTTTACGATACGCACAAAGACAAGC
TGTTGAGCGCGAATCCTGAATTTGGCGCACAAGTCGGCCGCACTTGGACGGCCTCCC
TTGCCTACCGCTTCCAAAATCCGAATCTGGAAATCGGCTGGCGCGGCCGTTATGTTC
AAAAAGCTACGGGTTCGATATTGGCGGCAGGTCAAAAAGACCGCAAAGGCAACTTGG
AAAACGTTGTACGCAAAGGTTTCGGTGTGAACGATGTCTTCGCCAACTGGAAACCGC
TGGGCAAAGACACGCTCAATGTCAATCTTTCGGTTAACAACGTGTTCAACAAGTTCT
ACTATCCGCACAGCCAACGCTGGACCAATACCCTGCCGGGCGTGGACGTGATGTAC
GCTTGGGCGTGAACTACAAGTTCTAAAACGCACGACGT
FrpB-mimo (start and mimotope insert are in bold and underlined) 
TGTTAATATAAATAAAAATAATTATTAATTATTTTTCTTATCCTGCCAAATCTTAACGGTTTGGATTTA
CTTCCCTTCATACTCAAGAGGACGATTGAATGAATACCCCATTGTTCCGTCTCAGCCTGCTCTCGCTCA
CACTTGCGGCAGGTTTTGCCCACGCGGCAGAAAATAATGCCAAGGTCGTACTGGATACCGTTACTGTAA
AAGGCGACCGCCAAGGCAGCAAAATCCGTACCAACATCGTTACGCTGCAACAAAAAGACGAAAGCACCG
CAACCGATATGCGCGAACTCTTAAAAGAAGAGCCGTCCATCGATTTCGGCGGCGGCAACGGCACGTCCC
AATTCCTGACGCTGCGCGGCATGGGTCAGAACTCTGTCGACATCAAGGTGGACAACGCCTATTCCGACA
GCCAAATCCTTTACCACCAAGGCAGATTTATTGTCGATCCCGCTTTGGTTAAAGTCGTTTCCGTACAAA
AAGGCGCGGGTTCCGCCTCTGCCGGTATCGGCGCGACCAACGGCGCGATCATCGCCAAAACCGTCGATG
CCCAAGACCTGCTCAAAGGCTTGGATAAAAACTGGGGCGTGCGCCTCAACAGCGGCTTTGCCAGCAACG
AAGGCGTAAGCTACGGCGCAAGCGTATTCGGAAAAGAGGGCAACTTCGACGGCTTGTTCTCTTACAACC
GCAACGATGAAAAAGATTACGAAGCCGGCAAAGGTTTCCGCAATGTCAACGGCGGCAAAACCGTACCGT
ACAGCGCGCTGGACAAACGCAGCTACCTCGCCAAAATCGGAACAACCTTCGGCGACGACGACCACCGCA
TCGTGTTGAGCCACATGAAAGACCAACACCGGGGCATCCGCACTGTGCGTGAAGAATTTACCGTCGGCG
ACAAAAGTTCACGGATAAATATTGACCGCCAAGCCCCTGCTTACCGCGAAACTACCCAATCCAACACCA
ACTTGGCGTACACGGGTAAAAACCTGGGCTTTGTCGAAAAACTGGATGCCAACGCCTATGTGTTGGAAA
AAGAACGCTATTCCGCCGATGACAGCGGCACCGGCTACGCAGGCAATGTAAAAGGCCCCAACCATACCC
GAATCACCACTCGTGGTGCGAACTTCAACTTCGACAGCCGCCTTGCCGAACAAACCCTGTTGAAATACG
GTATCAACTACCGCCATCAGGAAATCAAACCGCAAGCATTTTTGAACTCGAAATTCTCCATCCCGACGA
CAGAAGAGAAAAACGGTCAAAAAGTCGATGAAGTCTTATTTCGTGGCACGAAACCGATGGAACAACAAA
TGAAAGACCGTGCAGATGAAGACACTGTTCACGCCTACAAACTTTCCAACCCGACCAAAACCGATACCG
GCGTATATGTTGAAGCCATTCACGACATCGGCGATTTCACGCTGACCGGCGGGCTGCGTTACGACCGCT
TCAAGGTGAAAACCCATGACGGCAAAACCGTTTCAAGCAGCAACCTTAACCCGAGTTTCGGTGTGATTT
GGCAGCCGCACGAACACTGGAGCTTCAGCGCGAGCCACAACTACGCCAGCCGCAGCCCGCGCCTGTATG
ACGCGCTGCAAACCCACGGTAAACGCGGCATCATCTCGATTGCCGACGGCACAAAAGCCGAACGCGCGC
GCAATACCGAAATCGGCTTCAACTACAACGACGGCACGTTTGCCGCAAACGGCAGCTACTTCTGGCAGA
CCATCAAAGACGCGCTTGCCAATCCGCAAAACCGCCACGACTCTGTCGCCGTCCGTGAAGCCGTCAATG
CCGGTTACATCAAAAACCACGGTTACGAATTGGGCGCGTCCTACCGCACCGGCGGCCTGACTGCCAAAG
TCGGCGTCAGCCACAGCAAACCGCGCTTTTACGATACGCACAAAGACAAGCTGTTGAGCGCGAATCCTG
AATTTGGCGCACAAGTCGGCCGCACTTGGACGGCCTCCCTTGCCTACCGCTTCCAAAATCCGAATCTGG
AAATCGGCTGGCGCGGCCGTTATGTTCAAAAAGCTACGGGTTCGATATTGGCGGCAGGTCAAAAAGACC
GCAAAGGCAACTTGGAAAACGTTGTACGCAAAGGTTTCGGTGTGAACGATGTCTTCGCCAACTGGAAAC
CGCTGGGCAAAGACACGCTCAATGTCAATCTTTCGGTTAACAACGTGTTCAACAAGTTCTACTATCCGC
ACAGCCAACGCTGGACCAATACCCTGCCGGGCGTGGGACGTGATGTACGCTTGGGCGTGAACTACAAGT
TCTAAAACGCACATCCCGAAAAAATGCCGTCTGAAAGCCTTTCAGACGGCA
FrpB-mimo amino-acid sequence (start and mimotope insert are in bold and underlined)

MNTPLFRLSLLSLTLAAGFAHAAENNAKVVLDTVTVKGDRQGSKIRTNIVTLQQKDESTATDMRELLKE
EPSIDFGGGNGTSQFLTLRGMGQNSVDIKVDNAYSDSQILYHQGRFIVDPALVKVVSVQKGAGSASAGI
GATNGAIIAKTVDAQDLLKGLDKNWGVRLNSGFASNEGVSYGASVFGKEGNFDGLFSYNRNDEKDYEAG
KGFRNVNGGKTVPYSALDKRSYLAKIGTTFGDDDHRIVLSHMKDQHRGIRTVREEFTVGDKSSRINIDR
QAPAYRETTQSNTNLAYTGKNLGFVEKLDANAYVLEKERYSADDSGTGYAGNVKGPNHTRITTRGANFN
FDSRLAEQTLLKYGI
NYRHQEIKPQAFLNSKFSIPTTEEKNGQKVDEVLFRGTKPMEQQMKDRADEDTVHAYKLSNPTKTDTGV
YVEAIHDIGDFTLTGGLRYDRFKVKTHDGKTVSSSNLNPSFGVIWQPHEHWSFSASHNYASRSPRLYDA
LQTHGKRGIISIADGTKAERARNTEIGFNYNDGTFAANGSYFWQTIKDALANPQNRHDSVAVREAVNAG

YIKNHGYELGASYRTGGLTAKVGVSHSKPRFYDTHKDKLLSANPEFGAQVGRTWTASLAYRFQNPNLEI
GWRGRYVQKATGSILAAGQKDRKGNLENVVRKGFGVNDVFANWKPLGKDTLNVNLSVNNVFNKFYYPHS
QRWTNTLPGVGRDVRLGVNYKF

## Claims

1. A neisserial FrpB protein having one or more deletions of non-conserved amino acids of loop 7 and loop 5, according to the loop numbering of Figure 6, compared to a corresponding wild-type neisserial FrpB protein.

2. The protein according to claim 1 which is cross-protective.

3. The protein according to any preceding claim in which one or more amino acids of any one or more of loops 1, 2, 3, 4, 6, 8, 9, 10, 11, 12 and 13 have been deleted.

4. The protein according to any preceding claim in which 11 to 33 amino acids have been deleted from loop 7.

5. The protein according to any preceding claim in which 23-33 amino acids have been deleted from loop 7.

6. The protein according to any preceding claim in which 28 amino acids have been deleted from loop 7.

7. The protein according to any preceding claim in which 18-29 amino acids have been deleted from loop 5.

8. The protein according to any preceding claim in which 19-29 amino acids have been deleted from loop 5.

9. The protein according to any preceding claim in which 24 amino acids have been deleted from loop 5.

10. The protein according to any preceding claim in which, with reference to FrpB strain H44/76, the amino acid deletion is made in the range of amino acids 376-413, or a corresponding deletion made, from loop 7.

11. The protein according to any preceding claim in which, with reference to FrpB strain H44/76, the amino acid deletion is made in the range of amino acids 381-408, or a corresponding deletion made, from loop 7.

12. The protein according to any preceding claim in which, with reference to FrpB strain H44/76, an amino acid sequence comprising TTEEKNGQKVDKPMEQQMKDRADEDTVH has been deleted, or a corresponding deletion made, from loop 7.

13. The protein according to any preceding claim in which, with reference to FrpB strain H44/76, the amino acid deletion is made in the range of amino acids 247-280, or a corresponding deletion made, from loop 5.

14. The protein according to any preceding claim in which, with reference to FrpB strain H44/76, the amino acid deletion is made in the range of amino acids 252-275, or a corresponding deletion made, from loop 5.

15. The protein according to any preceding claim in which, with reference to FrpB strain H44/76, an amino acid sequence comprising QHRGIRTVREEFTVGDKSSRINID has been deleted, or a corresponding deletion made, from loop 5.

16. The protein of any preceding claim in which the deleted sequence is replaced by another amino acid sequence.

17. The protein of claim 16 in which the sequence is deleted by mutagenesis.

18. A polynucleotide encoding the protein of any preceding claim.

19. An expression vector comprising the polynucleotide of claim 18

20. A host cell comprising the expression vector of claim 19.

21. A method for producing the protein of any one of claims 1 to 17 comprising:
culturing the host cell of claim 20, and recovering the expressed protein.

22. A method for refolding an FrpB protein comprising contacting the FrpB protein with an alkaline refolding buffer comprising 3-dimethyldodecylammoniopropanesulfonate (Zwittergent 3-12 or SB-12), wherein the protein is a protein according to any one of claims 1 to 17.

23. A method according to claim 22 wherein the refolding buffer comprises ethanolamine and SB-12.

24. A method according to claim 23 wherein the ethanolamine is 20mM ethanolamine.

25. A method according to any one of claims 22 to 24 wherein the refolding buffer has pH11.

26. A method according to any one of claims 22 to 25 wherein the SB-12 is 0.2% SB-12.

27. A method according to any one of claims 22 to 25 wherein the SB-12 is 0.5% SB-12.

28. A method according to any one of claims 22 to 27 wherein the refolding buffer further comprises guanidium chloride, NaCl and/or urea.

29. A method according to claim 28 wherein the refolding buffer further comprises guanidium chloride.

30. A method according to claim 29 wherein the refolding buffer further comprises 0.4M guanidium chloride.

31. A method of any one of claims 22 to 30 comprising the following steps:
a. expressing an FrpB protein in a host cell; breaking the host cell to obtain an inclusion body comprising the FrpB protein; washing the inclusion body;
b. solubilisation of at least part of the inclusion body and the FrpB protein with Guanidinium hydrochloride;
c. contacting the solubilised FrpB protein with the refolding buffer; and
d. removing or changing the refolding buffer from the FrpB protein.

32. A refolding buffer comprising ethanolamine, SB-12 and guandinium chloride for use in the method of any one of claims 22 to 31.

33. An isolated, refolded FrpB protein of claims 1-17 obtained or obtainable by the method of any one of claims 22 to 31.

34. A pharmaceutical composition comprising at least one FrpB protein of any one of claims 1 to 17 and 33, and a pharmaceutically acceptable carrier.

35. A pharmaceutical composition according to claim 34 wherein at least 30%, 50%, 70%, or 90% of the FrpB protein present in the composition is refolded.

36. A pharmaceutical composition according to claim 34 or 35 in the form of a vaccine.

37. The pharmaceutical composition of any one of claims 34 to 36 comprising a FrpB protein derived from *Neisseria meningitidis.*

38. The pharmaceutical composition of any one of claims 34 to 37 comprising a FrpB protein derived from *Neisseria gonorrhoeae.*

39. The pharmaceutical composition according to any one of claims 34 to 38 wherein said composition comprises at least one other Neisserial antigen.

40. The pharmaceutical composition of claim 39 comprising at least one other Neisserial antigen derived from *Neisseria gonorrhoeae.*

41. The pharmaceutical composition of claim 39 or 40 comprising at least one other Neisserial antigen derived from *Neisseria meningitidis.*

42. The pharmaceutical composition of any one of claims 34 to 41 in the form of a subunit composition.

43. The pharmaceutical composition of any one of claims 34 to 41 in the form of an outer membrane vesicle preparation, or a mixed subunit plus outer membrane vesicle preparation.

44. A pharmaceutical composition according to any one of claims 34 to 43 further comprising at least one other Neisserial antigen selected from one or more of the following classes:
a. at least one Neisserial adhesin selected from the group consisting of FhaB, NspA, Hsf, NadA, PilC, Hap, MafA, MafB, Omp26, NMB0315, NMB0995 and NMB1119;
b. at least one Neisserial autotransporter selected from the group consisting of Hsf, Hap, IgA protease, AspA and NadA;
c. at least one Neisserial toxin selected from the group consisting of FrpA, FrpC, FrpA/C, VapD, NM-ADPRT, and either or both of LPS immunotype L2 and LPS immunotype L3;
d. at least one Neisserial Fe acquisition protein selected from the group consisting of TbpA high, TbpA low, TbpB high, TbpB low, LbpA, LbpB, P2086, HpuA, HpuB, Lipo28, Sibp, FbpA, BfrA, BfrB, Bcp, NMB0964 and NMB0293; and
e. at least one Neisserial membrane associated protein, preferably outer membrane protein, selected from the group consisting of PldA, NspA, TspA, FhaC, NspA, TbpA(high), TbpA(low), LbpA, HpuB, TdfH, PorB, HimD, HisD, GNA1870, OstA, HlpA, MltA, NMB 1124, NMB 1162, NMB 1220, NMB 1313, NMB 1953, HtrA, TspB, PilQ and OMP85.

45. The pharmaceutical composition of any one of claims 34 to 44 further comprising one or more bacterial capsular polysaccharides or oligosaccharides.

46. The pharmaceutical composition of claim 45 wherein the one or more capsular polysaccharides or oligosaccharides is derived from bacteria selected from the group consisting of *Neisseria meningitidis* serogroup A, C, Y, and/or W-135, *Haemophilus influenzae* b, *Streptococcus pneumoniae,* Group A Streptococci, Group B Streptococci, *Staphylococcus aureus* and *Staphylococcus epidermidis,* and are conjugated to a source ot T-helper epitopes.

47. Use of an FrpB protein of any one of claims 1 to 17 and 33 (or a pharmaceutical composition of claims 34 to 46) in the preparation of a medicament for treatment or prevention of Neisserial infection.

48. The use of claim 47 in which *Neisseria meningitidis* infection is prevented or treated.

49. The use of claims 47 or 48 in which *Neisseria gonorrhoeae* infection is prevented or treated.

50. An antibody immunospecific for the FrpB protein as claimed in any one of claims 1 to 17 and 33.

51. A pharmaceutical composition useful in treating humans with a Neisserial disease comprising at least one antibody according to claim 50 and a suitable pharmaceutical carrier.

52. Use of the antibody of claim 50 (or a composition of claim 51) in the manufacture of a medicament for the treatment or prevention of Neisserial disease.

53. The use of claim 52 in which *Neisseria meningitidis* infection is prevented or treated.

54. The use of claim 52 or 53 in which *Neisseria gonorrhoeae* infection is prevented or treated.

55. A method of diagnosing a Neisserial infection, comprising the steps of identifying an FrpB protein, or an antibody thereto, within a biological sample from an animal suspected of having such an infection using a FrpB protein as claimed in any one of claims 1 to 17 and 33, or an antibody as claimed in claim 50.

56. The method of claim 55 in which *Neisseria meningitidis* infection is diagnosed.

57. The method of claim 55 or 56 in which *Neisseria gonorrhoeae* infection is diagnosed.

## Patentansprüche

1. Neisseria-FrpB-Protein mit einer oder mehreren Deletionen nicht-konservierter Aminosäure der Schleife 7 und Schleife 5, gemäß der Schleifen-Numerierung der Figur 6, im Vergleich zu einem entsprechenden Wildtyp-Neisseria-FrpB-Protein.

2. Protein gemäß Anspruch 1, das kreuzprotektiv ist.

3. Protein gemäß einem der vorangehenden Ansprüche, in dem eine oder mehrere Aminosäuren einer oder mehrerer der Schleifen 1, 2, 3, 4, 6, 8, 9, 10, 11, 12 und 13 deletiert worden sind.

4. Protein gemäß einem der vorangehenden Ansprüche, in dem 11 bis 33 Aminosäuren von Schleife 7 deletiert worden sind.

5. Protein gemäß einem der vorangehenden Ansprüche, in dem 23 bis 33 Aminosäuren von Schleife 7 deletiert worden sind.

6. Protein gemäß einem der vorangehenden Ansprüche, in dem 28 Aminosäuren von Schleife 7 deletiert worden sind.

7. Protein gemäß einem der vorangehenden Ansprüche, in dem 18 bis 29 Aminosäuren von Schleife 5 deletiert worden sind.

8. Protein gemäß einem der vorangehenden Ansprüche, in dem 19 bis 29 Aminosäuren von Schleife 5 deletiert worden sind.

9. Protein gemäß einem der vorangehenden Ansprüche, in dem 24 Aminosäuren von Schleife 5 deletiert worden sind.

10. Protein gemäß einem der vorangehenden Ansprüche, in dem mit Bezug auf den FrpB-Stamm H44/76 die Aminosäuredeletion im Bereich der Aminosäuren 376 bis 413 von Schleife 7 erfolgt ist oder eine erfolgte entsprechenden Deletion.

11. Protein gemäß einem der vorangehenden Ansprüche, in dem mit Bezug auf den FrpB-Stamm H44/76 die Aminosäuredeletion im Bereich der Aminosäuren 381 bis 408 von Schleife 7 erfolgt ist oder eine erfolgte entsprechenden Deletion.

12. Protein gemäß einem der vorangehenden Ansprüche, in dem mit Bezug auf den FrpB-Stamm H44/76 eine Aminosäuresequenz, umfassend TTEEKNGQKVDKPMEQQMKDRADEDTVH, von Schleife 7 deletiert worden ist oder eine erfolgte entsprechende Deletion.

13. Protein gemäß einem der vorangehenden Ansprüche, in dem mit Bezug auf den FrpB-Stamm H44/76 die Aminosäuredeletion im Bereich der Aminosäuren 247 bis 280 von Schleife 5 erfolgt ist oder eine erfolgte entsprechenden Deletion.

14. Protein gemäß einem der vorangehenden Ansprüche, in dem mit Bezug auf den FrpB-Stamm H44/76 die Aminosäuredeletion im Bereich der Aminosäuren 252 bis 275 von Schleife 5 erfolgt ist oder eine erfolgte entsprechenden Deletion.

15. Protein gemäß einem der vorangehenden Ansprüche, in dem mit Bezug auf den FrpB-Stamm H44/76 eine Aminosäuresequenz, umfassend QHRGIRTVREEFTVGDKSSRINID, von Schleife 5 deletiert worden ist oder eine erfolgte entsprechende Deletion.

16. Protein gemäß einem der vorangehenden Ansprüche, in dem die deletierte Sequenz durch eine andere Aminosäuresequenz ersetzt ist.

17. Protein gemäß Anspruch 16, in dem die Sequenz durch Mutagenese deletiert ist.

18. Polynukleotid, das für das Protein gemäß einem der vorangehenden Ansprüche codiert.

19. Expressionsvektor, der das Polynukleotid gemäß Anspruch 18 umfaßt.

20. Wirtszelle, die den Expressionsvektor gemäß Anspruch 19 umfaßt.

21. Verfahren zur Herstellung des Proteins gemäß einem der Ansprüche 1 bis 17, umfassend: Kultivieren der Wirtszelle gemäß Anspruch 20 und Gewinnen des exprimierten Proteins.

22. Verfahren zur Rückfaltung eines FrpB-Proteins, umfassend das Kontaktieren des FrpB-Proteins mit einem alkalischen Rückfaltungspuffer, der 3-Dimethyldodecylammoniopropansulfonat (Zwittergent 3-12 oder SB-12) umfaßt, wobei das Protein ein Protein gemäß einem der Ansprüche 1 bis 17 ist.

23. Verfahren gemäß Anspruch 22, wobei der Rückfaltungspuffer Ethanolamin und SB-12 umfaßt.

24. Verfahren gemäß Anspruch 23, wobei das Ethanolamin 20 mM Ethanolamin ist.

25. Verfahren gemäß einem der Ansprüche 22 bis 24, wobei der Rückfaltungspuffer einen pH-Wert von 11 aufweist.

26. Verfahren gemäß einem der Ansprüche 22 bis 25, wobei das SB-12 0,2 % SB-12 ist.

27. Verfahren gemäß einem der Ansprüche 22 bis 25, wobei das SB-12 0,5 % SB-12 ist.

28. Verfahren gemäß einem der Ansprüche 22 bis 27, wobei der Rückfaltungspuffer ferner Guanidiumchlorid, NaCl und/oder Harnstoff umfaßt.

29. Verfahren gemäß Anspruch 28, wobei der Rückfaltungspuffer ferner Guanidiumchlorid umfaßt.

30. Verfahren gemäß Anspruch 29, wobei der Rückfaltungspuffer ferner 0,4 M Guanidiumchlorid umfaßt.

31. Verfahren gemäß einem der Ansprüche 22 bis 30, umfassend die folgenden Schritte:
a. Exprimieren eines FrpB-Proteins in einer Wirtszelle; Aufbrechen der Wirtszellen zum Erhalten eines Einschlußkörpers, der das FrpB-Protein umfaßt; Waschen des Einschlußkörpers;
b. Solubilisierung wenigstens eines Teils des Einschlußkörpers und des FrpB-Proteins mit Guanidiniumhydrochlorid;
c. Kontaktieren des solubilisierten FrpB-Proteins mit dem Rückfaltungspuffer; und
d. Entfernen oder Ändern des Rückfaltungspuffers von dem FrpB-Protein.

32. Rückfaltungspuffer, umfassend Ethanolamin, SB-12 und Guanidiniumchlorid, zur Verwendung in dem Verfahren gemäß einem der Ansprüche 22 bis 31.

33. Isoliertes, rückgefaltenes FrpB-Protein gemäß den Ansprüchen 1 bis 17, erhalten oder erhältlich durch das Verfahren gemäß einem der Ansprüche 22 bis 31.

34. Pharmazeutische Zusammensetzung, die wenigstens ein FrpB-Protein gemäß einem der Ansprüche 1 bis 17 und 33 und einen pharmazeutisch annehmbaren Träger umfaßt.

35. Pharmazeutische Zusammensetzung gemäß Anspruch 34, worin wenigstens 30 %, 50 %, 70 % oder 90 % des in der Zusammensetzung vorliegenden FrpB-Proteins rückgefalten ist.

36. Pharmazeutische Zusammensetzung gemäß Anspruch 34 oder 35 in der Form eines Impfstoffs.

37. Pharmazeutische Zusammensetzung gemäß einem der Ansprüche 34 bis 36, die ein aus Neisseria meningitidis stammendes FrpB-Protein umfaßt.

38. Pharmazeutische Zusammensetzung gemäß einem der Ansprüche 34 bis 37, die ein aus Neisseria gonorrhoeae stammendes FrpB-Protein umfaßt.

39. Pharmazeutische Zusammensetzung gemäß einem der Ansprüche 34 bis 38, wobei die Zusammensetzung wenigstens ein weiteres Neisseria-Antigen umfaßt.

40. Pharmazeutische Zusammensetzung gemäß Anspruch 39, die wenigstens ein weiteres aus Neisseria gonorrhoeae stammendes Neisseria-Antigen umfaßt.

41. Pharmazeutische Zusammensetzung gemäß Anspruch 39 oder 40, die wenigstens ein weiteres aus Neisseria meningitidis stammendes Neisseria-Antigen umfaßt.

42. Pharmazeutische Zusammensetzung gemäß einem der Ansprüche 34 bis 41 in der Form einer Untereinheiten-Zusammensetzung.

43. Pharmazeutische Zusammensetzung gemäß einem der Ansprüche 34 bis 41 in der Form einer Außenmembranvesikel-Zubereitung oder einer gemischten Untereinheit zuzüglich einer Außenmembranvesikel-Zubereitung.

44. Pharmazeutische Zusammensetzung gemäß einem der Ansprüche 34 bis 43, ferner umfassend wenigstens ein weiteres Neisseria-Antigen, ausgewählt aus einer oder mehreren der folgenden Klassen:
a. wenigstens ein Neisseria-Adhäsin, ausgewählt aus der Gruppe, bestehend aus FhaB, NspA, Hsf, NadA, PilC, Hap, MafA, MafB, Omp26, NMB0315, NMB0995 und NMB1119;
b. wenigstens ein Neisseria-Autotransporter, ausgewählt aus der Gruppe, bestehend aus Hsf, Hap, IgA-Protease, AspA und NadA;
c. wenigstens ein Neisseria-Toxin, ausgewählt aus der Gruppe, bestehend aus FrpA, FrpC, FrpA/C, VapD, NM-ADPRT und entweder oder beide von LPS vom Immuntyp L2 und LPS vom Immuntyp L3;
d. wenigstens ein Fe-aufnehmendes Neisseria-Protein, ausgewählt aus der Gruppe, bestehend aus TbpA high, TbpA low, TbpB high, TbpB low, LbpA, LbpB, P2086, HpuA, HpuB, Lipo28, Sibp, FbpA, BfrA, BfrB, Bcp, NMB0964 und NMB0293; und
e. wenigstens ein Membran-assoziiertes Neisseria-Protein, vorzugsweise ein Außenmembranprotein, ausgewählt aus der Gruppe, bestehend aus PldA, NspA, TspA, FhaC, NspA, TbpA(high), TbpA(low), LbpA, HpuB, TdfH, PorB, HimD, HisD, GNA1870, OstA, HlpA, MltA, NMB 1124, NMB 1162, NMB 1220, NMB 1313, NMB 1953, HtrA, TspB, PilQ und OMP85.

45. Pharmazeutische Zusammensetzung gemäß einem der Ansprüche 34 bis 44, ferner umfassend ein oder mehrere bakterielle Kapsel-Polysaccharide oder -Oligosaccharide.

46. Pharmazeutische Zusammensetzung gemäß Anspruch 45, worin das eine oder die mehreren Kapsel-Polysaccharide oder -Oligosaccharide von Bakterien stammen, ausgewählt aus der Gruppe, bestehend aus Neisseria meningitidis Serogruppe A, C, Y und/oder W-135, Haemophilus influenzae b, Streptococcus pneumoniae, Streptococci der Gruppe A, Streptococci der Gruppe B, Staphylococcus aureus und Staphylococcus epidermidis, und an eine Quelle von T-Helfer-Epitopen konjugiert sind.

47. Verwendung eines FrpB-Proteins gemäß einem der Ansprüche 1 bis 17 und 33 (oder einer pharmazeutischen Zusammensetzung der Ansprüche 34 bis 46) in der Herstellung eines Medikaments zur Behandlung oder Vorbeugung einer Neisseria-Infektion.

48. Verwendung gemäß Anspruch 47, bei der eine(r) Neisseria meningitidis-Infektion vorgebeugt oder behandelt wird.

49. Verwendung gemäß Anspruch 47 oder 48, bei der eine(r) Neisseria gonorrhoeae-Infektion vorgebeugt oder behandelt wird.

50. Antikörper, der für das FrpB-Protein gemäß einem der Ansprüche 1 bis 17 und 33 immunspezifisch ist.

51. Pharmazeutische Zusammensetzung, die in der Behandlung von Menschen mit einer Neisseria-Erkrankung nützlich ist, umfassend wenigstens einen Antikörper gemäß Anspruch 50 und einen geeigneten pharmazeutischen Träger.

52. Verwendung des Antikörpers gemäß Anspruch 50 (oder einer Zusammensetzung gemäß Anspruch 51) in der Herstellung eines Medikaments zur Behandlung oder Vorbeugung einer Neisseria-Erkrankung.

53. Verwendung gemäß Anspruch 52, bei der eine(r) Neisseria meningitidis-Infektion vorgebeugt oder behandelt wird.

54. Verwendung gemäß Anspruch 52 oder 53, bei der eine(r) Neisseria gonorrhoeae-Infektion vorgebeugt oder behandelt wird.

55. Verfahren zur Diagnose einer Neisseria-Infektion, umfassend die Schritte der Identifizierung eines FrpB-Proteins oder eines dagegen gerichteten Antikörpers in einer biologischen Probe eines Tiers, für das angenommen wird, daß es solch eine Infektion aufweist, unter Verwendung eines FrpB-Proteins gemäß einem der Ansprüche 1 bis 17 und 33 oder eines Antikörpers gemäß Anspruch 50.

56. Verfahren gemäß Anspruch 55, bei dem eine Neisseria meningitidis-Infektion diagnostiziert wird.

57. Verfahren gemäß Anspruch 55 oder 56, bei dem eine Neisseria gonorrhoeae-Infektion diagnostiziert wird.

## Revendications

1. Protéine neissérienne FrpB présentant une ou plusieurs délétions d'acides aminés non conservés de la boucle 7 et de la boucle 5, selon la numérotation des boucles de la figure 6, comparativement à une protéine neissérienne FrpB de type sauvage correspondante.

2. Protéine selon la revendication 1 qui présente une réactivité croisée.

3. Protéine selon l'une quelconque des revendications précédentes, dans laquelle un ou plusieurs acides aminés de l'une quelconque ou de plusieurs des boucles 1, 2, 3, 4, 6, 8, 9, 10, 11, 12 et 13 ont été délétés.

4. Protéine selon l'une quelconque des revendications précédentes, dans laquelle 11 à 33 acides aminés ont été délétés de la boucle 7.

5. Protéine selon l'une quelconque des revendications précédentes, dans laquelle 23 à 33 acides aminés ont été délétés de la boucle 7.

6. Protéine selon l'une quelconque des revendications précédentes, dans laquelle 28 acides aminés ont été délétés de la boucle 7.

7. Protéine selon l'une quelconque des revendications précédentes, dans laquelle 18 à 29 acides aminés ont été délétés de la boucle 5.

8. Protéine selon l'une quelconque des revendications précédentes, dans laquelle 19 à 29 acides aminés ont été délétés de la boucle 5.

9. Protéine selon l'une quelconque des revendications précédentes, dans laquelle 24 acides aminés ont été délétés de la boucle 5.

10. Protéine selon l'une quelconque des revendications précédentes, dans laquelle, en référence à la FrpB de la souche H44/76, la délétion d'acides aminés est réalisée dans la plage des acides aminés 376 à 413 ou une délétion correspondante réalisée, à partir de la boucle 7.

11. Protéine selon l'une quelconque des revendications précédentes, dans laquelle, en référence à la FrpB de la souche H44/76, la délétion d'acides aminés est réalisée dans la plage des acides aminés 381 à 408 ou une délétion correspondante réalisée, à partir de la boucle 7.

12. Protéine selon l'une quelconque des revendications précédentes, dans laquelle, en référence à la FrpB de la souche H44/76, une séquence d'acides aminés comprenant TTEEKNGQKVDKPMEQQMKDRADEDTVH a été délétée, ou une délétion correspondante réalisée, à partir de la boucle 7.

13. Protéine selon l'une quelconque des revendications précédentes, dans laquelle, en référence à la FrpB de la souche H44/76, la délétion d'acides aminés est réalisée dans la plage des acides aminés 247 à 280 ou une délétion correspondante réalisée, à partir de la boucle 5.

14. Protéine selon l'une quelconque des revendications précédentes, dans laquelle, en référence à la FrpB de la souche H44/76, la délétion d'acides aminés est réalisée dans la plage des acides aminés 252 à 275 ou une délétion correspondante réalisée, à partir de la boucle 5.

15. Protéine selon l'une quelconque des revendications précédentes, dans laquelle, en référence à la FrpB de la souche H44/76, une séquence d'acides aminés comprenant QHRGIRTVREEFTVGDKSSRINID a été délétée, ou une délétion correspondante réalisée, à partir de la boucle 5.

16. Protéine selon l'une quelconque des revendications précédentes, dans laquelle la séquence délétée est remplacée par une autre séquence d'acides aminés.

17. Protéine selon la revendication 16, dans laquelle la séquence est délétée par mutagenèse.

18. Polynucléotide codant pour la protéine selon l'une quelconque des revendications précédentes.

19. Vecteur d'expression comprenant le polynucléotide selon la revendication 18.

20. Cellule hôte comprenant le vecteur d'expression selon la revendication 19.

21. Procédé de production de la protéine selon l'une quelconque des revendications 1 à 17 comprenant :
la culture de la cellule hôte selon la revendication 20 et la récupération de la protéine exprimée.

22. Procédé de repliement d'une protéine FrpB comprenant la mise en contact de la protéine FrpB avec un tampon alcalin de repliement comprenant du sulfonate de 3-diméthyldodécylammoniopropane (Zwittergent 3-12 ou SB-12), dans lequel la protéine est une protéine selon l'une quelconque des revendications 1 à 17.

23. Procédé selon la revendication 22, dans lequel le tampon de repliement comprend de l'éthanolamine et du SB-12.

24. Procédé selon la revendication 23, dans lequel l'éthanolamine est de l'éthanolamine à 20 mM.

25. Procédé selon l'une quelconque des revendications 22 à 24, dans lequel le tampon de repliement a un pH de 11.

26. Procédé selon l'une quelconque des revendications 22 à 25, dans lequel le SB-12 est du SB-12 à 0,2 %.

27. Procédé selon l'une quelconque des revendications 22 à 25, dans lequel le SB-12 est du SB-12 à 0,5 %.

28. Procédé selon l'une quelconque des revendications 22 à 27, dans lequel le tampon de repliement comprend en outre du chlorure de guanidium, du NaCl et/ou de l'urée.

29. Procédé selon la revendication 28, dans lequel le tampon de repliement comprend en outre du chlorure de guanidium.

30. Procédé selon la revendication 29, dans lequel le tampon de repliement comprend en outre du chlorure de guanidium à 0,4 M.

31. Procédé selon l'une quelconque des revendications 22 à 30, comprenant les étapes suivantes :
a. l'expression d'une protéine FrpB dans une cellule hôte ;
la rupture de la cellule hôte pour obtenir un corps d'inclusion comprenant la protéine FrpB ;
le lavage du corps d'inclusion ;
b. La solubilisation d'au moins une partie du corps d'inclusion et de la protéine FrpB avec du chlorhydrate de guanidinium ;
c. la mise en contact de la protéine FrpB solubilisée avec le tampon de repliement ; et
d. le prélèvement ou le changement du tampon de repliement à partir de la protéine FrpB.

32. Tampon de repliement comprenant de l'éthanolamine, du SB-12 et du chlorure de guanidinium pour une utilisation dans le procédé selon l'une quelconque des revendications 22 à 31.

33. Protéine FrpB repliée isolée selon les revendications 1 à 17, obtenue ou pouvant être obtenue par le procédé selon l'une quelconque des revendications 22 à 31.

34. Composition pharmaceutique comprenant au moins une protéine FrpB selon l'une quelconque des revendications 1 à 17 et 33, et un support pharmaceutiquement acceptable.

35. Composition pharmaceutique selon la revendication 34, dans laquelle au moins 30 %, 50 %, 70 % ou 90 % de la protéine FrpB présente dans la composition sont repliés.

36. Composition pharmaceutique selon la revendication 34 ou 35, sous la forme d'un vaccin.

37. Composition pharmaceutique selon l'une quelconque des revendications 34 à 36, comprenant une protéine FrpB dérivée de *Neisseria meningitidis.*

38. Composition pharmaceutique selon l'une quelconque des revendications 34 à 37, comprenant une protéine FrpB dérivée de *Neisseria gonorrhoeae.*

39. Composition pharmaceutique selon l'une quelconque des revendications 34 à 38, où ladite composition comprend au moins un autre antigène neissérien.

40. Composition pharmaceutique selon la revendication 39, comprenant au moins un autre antigène neissérien dérivé de *Neisseria gonorrhoeae.*

41. Composition pharmaceutique selon la revendication 39 ou 40, comprenant au moins un autre antigène neissérien dérivé de *Neisseria meningitidis.*

42. Composition pharmaceutique selon l'une quelconque des revendications 34 à 41, sous la forme d'une composition sous-unitaire.

43. Composition pharmaceutique selon l'une quelconque des revendications 34 à 41, sous la forme d'une préparation de vésicules de la membrane externe ou d'une sous-unité plus la préparation des vésicules de la membrane externe mélangées.

44. Composition pharmaceutique selon l'une quelconque des revendications 34 à 43, comprenant en outre au moins un autre antigène neissérien choisi parmi une ou plusieurs des classes suivantes :
a. au moins une adhésine neissérienne choisie dans le groupe constitué par FhaB, NspA, Hsf, NadA, PilC, Hap, MafA, MafB, Omp26, NMB0315, NMB0995 et NMB1119 ;
b. au moins un autotransporteur neissérien choisi dans le groupe constitué par Hsf, Hap, protéase d'IgA, AspA et NadA ;
c. au moins une toxine neissérienne choisie dans le groupe constitué par FrpA, FrpC, FrpA/C, VapD, NM-ADPRT, et l'un ou l'autre ou les deux du LPS immunotype L2 et du LPS immunotype L3 ;
d. au moins une protéine neissérienne d'acquisition de Fe choisie dans le groupe constitué par TbpA high, TbpA low, TbpB high, TbpB low, LbpA, LbpB, P2086, HpuA, HpuB, Lipo28, Sibp, FbpA, BfrA, BfrB, Bcp, NMB0964 et NMB0293 ; et
e. au moins une protéine neissérienne associée à la membrane, de préférence une protéine de la membrane externe, choisie dans le groupe constitué par PldA, NspA, TspA, FhaC, NspA, PbpA (high), TbpA (low), LbpA, HpuB, TdfH, PorB, HimD, HisD, GNA1870, OstA, HlpA, MltA, NMB 1124, NMB 1162, NMB 1220, NMB 1313, NMB 1953, HtrA, TspB, PilQ et OMP85.

45. Composition pharmaceutique selon l'une quelconque des revendications 34 à 44, comprenant en outre un ou plusieurs polysaccharides ou oligosaccharides capsulaires bactériens.

46. Composition pharmaceutique selon la revendication 45, dans laquelle un ou plusieurs polysaccharides ou oligosaccharides capsulaires sont dérivés de bactéries choisies dans le groupe constitué par *Neisseria meningitidis* du sérogroupe A, C, Y et/ou W-135, *Haemophilus influenzae* b, *Streptococcus pneumoniae,* des streptocoques du groupe A, des streptocoques du groupe B, *Staphylococcus aureus* et *Staphylococcus epidermidis* et sont conjugués à une source d'épitopes T auxiliaires.

47. Utilisation d'une protéine FrpB selon l'une quelconque des revendications 1 à 17 et 33 (ou d'une composition pharmaceutique selon les revendications 34 à 46) dans la préparation d'un médicament destiné au traitement ou à la prévention d'une infection neissérienne.

48. Utilisation selon la revendication 47, où une infection à *Neisseria meningitidis* est prévenue ou traitée.

49. Utilisation selon la revendication 47 ou 48, où une infection à *Neisseria gonorrhoeae* est prévenue ou traitée.

50. Anticorps immunospécifique de la protéine FrpB selon l'une quelconque des revendications 1 à 17 et 33.

51. Composition pharmaceutique utile dans le traitement d'êtres humains souffrant d'une maladie neissérienne comprenant au moins un anticorps selon la revendication 50 et un support pharmaceutique approprié.

52. Utilisation de l'anticorps selon la revendication 50 (ou d'une composition selon la revendication 51) dans la fabrication d'un médicament destiné au traitement ou à la prévention d'une maladie neissérienne.

53. Utilisation selon la revendication 52, où une infection à *Neisseria meningitidis* est prévenue ou traitée.

54. Utilisation selon la revendication 52 ou 53, où une infection à *Neisseria gonorrhoeae* est prévenue ou traitée.

55. Procédé de diagnostic d'une infection neissérienne, comprenant les étapes consistant à identifier une protéine FrpB, ou un anticorps dirigé contre celle-ci, au sein d'un échantillon biologique provenant d'un animal suspecté de souffrir d'une telle infection en utilisant une protéine FrpB selon l'une quelconque des revendications 1 à 17 et 33, ou un anticorps selon la revendication 50.

56. Procédé selon la revendication 55, dans lequel une infection à *Neisseria meningitidis* est diagnostiquée.

57. Procédé selon la revendication 55 ou 56, dans lequel une infection à *Neisseria gonorrhoeae* est diagnostiquée.
